# EUROPEAN PATENT SPECIFICATION

(11) **EP 3 722 431 B1**
(45) Date of publication and mention of the grant of the patent: **10.06.2026**
(21) Application number: 20157598.2
(22) Date of filing: 11.09.2014
(51) Int. Cl.: C12N 15/82, C07K 14/805

(54) **SECRETION OF HEME-CONTAINING POLYPEPTIDES**
AUSSCHEIDUNG HÄMHALTIGER POLYPEPTIDE
SÉCRÉTION DE POLYPEPTIDES CONTENANT DE L'HÈME

(30) Priority: 11.09.2013 US 201361876676 P; 25.11.2013 US 201361908689 P
(43) Date of publication of application: 14.10.2020
(62) Divisional of application: 14844701.4
(73) Proprietor: Impossible Foods Inc., Redwood City, CA 94063 (US)
(72) Inventor: FRASER, Rachel, San Francisco, CA California 94110 (US); DAVIS, Simon Christopher, San Francisco, CA California 94110 (US); BROWN, Patrick O'Reilly, Stanford, CA California 94305 (US)
(74) Representative: Haseltine Lake Kempner LLP

(56) References cited:
- WO-A1-2004/099405
- WO-A1-2013/010042
- WO-A1-98/12913
- WO-A2-00/00597
- WO-A2-2004/057946
- WO-A2-2006/091094
- US-A- 5 753 465
- US-A1- 2002 194 643
- LONDER Y Y ET AL: "Production and preliminary characterization of a recombinant triheme cytochrome c"7 from Geobacter sulfurreducens in Escherichia coli", BIOCHIMICA ET BIOPHYSICA ACTA. BIOENERGETICS, AMSTERDAM, NL, vol. 1554, no. 3, 1 July 2002 (2002-07-01), pages 202 - 211, XP004373902, ISSN: 0005-2728, DOI: 10.1016/S0005-2728(02)00244-X
- XU-CHU WU ET AL: "Efficient Production of a Functional Single-Chain Antidigoxin Antibody via an Engineered Bacillus subtilis Expression-Secretion System", BIOTECHNOLOGY. THE INTERNATIONAL MONTHLY FOR INDUSTRIAL BIOLOGY, vol. 11, no. 1, 1 January 1993 (1993-01-01), US, pages 71 - 76, XP055329301, ISSN: 0733-222X, DOI: 10.1038/nbt0193-71
- S. JOKIPII-LUKKARI ET AL: "Intrinsic non-symbiotic and truncated haemoglobins and heterologous Vitreoscilla haemoglobin expression in plants", JOURNAL OF EXPERIMENTAL BOTANY, vol. 60, no. 2, 6 January 2009 (2009-01-06), GB, pages 409 - 422, XP055339589, ISSN: 0022-0957, DOI: 10.1093/jxb/ern320

## Description

### TECHNICAL FIELD

This invention relates to methods and material for producing heme-containing polypeptides, and more particularly, to producing heme-containing polypeptides in recombinant bacterial cells such as *Bacillus.*

### BACKGROUND

There is a continuing need for methods to produce proteins at large scale for industrial and food purposes. *Bacillus* species can be used in the production of industrial enzymes such as lipases and proteases. In addition a number of food additives such as glucoamylase, lipases, and amylases can be produced in these hosts, providing a long history of safe use in the food industry. *Bacillus* species can secrete high levels of protein into the media surrounding the bacteria. Plant species, such as *Nicotiana tabacum* or *Glycine max* can also be used for the production of proteins.

Heme-containing polypeptides can be difficult to secrete as the cofactor must be inserted into the polypeptide and remain associated with the polypeptide throughout the secretion process in its native configuration. *Bacillus* species may use two different systems to secrete proteins (SEC and TAT). The SEC pathway unfolds the protein as they pass through the cell membrane. The TAT system can secrete the proteins in the folded state. However, it is unclear whether a recombinant hemoprotein containing a non-covalently bound heme group can be expressed, secreted and folded properly by the *Bacillus* system, until it is successfully done.

WO 00/00597 relates to recombinant expression systems that can express a gene encoding a nonsymbiotic hemoglobin, transformed cells and organisms and methods of using the same.

US 5,753,465 relates to methods for obtaining unmodified recombinant human normal adult hemoglobin involve a novel expression plasmid that coexpresses human alpha - and beta -globin genes and E. coli methionine aminopeptidase genes under the control of separate tac promoters.

Xu_Chu Wu et al. (Biotechnology, Vol. 11, No. 1, 1993, pages 71-76) relates to the production of a functional single-chain antidigoxin antibody via an engineered *Bacillus subtilis* expression-secretion system.

Londer et al. (Biochmica et Biophysia Acta. Bioenergetics, Vol. 1554, No. 3, July 2002, pages 202-211) relates to the production and preliminary characterization of a recombinant triheme cytochrome from *Grobacter sulferreducens* in *Escherichia coli.*

### SUMMARY

In a first aspect, the present invention provides a recombinant bacterium cell (e.g., a *Bacillus* cell such as a *Bacillus subtilis, Bacillus megaterium,* or *Bacillus licheniformis* cell), wherein said recombinant cell comprises at least one exogenous nucleic acid encoding a heme-containing polypeptide and is capable of secreting said heme-containing polypeptide, wherein said at least one exogenous nucleic acid encoding said heme-containing polypeptide comprises first and second nucleic acid sequences, wherein said first nucleic acid sequence encodes a signal peptide and said second nucleic acid sequence encodes said heme-containing polypeptide, wherein said first and second nucleic acid sequences are operably linked to produce a fusion polypeptide comprising said signal peptide and said heme-containing polypeptide; wherein said heme-containing polypeptide is selected from the group consisting of an androglobin, a cytoglobin, globin E, globin X, globin Y, a hemoglobin, a myoglobin, a leghemoglobin, an erythrocruorin, a beta hemoglobin, an alpha hemoglobin, a non symbiotic hemoglobin, a flavohemoglobin, a protoglobin, a cyanoglobin, a Hell's gate globin I, a bacterial hemoglobin, a ciliate myoglobin, a histoglobin, a neuroglobin, a protoglobin, and a truncated globin, wherein said truncated globin is preferably truncated 2/2 globin, HbN, HbO, or Glb3; wherein said at least one exogenous nucleic acid is a vector; and wherein said vector further comprises a polynucleotide sequence encoding two or more heme biosynthesis pathway enzymes. The exogenous nucleic acid also can include a third nucleic acid sequence encoding a tag such as an affinity tag. The cell can secrete the heme-containing polypeptide from the cell, and upon secretion, the signal peptide is removed from the heme-containing polypeptide. The signal peptide can comprise or consist of an amino acid sequence having at least 60% identity to a signal peptide set forth in SEQ ID NO: 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, or 93. For example, the signal peptide can comprise or consist of an amino acid sequence having at least 60% amino acid sequence identity to the amino acid sequence set forth in SEQ ID NO:55 or to residues 1-52 of SEQ ID NO:55

In a second aspect, the present invention provides a method for producing a heme-containing polypeptide, said method comprising culturing a recombinant bacterium cell of the first aspect (e.g., a *Bacillus* cell such as a *Bacillus subtilis, Bacillus megaterium,* or *Bacillus licheniformis* cell) in a culture medium under conditions that allow the heme-containing polypeptide to be secreted into said culture medium, wherein upon secretion of said fusion polypeptide from said recombinant bacterial cell into said culture medium, said signal peptide is removed from said heme-containing polypeptide. The method further can include recovering the heme-containing polypeptide from the culture medium. The signal peptide can comprise or consist of an amino acid sequence having at least 60% identity to a signal peptide set forth in SEQ ID NO: 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, or 93. For example, the signal peptide can comprise or consist of an amino acid sequence having at least 60% amino acid sequence identity to the amino acid sequence set forth in SEQ ID NO:55 or to residues 1-52 of SEQ ID NO:55.

### BRIEF DESCRIPTION OF THE DRAWINGS

The novel features of the disclosure are set forth with particularity in the appended claims. A better understanding of the features and advantages of the present will be obtained by reference to the following detailed description that sets forth illustrative embodiments, in which the principles are utilized, and the accompanying drawings of which:

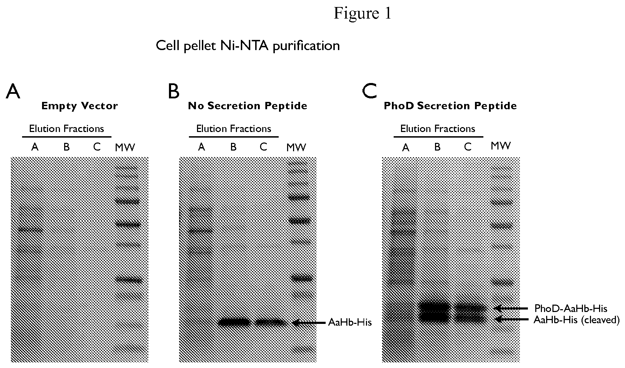

Figure 1 contains three SDS-PAGE gels of the proteins after Ni-NTA affinity purification of the cell pellet, showing a comparison of the effect of a secretion signal peptide (*PhoD*) on cytosolic expression of *Aquifex aeolicus* hemoglobin (*AaHb*). Figure 1A is the empty vector control. Figure 1B is without the secretion signal peptide and Figure 1C is with the secretion signal peptide.

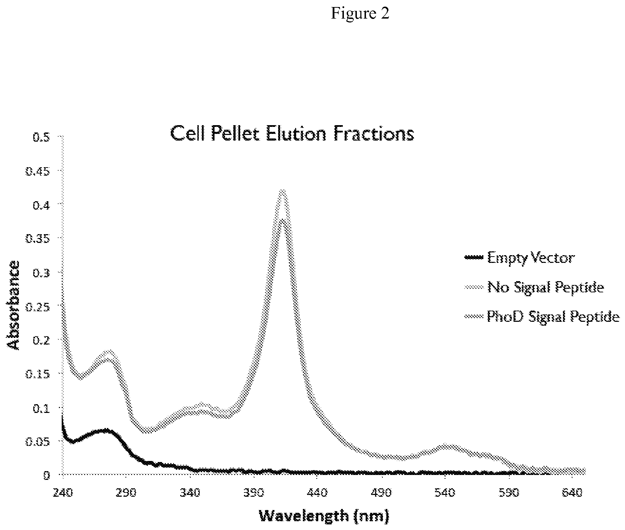

Figure 2 is a graph depicting the heme content of a cytosolically expressed polypeptide (*AaHb*). The line corresponding to the empty vector is the line with the lowest peak. The line corresponding to the polypeptide with no signal peptide is the line with the highest peak. The line corresponding to the polypeptide with the *PhoD* signal peptide is the line with the second highest peak.

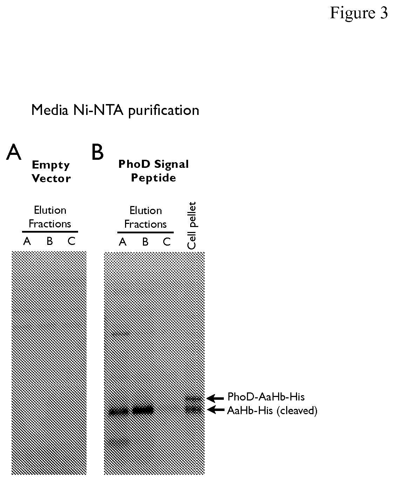

Figure 3 contains two SDS-PAGE gels of the proteins after Ni-NTA affinity purification of the media, showing a comparison of the effect of a secretion signal peptide (*PhoD*) on secretory expression of a polypeptide (*AaHb*). Figure 3A is the empty vector control. Figure 3B is with the secretion signal peptide.

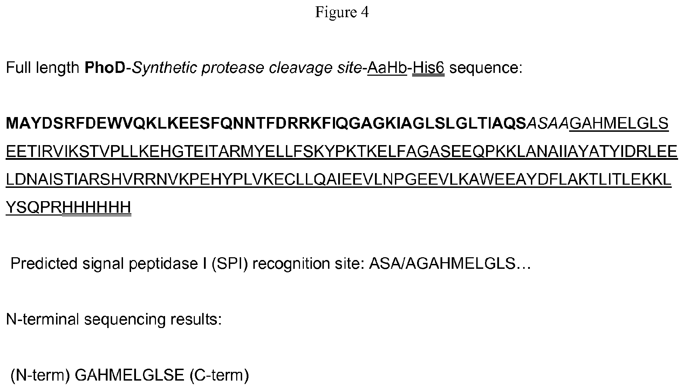

Figure 4 is the sequence of the fusion polypeptide containing the PhoD (in bold text)-synthetic protease cleavage site (in italics, ASAA) -AaHb (underlined)-His6 (double underlined) sequence (SEQ ID NO: 94). The predicted signal peptidase I (SPI) recognition site is shown (SEQ ID NO:95), with the cleavage site indicated. N-terminal sequencing (SEQ ID NO:96) of the secreted polypeptide present in the media after cytosolic expression of a *PhoD-AaHb* fusion polypeptide indicated the N-terminus corresponded to the N-terminus of the AaHb protein.

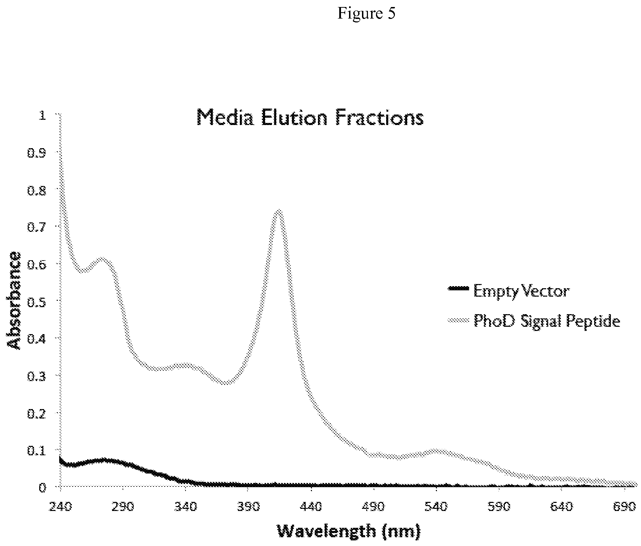

Figure 5 depicts the heme content of a secreted polypeptide (after expression of the fusion polypeptide *PhoD-AaHb*). The line corresponding to the empty vector is the line with the lowest peak. The line corresponding to the secreted polypeptide after expression of the fusion polypeptide which included the *PhoD* signal peptide is the line with the highest peak.

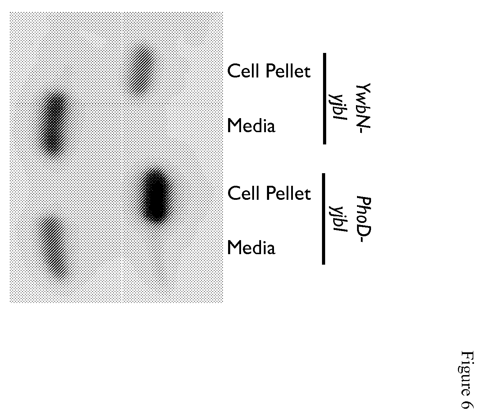

Figure 6 illustrates 1) detection of two exemplary fusion polypeptides (*PhoD-yjbI* and *YwbN-yjbI*) in the cell pellet following expression of an endogenous polypeptide (*yjbI*) fused to one of two different signaling peptides (*PhoD* or *YwbN*), and 2) media detection of the polypeptides demonstrating proper cleavage of the signal peptides.

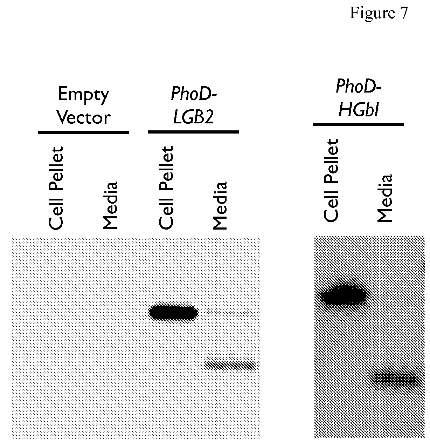

Figure 7 illustrates 1) detection of two exemplary fusion polypeptides (*PhoD-LGB2* and *PhoD-HGbI*) in the cell pellet following expression of two heterologous polypeptides (*LGB2* and *HGbI*) fused to the signaling peptide (*PhoD*) and 2) media detection of the polypeptides, demonstrating proper cleavage of the signal peptide.

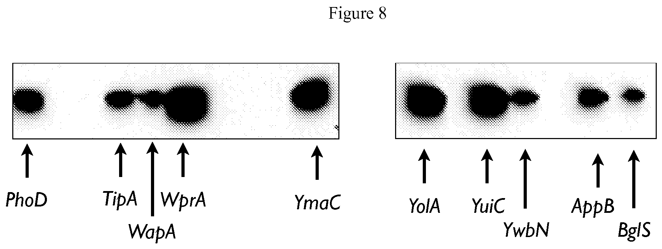

Figure 8 illustrates media detection of a polypeptide (*AaHb*) after fusion with a subset of a number of different exemplary secretion signaling peptides (*PhoD, TipA, WapA, WprA, YmaC, YolA, YuiC, YwbN, AppB,* and *BglS*), expression, and cleavage of the secretion signal peptide. Labels indicate the secretion signal peptide that was fused to the 5' end of the polypeptide prior to cleavage.

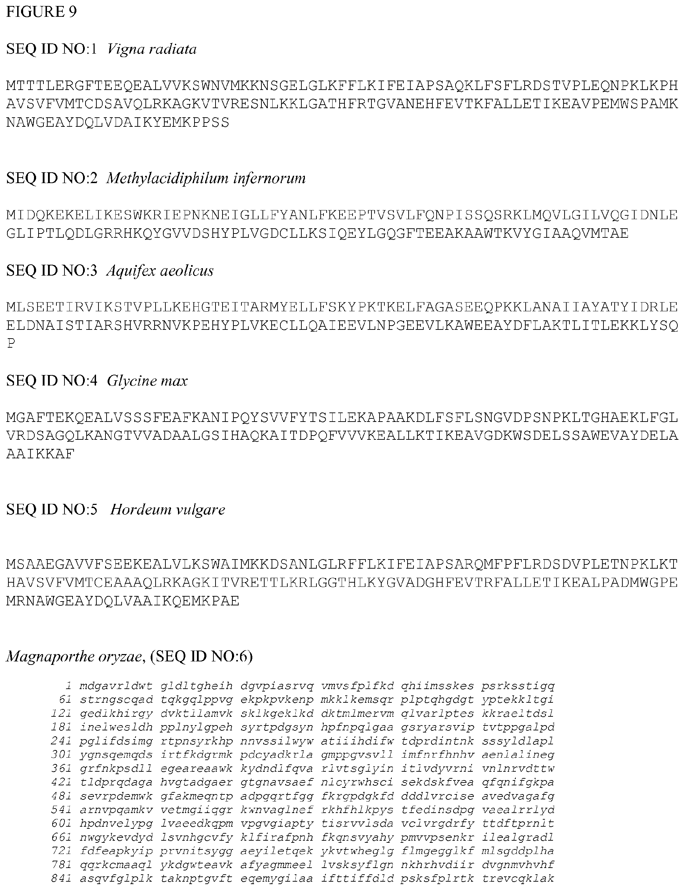

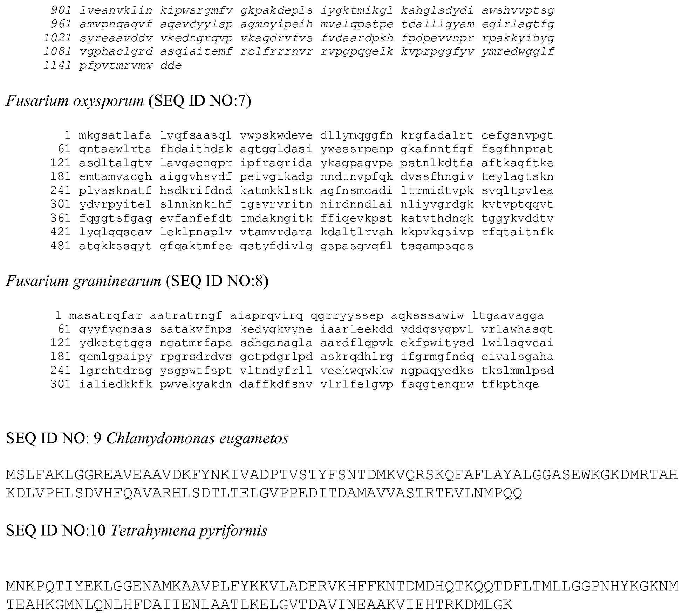

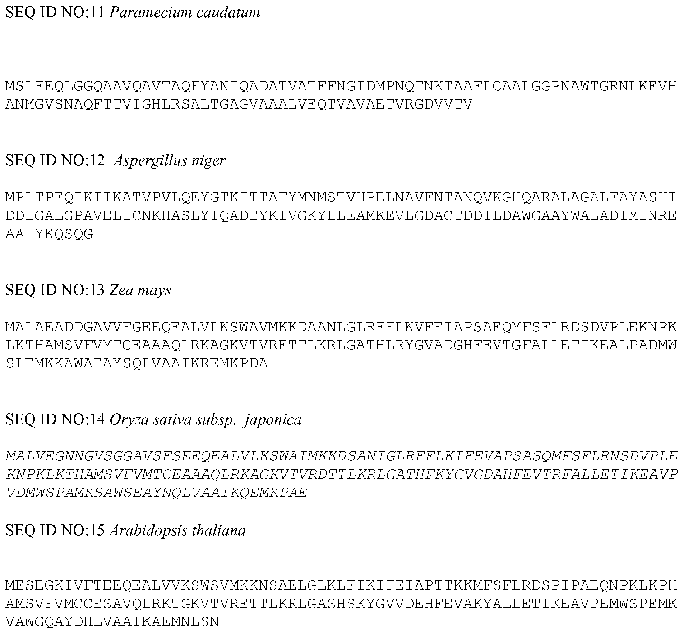

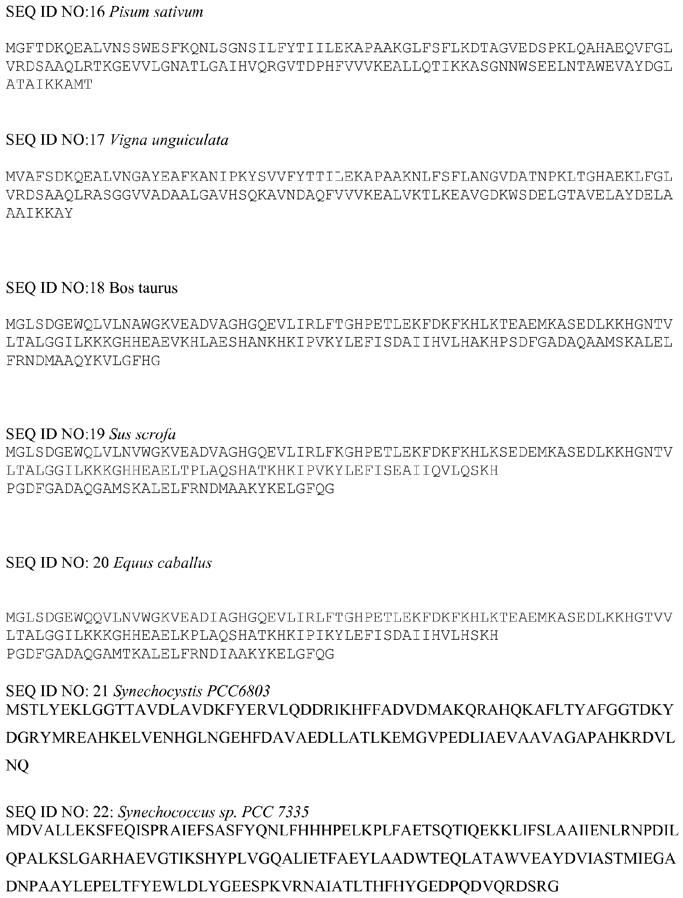

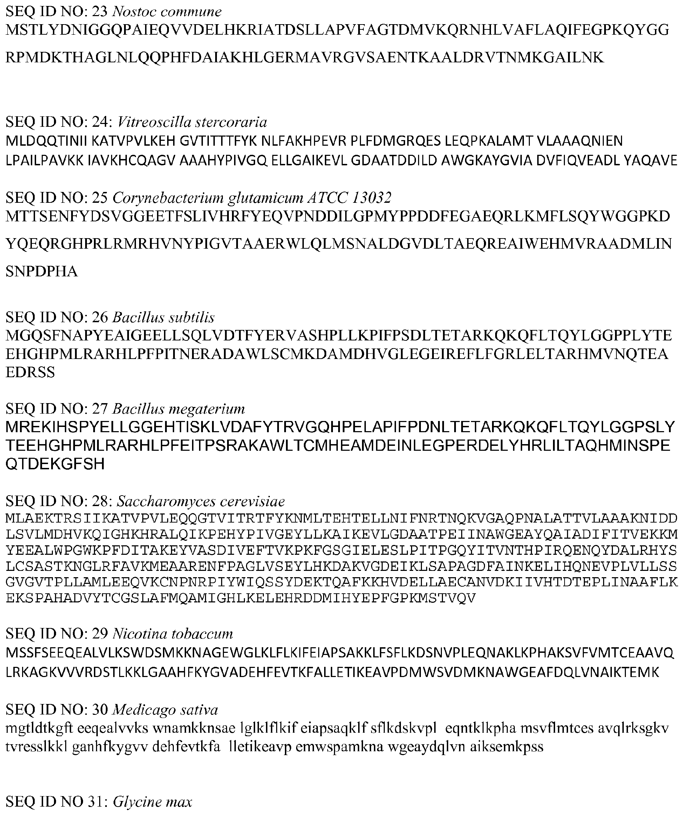

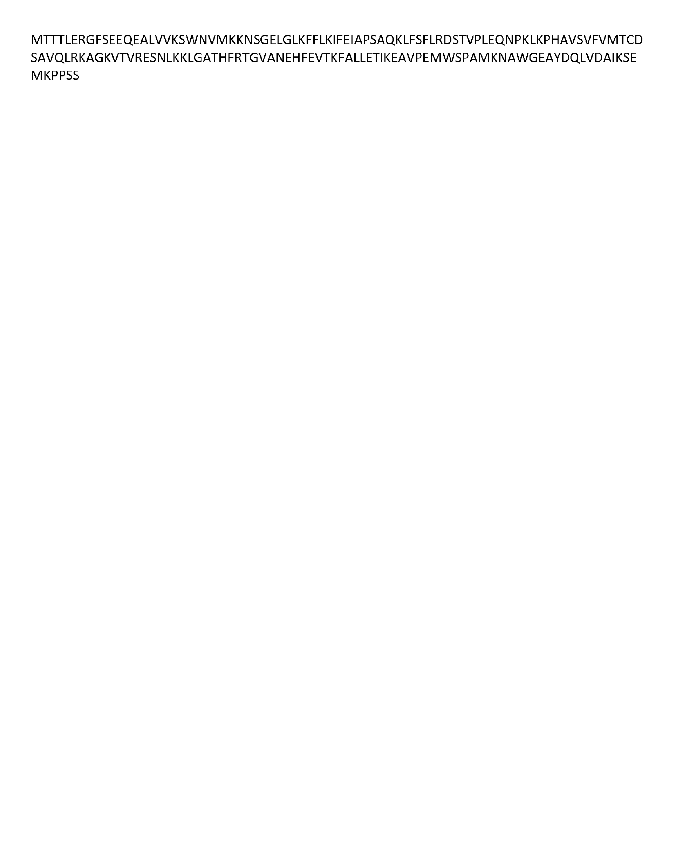

Figure 9 contains the amino acid sequences of exemplary heme-containing polypeptides (SEQ ID NOs: 1-31).

### DETAILED DESCRIPTION

### Polypeptides

This disclosure describes compositions and methods for the expression of a polypeptide in a host cell (e.g., bacteria and/or plants). A polypeptide can refer to subunits or domains of a polypeptide. A polypeptide of the disclosure can be a heme-containing polypeptide. The term heme-containing polypeptide can refer to all proteins or protein subunits that are capable of covalently or noncovalently binding a heme moiety. Heme-containing polypeptides can transport or store oxygen. In some instances the polypeptide of the disclosure can be a globin. Polypeptides can comprise the globin fold, which can comprise a series of eight alpha helices. A polypeptide can comprise an alpha globin and/or a beta globin. A polypeptide can comprise a characteristic higher structure (e.g., the "myoglobin fold") generally associated with globins. A polypeptide can be an oligomer. Polypeptides can be monomers, dimers, trimers, tetramers, and/or higher order oligomers. In some instances, a polypeptide can be an iron-containing polypeptide.

A polypeptide of the disclosure can include, but is not limited to, androglobin, cytoglobin, globin E, globin X, globin Y, hemoglobin, myoglobin, leghemoglobins, erythrocruorins, beta hemoglobins, alpha hemoglobins, non-symbiotic hemoglobins, flavohemoglobins, protoglobins, cyanoglobins, cytoglobin, Hell's gate globin I, bacterial hemoglobins, ciliate myoglobins, histoglobins, neuroglobins, chlorocruorin, erythrocruorin, protoglobin, truncated 2/2 globin, HbN, HbO, Glb3, and cytochromes, ribosomal proteins, actin, hexokinase, lactate dehydrogenase, fructose bisphosphate aldolase, phosphofructokinases, triose phosphate isomerases, phosphoglycerate kinases, phosphoglycerate mutases, enolases, pyruvate kinases, proteases, lipases, amylases, glycoproteins, lectins, mucins, glyceraldehyde-3-phosphate dehydrogenases, pyruvate decarboxylases, actins, translation elongation factors, histones, ribulose-1,5-bisphosphate carboxylase oxygenase (rubisco), ribulose-1,5-bisphosphate carboxylase oxygenase activase (rubisco activase), albumins, glycinins, conglycinins, globulins, vicilins, conalbumin, gliadin, glutelin, gluten, glutenin, hordein, prolamin, phaseolin (protein), proteinoplast, secalin, extensins, triticeae gluten, collagens, zein, kafirin, avenin, dehydrins, hydrophilins, late embyogenesis abundant proteins, natively unfolded proteins, any seed storage protein, oleosins, caloleosins, steroleosins orother oil body proteins, vegetative storage protein A, vegetative storage protein B, moong seed storage 8S globulin, globulin, pea globulins, and pea albumins. In some instances, a polypeptide of the disclosure can comprise or can be a polypeptide listed in Figure 9. In some instances, a polypeptide can be introduced into a host cell. For example, a polypeptide can be expressed, secreted, and/or purified from bacteria such as a species of *Bacillus*. A polypeptide can be expressed and/or purified from a plant.

A polypeptide listed in Figure 9, may be expressed, but may not be properly secreted and/or folded using the methods of the disclosure. A polypeptide listed in Figure 9 may be expressed, but may be not be correctly localized in the cell using the methods of the disclosure. A polypeptide listed in Figure 9, may be expressed, but may not retain levels of activity comparable to a wild-type polypeptide. A polypeptide listed in Figure 9 may retain at least about 1, 5, 10, 20, 30, 40, 50, 60, 70, 80, 90, 100% activity level of a wild-type polypeptide. A polypeptide listed in Figure 9 may retain at most about 1, 5, 10, 20, 30, 40, 50, 60, 70, 80, 90, or 100% activity level of a wild-type polypeptide. A polypeptide comprising at least about 10, 20, 30, 40, 50, 60, 70, 80, 90, or 100% amino acid sequence identity to a polypeptide listed in Figure 9 may be expressed, but may not be properly secreted and/or folded using the methods of the disclosure. A polypeptide comprising at most about 10, 20, 30, 40, 50, 60, 70, 80, 90, or 100% amino acid sequence identity to a polypeptide listed in Figure 9 may be expressed, but may not be properly secreted and/or folded using the methods of the disclosure. A polypeptide comprising at most about 10, 20, 30, 40, 50, 60, 70, 80, 90, or 100% amino acid sequence identity to a polypeptide listed in Figure 9 may be expressed, but may not be retain activity compared to a wild-type polypeptide. A polypeptide comprising at most about 10, 20, 30, 40, 50, 60, 70, 80, 90, 100% amino acid sequence identity to a polypeptide listed in Figure 9 may be expressed, but may contain less heme cofactor compared to a wild-type polypeptide.

In some instances, a sequence of a polypeptide to be expressed in a host cell can be a sequence comprising at least about 10, 20, 30, 40, 50, 60, 70, 80, 90, or 100% amino acid sequence identity to an endogenous polypeptide sequence, e.g., an endogenous heme-containing polypeptide, of the host cell. In some instances, a sequence of a polypeptide to be expressed in a host cell can be a sequence comprising at most about 10, 20, 30, 40, 50, 60, 70, 80, 90, or 100% amino acid sequence identity to an endogenous polypeptide sequence of the host cell. For example, a polypeptide can be a polypeptide sequence found in an animal, a mammal, a vertebrate, an invertebrate, a plant, a fungus, a bacterium, a yeast, an alga, an archaea, a genetically modified organism such as a genetically modified bacterium or yeast. A polypeptide sequence can be chemically synthesized, and/or synthesized by *in vitro* synthesis.

A polypeptide sequence can be a sequence of a polypeptide, e.g., a heme-containing polypeptide, found in plants. Non-limiting examples of plants can include grains such as, e.g., corn, maize, oats, rice, wheat, barley, rye, triticale, teff, oilseeds including cottonseed, sunflower seed, safflower seed, crambe, camelina, mustard, rapeseed, leafy greens such as, e.g., lettuce, spinach, kale, collard greens, turnip greens, chard, mustard greens, dandelion greens, broccoli, cabbage, sugar cane, trees, root crops such as cassava, sweet potato, potato, carrots, beets, turnips, plants from the legume family, such as, e.g., clover, peas such as cowpeas, English peas, yellow peas, green peas, beans such as, e.g., soybeans, fava beans, lima beans, kidney beans, garbanzo beans, mung beans, pinto beans, lentils, lupins, mesquite, carob, soy, and peanuts, coconut, vetch (vicia), stylo (stylosanthes), arachis, indigofera, acacia, leucaena, cyamopsis, and sesbania. Plants not ordinarily consumed by humans, including biomass crops, including, for example, switchgrass, miscanthus, tobacco, *Arundo donax,* energy cane, sorghum, other grasses, alfalfa, corn stover, kelp, or other seaweeds. Polypeptides that can be found in any organism in the plant kingdom may be used in the present disclosure. In some instances, the plant can be soy. In some instances, the plant can be barley.

In some instances, a polypeptide sequence can be a sequence, e.g., a heme-containing polypeptide sequence, found in metazoa. For example, a polypeptide sequence of the disclosure can be a polypeptide sequence found in mammals such as cow, pig, rat, dog, or horse. In some instances, the polypeptide sequence comes from cow. In some instances, the polypeptide sequence comes from pig. In some instances, a polypeptide sequence can be a sequence found in protists. For example, a polypeptide sequence of the disclosure can be a polypeptide sequence found in protists such as algae. In some instances, a polypeptide sequence can be a sequence found in archaea. For example, a polypeptide sequence of the disclosure can be a polypeptide sequence found in archaea such as halobacteria or pyrococcus. In some instances, a polypeptide sequence can be a sequence found in eubacteria. For example, a polypeptide sequence of the disclosure can be a polypeptide sequence found in eubacteria such as *Bacillus, Clostridia,* or *Escherichia.*

As used herein, the term "heme containing protein" includes any polypeptide that can covalently or noncovalently bind to a heme moiety. In some embodiments, the heme-containing polypeptide is a globin and can include a globin fold, which comprises a series of seven to nine alpha helices. Globin type proteins can be of any class (e.g., class I, class II, or class III), and in some embodiments, can transport or store oxygen. For example, a heme-containing polypeptide can be a non-symbiotic type of hemoglobin or a leghemoglobin. A heme-containing polypeptide can be a monomer, i.e., a single polypeptide chain, or can be a dimer, a trimer, tetramer, and/or higher order oligomers. The life-time of the oxygenated Fe²⁺ state of a heme-containing polypeptide can be similar to that of myoglobin or can exceed it by 10%, 20%, 30%, 40%, 50%, 100% or more.

Non-limiting examples of heme-containing polypeptides can include an androglobin, a cytoglobin, a globin E, a globin X, a globin Y, a hemoglobin, a myoglobin, an erythrocruorin, a beta hemoglobin, an alpha hemoglobin, a protoglobin, a cyanoglobin, a histoglobin, a neuroglobin, a chlorocruorin, a truncated hemoglobin (e.g., HbN, HbO, a truncated 2/2 globin, a hemoglobin 3 (e.g., Glb3)), a cytochrome, or a peroxidase.

Heme-containing polypeptides can be from mammals (e.g., farms animals such as cows, goats, sheep, pigs, ox, or rabbits), birds, plants, algae, fungi (e.g., yeast or filamentous fungi), ciliates, or bacteria. For example, a heme-containing polypeptide can be from a mammal such as a farm animal (e.g., a cow, goat, sheep, pig, ox, or rabbit) or a bird such as a turkey or chicken. Heme-containing polypeptides can be from a plant such as *Nicotiana tabacum* or *Nicotiana sylvestris* (tobacco); *Zea mays* (corn), *Arabidopsis thaliana,* a legume such as *Glycine max* (soybean), *Cicer arietinum* (garbanzo or chick pea), *Pisum sativum* (pea) varieties such as garden peas or sugar snap peas, *Phaseolus vulgaris* varieties of common beans such as green beans, black beans, navy beans, northern beans, or pinto beans, *Vigna unguiculata* varieties (cow peas), *Vigna radiate* (Mung beans), *Lupinus albus* (lupin), or *Medicago sativa* (alfalfa); *Brassica napus* (canola); *Triticum sps.* (wheat, including wheat berries, and spelt); *Gossypium hirsutum* (cotton); *Oryza sativa* (rice); *Zizania sps.* (wild rice); *Helianthus annuus* (sunflower); *Beta vulgaris* (sugarbeet); *Pennisetum glaucum* (pearl millet); *Chenopodium sp.* (quinoa); *Sesamum sp.* (sesame); *Linum usitatissimum* (flax); or *Hordeum vulgare* (barley). Heme-containing polypeptides can be isolated from fungi such as *Saccharomyces cerevisiae, Pichia pastoris, Magnaporthe oryzae, Fusarium graminearum,* or *Fusarium oxysporum.* Heme-containing polypeptides can be isolated from bacteria such as *Escherichia coli, Bacillus subtilis, Synechocistis sp., Aquifex aeolicus, Methylacidiphilum infernorum,* or thermophilic bacteria such as *Thermophilus.*

The sequences and structure of numerous heme-containing polypeptides are known. See for example, Reedy, et al., Nucleic Acids Research, 2008, Vol. 36, Database issue D307-D313 and the Heme Protein Database available on the world wide web at http://hemeprotein.info/heme.php.

A non-symbiotic hemoglobin can be from a plant selected from the group consisting of soybean, sprouted soybean, alfalfa, golden flax, black bean, black eyed pea, northern, garbanzo, moong bean, cowpeas, pinto beans, pod peas, quinoa, sesame, sunflower, wheat berries, spelt, barley, wild rice, or rice.

Any of the heme-containing polypeptides described herein can have at least 60% (e.g., at least 65%, 70%, 75%, 80%, 85%, 90%, 95%, 97%, 98%, 99%, or 100%) sequence identity to the amino acid sequence of the corresponding wild-type heme-containing polypeptide or fragments thereof that contain a heme-binding motif. For example, a heme-containing polypeptide can have at least 60% sequence identity to an amino acid sequence set forth in FIG. 9, including a non-symbiotic hemoglobin such as that from *Vigna radiata* (SEQ ID NO: 1), *Hordeum vulgare* (SEQ ID NO:5), *Zea mays* (SEQ ID NO: 13), *Oryza sativa subsp. japonica (rice)* (SEQ ID NO:14), *or Arabidopsis thaliana* (SEQ ID NO:15), a Hell's gate globin I such as that from *Methylacidiphilum infernorum* (SEQ ID NO:2), a flavohemoprotein such as that from *Aquifex aeolicus* (SEQ ID NO:3), a leghemoglobin such as that from *Glycine max* (SEQ ID NO:4), *Pisum sativum* (SEQ ID NO:16), or *Vigna unguiculata* (SEQ ID NO: 17), a heme-dependent peroxidase such as from *Magnaporthe oryzae,* (SEQ ID NO:6) or *Fusarium oxysporum* (SEQ ID NO:7), a cytochrome c peroxidase from *Fusarium graminearum* (SEQ ID NO:8), a truncated hemoglobin from *Chlamydomonas moewusii* (SEQ ID NO:9), *Tetrahymena pyriformis* (SEQ ID NO:10, group I truncated), *Paramecium caudatum* (SEQ ID NO: 11, group I truncated), a hemoglobin from *Aspergillus niger* (SEQ ID NO:12), or a mammalian myoglobin protein such as the *Bos taurus* (SEQ ID NO: 18) myoglobin, *Sus scrofa* (SEQ ID NO: 19) myoglobin, *Equus caballus* (SEQ ID NO:20) myoglobin, a *Synechocystis PCC6803* (SEQ ID NO:21) truncated hemoglobin, a *Synechococcus sp. PCC 7335* (SEQ ID NO:22) truncated hemoglobin, a *Nostoc commune* (SEQ ID NO:23) hemoglobin, a *Vitreoscilla stercoraria* (SEQ ID NO:24) hemoglobin, a *Corynebacterium glutamicum* (SEQ ID NO:25) hemoglobin, a *Bacillus subtilis* (SEQ ID NO:26) truncated hemoglobin, a *Bacillus megaterium* (SEQ ID NO:27) truncated hemoglobin, a *Saccharomyces cerevisiae* (SEQ ID NO:28) flavohemoglobin, a *Nicotina tobaccum* (SEQ ID NO:29) non-symbiotic hemoglobin, a *Medicago sativa* (SEQ ID NO:30) non-symbiotic hemoglobin, or a *Glycine max* (SEQ ID NO: 31) non-symbiotic hemoglobin.

The percent identity between two amino acid sequences can be determined as follows. First, the amino acid sequences are aligned using the BLAST 2 Sequences (B12seq) program from the stand-alone version of BLASTZ containing BLASTP version 2.0.14. This stand-alone version of BLASTZ can be obtained from Fish & Richardson's web site (e.g., www.fr.com/blast/) or the U.S. government's National Center for Biotechnology Information web site (www.ncbi.nlm.nih.gov). Instructions explaining how to use the B12seq program can be found in the readme file accompanying BLASTZ. Bl2seq performs a comparison between two amino acid sequences using the BLASTP algorithm. To compare two amino acid sequences, the options of B12seq are set as follows: -i is set to a file containing the first amino acid sequence to be compared (e.g., C:\seq1.txt); -j is set to a file containing the second amino acid sequence to be compared (e.g., C:\seq2.txt); -p is set to blastp; -o is set to any desired file name (e.g., C:\output.txt); and all other options are left at their default setting. For example, the following command can be used to generate an output file containing a comparison between two amino acid sequences: C:\Bl2seq-i c:\seq1.txt -j c:\seq2.txt -p blastp -o c:\output.txt. If the two compared sequences share homology, then the designated output file will present those regions of homology as aligned sequences. If the two compared sequences do not share homology, then the designated output file will not present aligned sequences. Similar procedures can be following for nucleic acid sequences except that blastn is used.

Once aligned, the number of matches is determined by counting the number of positions where an identical amino acid residue is presented in both sequences. The percent identity is determined by dividing the number of matches by the length of the full-length polypeptide amino acid sequence followed by multiplying the resulting value by 100. It is noted that the percent identity value is rounded to the nearest tenth. For example, 78.11, 78.12, 78.13, and 78.14 is rounded down to 78.1, while 78.15, 78.16, 78.17, 78.18, and 78.19 is rounded up to 78.2. It also is noted that the length value will always be an integer.

It will be appreciated that a number of nucleic acids can encode a polypeptide having a particular amino acid sequence. The degeneracy of the genetic code is well known to the art; i.e., for many amino acids, there is more than one nucleotide triplet that serves as the codon for the amino acid. For example, codons in the coding sequence for a given enzyme can be modified such that optimal expression in a particular species (e.g., bacteria or fungus) is obtained, using appropriate codon bias tables for that species.

Heme-containing polypeptides can be extracted from the source material (e.g., extracted from animal tissue, or plant, fungal, algal, or bacterial biomass, or from the culture supernatant for secreted proteins) or from a combination of source materials (e.g., multiple plant species). Leghemoglobin is readily available as an unused by-product of commodity legume crops (e.g., soybean, alfalfa, or pea). The amount of leghemoglobin in the roots of these crops in the United States exceeds the myoglobin content of all the red meat consumed in the United States.

In some embodiments, extracts of heme-containing polypeptides include one or more non-heme-containing polypeptides from the source material (e.g., other animal, plant, fungal, algal, or bacterial proteins) or from a combination of source materials (e.g., different animal, plant, fungi, algae, or bacteria).

A polypeptide of the disclosure (e.g., a globin, a heme-containing polypeptide, or an iron-containing protein), can be referred to as a "purified" polypeptide. A polypeptide of the disclosure can be purified from other components of the source material (e.g., other animal, plant, fungal, algal, or bacterial proteins). A purified polypeptide can refer to a polypeptide that has been enriched in a composition, has been manipulated in some fashion to remove unwanted debris (e.g., cell debris, genomic DNA, and/or other polypeptides), and/or is removed from the host cell in which it was synthesized (e.g., transcribed/translated) (e.g., cell lysis). A "purified" polypeptide can be a polypeptide extracted from its host cell. In some variants, a "purified" polypeptide is at least 1% pure, e.g., at least 2%, 5%, 10%, 15%, 20%, 25%, 30%, 35%, 40%, 45%, 50%, 55%, 60%, 65%, 70%, 75%, 80%, 85%, 90%, 95%, or 99% pure. Proteins can be separated on the basis of their molecular weight, for example, by size exclusion chromatography, ultrafiltration through membranes, or density centrifugation. In some cases, the proteins can be separated based on their surface charge, for example, by isoelectric precipitation, anion exchange chromatography, or cation exchange chromatography. Proteins also can be separated on the basis of their solubility, for example, by ammonium sulfate precipitation, isoelectric precipitation, surfactants, detergents or solvent extraction. Proteins also can be separated by their affinity to another molecule, using, for example, hydrophobic interaction chromatography, reactive dyes, or hydroxyapatite. Affinity chromatography can also include using antibodies having specific binding affinity for the heme-containing polypeptide, antibody to the protein, nickel NTA for His-tagged recombinant proteins, lectins to bind to sugar moieties on a glycoprotein, or other molecules which specifically binds the protein.

### Hemoglobin

Hemoglobin (Hb) can be the major constituent of an erythrocyte which can carry oxygen from the lungs throughout the body. When contained in red blood cells, human Hb can exist as a tetramer structure composed of two oxygen linked αβ dimers, each having a molecular weight of about 32 kD. Each α and β subunit of each dimer can have a protein chain and a heme molecule. Hemoglobin, or "Hb" can refer to (a) an iron-containing respiratory pigment found in vertebrate red blood cells that comprises a globin composed of four subunits (a tetramer) each of which is linked to a heme molecule, that functions in oxygen transport to the tissues after conversion to oxygenated form in the gills or lungs, and that assists in carbon dioxide transport back to the gills or lungs after surrender of its oxygen. A hemoglobin can refer to a recombinantly produced hemoglobin; αβ-dimers of hemoglobin, inter- or intramolecularly crosslinked hemoglobin, as well as modified versions of the hemoglobins described in the disclosure, which can include but are not limited to modifications increasing or decreasing the oxygen affinity of hemoglobin (e.g., such as substituting an alanine, valine, leucine, or phenylalanine for histidine at position E7 (e.g., position 62 of SEQ ID NO: 4). See, for example, Hargrove et al., J. Mol. Biol. (1997) 266, 1032-1042. All hemoglobins can be capable of binding heme. A hemoglobin can be a variant hemoglobin. Variant hemoglobins can comprise amino acid mutations, substitutions, additions, and/or deletions. Hemoglobin variants can include hemoglobin Kansas, hemoglobin S, hemoglobin C, hemoglobin E, hemoglobin D-Punjab, hemoglobin O-Arab, hemoglobin G-Philadelphia, hemoglobin Hasharon, hemoglobin Lepore, and hemoglobin M.

### Leghemoglobin

In some instances, the sequence (amino acid and/or nucleic acid) of a leghemoglobin can be a plant leghemoglobin sequence. Various legumes species and their varieties, for example, Soybean, Fava bean, Lima bean, Cowpeas, English peas, Yellow peas, Lupine, Kidney bean, Garbanzo beans, Peanut, Alfalfa, Vetch hay, Clover, Lespedeza and Pinto bean, comprise nitrogen- fixing root nodules in which leghemoglobin can have a key role in controlling oxygen concentrations. Leghemoglobins from different species can be homologs and have similar color properties. Some plant species can express several leghemoglobin isoforms (for example soybean has four leghemoglobin isoforms). Minor variations in precise amino acid sequence can modify overall charge of the protein at a particular pH and can modify precise structural conformation of an iron containing heme group in leghemoglobin. In some instances, an alanine, valine, leucine, or phenylalanine can be substituted for histidine at position 62 of SEQ ID NO: 4). Differences in structural conformation of the heme group of different leghemoglobins can influence oxidation and reduction rates of the heme iron. These differences may contribute to color and flavor generation properties of different leghemoglobins.

In other cases, the sequence (amino acid and/or nucleic acid) of a heme-containing polypeptide can be from a non-plant organism, such as from animals (e.g., a cow, pig, dog, rat, or horse), fish, archaea, protists, bacteria, fungus, eubacteria, metazoa, or yeast.

### Variants

A polypeptide of the disclosure can be a variant (e.g., comprise a mutation such as an amino acid substitution, e.g., a non-conservative or conservative amino acid substitution, an amino acid deletion, an amino acid insertion, or non-native sequence). In some instances a variant polypeptide can be a variant of a polypeptide listed in Figure 9 (see, e.g., SEQ ID NOs: 1-31). In some instances, a variant polypeptide can include at most 1, 2, 3, 4, 5, 6, 7, 8, 9, 10, 15, 20, 30, 40, or 50 mutations. In some instances, a variant polypeptide comprises at least 1, 2, 3, 4, 5, 6, 7, 8, 9, 10, 15, 20, 30, 40, or 50 or more mutations. In some instances, at least 1, 2, 3, 4, 5, 6, 7, 8, 9, 10, 20, 30, 40, or 50% of the sequence of a polypeptide of the disclosure can be mutated. In some instances, at most 1, 2, 3, 4, 5, 6, 7, 8, 9, 10, 20, 30, 40, or 50% of the sequence of a polypeptide of the disclosure can be mutated. In some instances, a polypeptide of the disclosure can comprise at least about 10, 20, 30, 40, 50, 60, 65, 70, 75, 76, 77, 78, 79, 80, 81, 82, 83, 84, 85, 86, 87, 88, 89, 90, 91, 92, 93, 94, 95, 96, 97, 98, 99 or 100% amino acid sequence identity to a naturally occurring polypeptide of the disclosure. In some instances, a polypeptide of the disclosure can comprise at most about 10, 20, 30, 40, 50, 60, 65, 70, 75, 76, 77, 78, 79, 80, 81, 82, 83, 84, 85, 86, 87, 88, 89, 90, 91, 92, 93, 94, 95, 96, 97, 98, 99 or 100% amino acid sequence identity to a naturally occurring polypeptide of the disclosure.

In some instances, the polypeptide of the disclosure comprises a non-native sequence (e.g., a tag or a label). A tag can be covalently bound to the polypeptide sequence of the polypeptide. The tag can be bound to the N-terminus, or the C-terminus, or to an intervening amino acid. The tag can be inserted in the polypeptide sequence (e.g., in a solvent accessible surface loop). Examples of tags can include, but are not limited to, affinity tags (e.g., myc, maltose binding protein, or 6xhis, metal chelating peptides such as histidine-tryptophan modules that allow purification on immobilized metals, protein A domains that allow purification on immobilized immunoglobulin, and the domain utilized in the FLAGS extension/affinity purification system), and fluorescent tags (e.g., green fluorescent protein).

A tag can be a composition detectable by spectroscopic, photochemical, biochemical, immunochemical, chemical, or other physical means. For example, tags suitable for use in the present disclosure can include biotin, digoxigenin, or haptens as well as proteins which can be made detectable, fluorescent dyes (e.g., fluorescein isothiocyanate, Texas red, rhodamine, and the like), radiolabels (e.g., 3 H, 125 I, 35 S, 14 C, or 32 P), dyes (e.g., alexa, cy3 cy5), chemical conjugates (e.g., quantum dots), enzymes (e.g., horse radish peroxidase, alkaline phosphatase and others commonly used in an ELISA), and colorimetric labels such as colloidal gold, colored glass or plastic beads (e.g., polystyrene, polypropylene, latex, etc. ).

A tag can be detected. For example, where the tag is radioactive, means for detection can include a scintillation counter or photographic film, as in autoradiography. Where the tag is a fluorescent tag, it may be detected by exciting the fluorochrome with the appropriate wavelength of light and detecting the resulting fluorescence. The fluorescence may be detected visually by the use of electronic detectors such as charge coupled devices (CCDs) or photomultipliers and the like. Similarly, enzymatic tags may be detected by providing the appropriate substrates for the enzyme and detecting the resulting reaction product. Colorimetric or chemiluminescent tags may be detected simply by observing the color associated with the tag.

In some instances, a tag can be a signal peptide. A signal peptide can be a peptide sequence usually present at the N-terminal end of newly synthesized secretory or membrane polypeptides which directs the polypeptide across or into a cell membrane of the cell (the plasma membrane in prokaryotes or the endoplasmic reticulum membrane in eukaryotes). It can be subsequently removed (e.g., by a protease). In particular the signal peptide may be capable of directing the polypeptide into a cell's secretory pathway. In some instances, the signal peptide is a secretory pathway signal peptide. In some such instances, the signal peptide can be referred to as a signal peptide or a secretion signal peptide.

Examples of signal peptides can include, but are not limited to, the signal peptides listed in Table 1 (SEQ ID NO: 33, 35, 37, 39, 41, 43, 45, 47, 49, 51, 53, 55, 57, 59, 61, 63, 65, 67, 69, 71, 73, 75,77, 79, 81, 83, 85, 87, 89, 91, or 93). Nucleotides in parenthesis in Table 1 may or may not be included and therefore, the signal peptide may or may not have the residues encoded by the nucleotides. For example, in some instances, the nucleic acid sequence set forth in SEQ ID NO:42 does not include the last 9 nucleotides (i.e., it would only contain nucleotides 1-84 of SEQ ID NO:42) and accordingly, the signal peptide set forth in SEQ ID NO:43 does not include the last three residues (i.e., the signal peptide only would contain amino acids 1-28 of SEQ ID NO:43). For example, in some instances, the nucleic acid sequence set forth in SEQ ID NO:44 does not include the last 6 nucleotides (i.e., it would only contain nucleotides 1-96 of SEQ ID NO:44) and accordingly, the signal peptide set forth in SEQ ID NO:45 does not include the last two residues (i.e., the signal peptide only would contain amino acids 1-32 of SEQ ID NO:45). For example, in some instancess, the nucleic acid sequence set forth in SEQ ID NO:54 does not include the last 12 nucleotides (i.e., it would only contain nucleotides 1-156 of SEQ ID NO:54) and accordingly, the signal peptide set forth in SEQ ID NO:55 does not include the last four residues (i.e., the signal peptide only would contain amino acids 1-52 of SEQ ID NO:55). In some instances, a signal peptide can comprise PhoD (e.g., SEQ ID NO:55 or residues 1-52 of SEQ ID NO:55). Similarly, the signal peptides of SEQ ID NOs: 59, 63, 65, 67, 71, 73, 75, 77, 79, 83, 85, 87, or 93 may lack from one to four C-terminal amino acids.

In some instances, a signal peptide can be a variant signal peptide. A signal peptide can be a variant of the signal peptides listed in Table 1 (SEQ ID NO: 33, 35, 37, 39, 41, 43, 45, 47, 49, 51, 53, 55, 57, 59, 61, 63, 65, 67, 69, 71, 73, 75,77, 79, 81, 83, 85, 87, 89, 91, or 93). In some instances, a variant signal peptide comprises at least about 5, 10, 15, 20, 25, 30, 35, 40, 45, 50, 55, 60, 65, 70, 75, 76, 77, 78, 79, 80, 81, 82, 83, 84, 85, 86, 87, 88, 89, 90, 91, 92, 93, 94, 95, 96, 97, 98, 99, or 100% amino acid sequence identity to a signal peptide (e.g., a signal peptide listed in Table 1). For example, a variant signal peptide can have at least about 20, 25, 30, 35, 40, 45, 50, 55, 60, 65, 70, 75, 76, 77, 78, 79, 80, 81, 82, 83, 84, 85, 86, 87, 88, 89, 90, 91, 92, 93, 94, 95, 96, 97, 98, 99, or 100% sequence identity to any one of the signal peptides set forth in Table 1. In some instances, a variant signal peptide can comprise at most about 5, 10, 15, 20, 25, 30, 35, 40, 45, 50, 55, 60, 65, 70, 75, 76, 77, 78, 79, 80, 81, 82, 83, 84, 85, 86, 87, 88, 89, 90, 91, 92, 93, 94, 95, 96, 97, 98, 99 or 100% amino acid sequence identity to a signal peptide (e.g., a signal peptide listed in Table 1). In some instances the cleavage site between the signal peptide and the polypeptide may be derived from the signal peptide. In some instances the cleavage site may be a synthetic protease cleavage site. Cleavage of the signal peptide can result in a polypeptide of the disclosure comprising all, some, or none of the signal peptide. Cleavage of the synthetic protease cleavage site can result in a polypeptide of the disclosure comprising all, some, or none of the synthetic protease cleavage site.

**Table 1: Exemplary Signal Peptides**

| **Gene** | **DNA sequence** | **SEQ ID NO** | **Peptide sequence (predicted cut site underlined)** | **SEQ ID NO:** | **Uniprot** |
|---|---|---|---|---|---|
| AbnA | ATGAAAAAGAAAAAAACATG GAAACGCTTCTTACACTTTTC GAGTGCAGCTCTGGCTGCAG GTTTGATATTCACTTCTGCTG CTCCCGCAGAGGCAG | 32 | MKKKKTWKRFLHFSSAALAAGLIFTSAAP AEAA | 33 | P94522 |
| AlbB | ATGTCACCAGCACAAAGAAG AATTTTACTGTATATCCTTTC ATTTATCTTTGTCATCGGCGC AGTCGTCTATTTTGTCAAAAG CGATTATCTGTTTACGCTGAT TTTCATTGCCATTGCCATTCT GTTCGG | 34 | MSPAQRRILL YILSFIFVIG AVVYFVKSDY LFTLIFIAIA ILF | 35 | P71010 |
| AmyX | ATGGTCAGCATCCGCCGCAGC TTCGAAGCGTATGTCGATGAC ATGAATATCATTACTGTTCTG ATTCCTGCTGAACAAAAGGA AATCATG | 36 | MVSIRRSFEA YVDDMNIITV LIPAEQKEIM | 37 | C0SPA0 |
| AppB | ATGGCTGCATATATTATCAGA AGAACCTTAATGTCTATCCCG ATTTTATTGGGAATTACGATT | 38 | MAAYIIRRTL MSIPILLGIT ILSFVIMKAA PGD | 39 | P42062 |
| | TTATCATTTGTTATCATGAAA GCCGCGCCCGGAGAT | | | | |
| BglC | ATGAAACGGTCAATCTCTATT TTTATTACGTGTTTATTGATTA CGTTATTGACAATGGGCGGCA TGATAGCTTCGCCGGCATCAG CA | 40 | MKRSISIFITCLLITLLTMGGMIASPASA | 41 | P42403 |
| BglS | ATGCCTTATCTGAAACGAGTG TTGCTGCTTCTTGTCACTGGA TTGTTTATGAGTTTGTTTGCA GTCACTGCTACTGCCTCAGCT (CAA ACA GGT) | 42 | MPYLKRVLLL LVTGLFMSLF AVTATASAQT G | 43 | P04957 |
| LipA | ATGAAATTTGTAAAAAGAAG GATCATTGCACTTGTAACAAT TTTGATGCTGTCTGTTACATC GCTGTTTGCGTTGCAGCCGTC AGCAAAAGCCGCT (GAA CAC) | 44 | MKFVKRRIIA LVTILMLSVT SLFALQPSAK AAEH | 45 | O32129 |
| LytD | ATGAAAAAGAGACTAATCGC ACCTATGCTTCTATCCGCCGC GTCCCTTGCCTTTTTTGCCATG TCTGGTTCTGCCCAGGCAGCC GCGTAT | 46 | MKKRLIAPML LSAASLAFFA MSGSAQAAAY | 47 | P39848 |
| OppA | ATGAAAAAACGTTGGTCGATT GTCACGTTGATGCTCATTTTC ACTCTCGTGCTGAGCGCGTGC GGCTTTGGC | 48 | MKKRWSIVTL MLIFTLVLSA CGFG | 49 | P24141 |
| OppB | ATGCTAAAATATATCGGAAG ACGCTTAGTCTATATGATTAT CACACTATTTGTGATTGTAAC TGTGACATTCTTCTTAATGCA AGCAGCACCGGGCGGG | 50 | MLKYIGRRLV YMIITLFVIV TVTFFLMQAA PGG | 51 | P24138 |
| PbpX | ATGACAAGCCCAACCCGCAG AAGAACTGCGAAACGCAGAC GGAGAAAACTAAATAAAAGA GGAAAACTGTTGTTTGGTCTT TTAGCAGTGATGGTTTGCATT ACGATTTGGAATGCTCTTCAT CGA | 52 | MTSPTRRRTA KRRRRKLNKR GKLLFGLLAV MVCITIWNAL HR | 53 | O31773 |
| PhoD | ATGGCATACGACAGTCGTTTT GATGAATGGGTACAGAAACT GAAAGAGGAAAGCTTTCAAA ACAATACGTTTGACCGCCGCA AATTTATTCAAGGAGCGGGG AAGATTGCAGGACTTTCTCTT GGATTAACGATTGCCCAGTCG | 54 | MAYDSRFDEWVQKLKEESFQNNTFDRRK FIQGAGKIAGLSLGLTIAQSVGAFEVNA | 55 | P42251 |
| | GTTGGGGCCTTT(GAA GTA AAT GCT) | | | | |
| QcrA | ATGGGCGGAAAACATGATAT ATCCAGACGTCAATTTTTGAA TTATACGCTCACAGGCGTAGG AGGTTTTATGGCGGCTAGTAT GCTCATGCCTATGGTTCGCTT CGCACTCGACCCG | 56 | MGGKHDISRR QFLNYTLTGV GGFMAASMLM PMVRFALDP | 57 | P46911 |
| SpoIIIJ | ATGTTGTTGAAAAGGAGAAT AGGGTTGCTATTAAGTATGGT TGGCGTATTCATGCTTTTGGC TGGA (TGC TCG AGT GTG) | 58 | MLLKRRIGLL LSMVGVFMLL AG CSSV | 59 | Q01625 |
| TipA | ATGAAAAAAACACTCACCAC TATTCGCAGATCATCAATTGC AAGGAGACTTATTATTTCTTT CCTGCTGATCTTAATTGTTCC GATAACCGCCCTTTCGGTTAG CGCTTATCAATCAGCAGTTGC CTCA | 60 | mkktlttirr ssiarrliis fllilivpit alsvsayqsa vas | 61 | |
| WapA | ATGAAAAAAAGAAAGAGGCG AAACTTTAAAAGGTTCATTGC AGCATTTTTAGTGTTGGCTTT AATGATTTCATTAGTGCCAGC | 62 | MKKRKRRNFK RFIAAFLVLA LMISLVPADV LA KST | 63 | Q07833 |
| | CGATGTACTAGCA (AAA TCT ACA) | | | | |
| WprA | ATGAAACGCAGAAAATTCAG CTCGGTTGTGGCGGCAGTGCT TATTTTTGCACTGATTTTCAG CCTTTTTTCTCCGGGAACCAA AGCTGCAGCG (GCC GGC GCG) | 64 | MKRRKFSSVV AAVLIFALIF SLFSPGTKAA A AGA | 65 | P54423 |
| YdeJ | ATGAAGAAACGCAGAAAGAT ATGTTATTGCAATACTGCCCT GCTGCTTATGATTTTGCTTGC TGGA (TGT ACG GAC AGT) | 66 | MKKRRKICYC NTALLLMILL AG CTDS | 67 | P96667 |
| YesM | ATGAAGAAAAGAGTTGCTGG CTGGTACAGGCGGATGAAGA TTAAGGATAAGCTGTTTGTGT TTCTATCGTTGATTATGGCCG TATCCTTTCTGTTTGTATACA GCGGGGTCCAGTATGCCTTTC ATGTG | 68 | MKKRVAGWYR RMKIKDKLFV FLSLIMAVSF LFVYSGVQYA FHV | 69 | O31516 |
| YesW | ATGAGAAGGAGCTGTCTGAT GATTAGACGAAGGAAACGCA TGTTTACCGCTGTTACGTTGC TGGTCTTGTTGGTGATGGGAA | 70 | MRRSCLMIRR RKRMFTAVTL LVLLVMGTSV CPVKAEG A | 71 | O31526 |
| | CCTCTGTATGTCCTGTGAAAG CTGAAGGG (GCA) | | | | |
| YfkN | ATGAGAATACAGAAAAGACG AACACACGTCGAAAACATTCT CCGTATTCTTTTGCCCCCAAT TATGATACTTAGCCTAATCCT CCCAACACCACCCATTCATGC A (GAA GAA AGC) | 72 | MRIQKRRTHV ENILRILLPP IMILSLILPT PPIHA EES | 73 | O34313 |
| YhcR | ATGCTGTCTGTCGAAATGATA AGCAGACAAAATCGTTGTCAT TATGTGTATAAGGGAGGAAA TATGATGAGGCGTATTCTGCA TATTGTGTTGATCACGGCATT AATGTTCTTAAATGTAATGTA CACGTTCGAAGCT (GTA AAG GCA) | 74 | MLSVEMISRQ NRCHYVYKGG NMMRRILHIV LITALMFLNV MYTFEA VKA | 75 | P54602 |
| YkpC | ATGCTAAGAGATTTAGGAAG AAGAGTAGCGATCGCAGCCA TTTTAAGCGGAATTATTCTTG GAGGCATGAGCATTTCTTTGG CA (AAT ATG CCC) | 76 | MLRDLGRRVA IAAILSGIIL GGMSISLA NM P | 77 | Q45492 |
| YkuE | ATGAAAAAGATGTCCAGAAG ACAATTTCTAAAAGGAATGTT CGGCGCTCTTGCTGCCGGGGC | 78 | MKKMSRRQFL KGMFGALAAG ALTAGGGYGY A RYL | 79 | O34870 |
| | TTTAACGGCCGGCGGGGGAT ATGGCTATGCC (AGG TAT CTC) | | | | |
| YmaC | ATGAGAAGATTTTTACTAAAT GTCATATTAGTCTTAGCCATT GTCTTGTTCTTGAGATATGTT CATTACTCATTGGAACCAGAA | 80 | MRRFLLNVIL VLAIVLFLRY VHYSLEPE | 81 | O31789 |
| YmzC | ATGTTTGAAAGTGAAGCAGA ACTGAGACGAATCAGGATTG CACTTGTATGGATAGCTGTCT TTTTACTGTTCGGGGCG (TGC GGG AAT) | 82 | MFESEAELRR IRIALVWIAV FLLFGA CGN | 83 | O31797 |
| YolA | ATGAAGAAGAGAATTACATA TTCACTGCTTGCTCTTCTAGC AGTTGTTGCTTTCGCTTTCACT GATTCATCAAAAGCAAAAGC G (GCA GAA GCA) | 84 | MKKRITYSLL ALLAVVAFAF TDSSKAKA AE A | 85 | O31994 |
| YubF | ATGCAGAAATATAGACGCAG AAACACGGTTGCCTTTACAGT ACTAGCTTATTTTACTTTTTTT GCGGGAGTATTTTTGTTTAGT ATCGGACTCTATAATGCTGAT AATCTGGAACT | 86 | MQKYRRRNTV AFTVLAYFTF FAGVFLFSIG LYNADNLE | 87 | O32082 |
| YuiC | ATGATGTTGAATATGATCAGA CGTTTGCTGATGACCTGTTTA TTTCTGCTTGCATTTGGCACG ACATTTTTATCAGTGTCAGGA ATTGAAGCG (AAG GAC TTG) | 88 | MMLNMIRRLL MTCLFLLAFG TTFLSVSGIE A KDL | 89 | O32108 |
| YvhJ | ATGGCTGAACGCGTTAGAGT GCGTGTGCGAAAAAAGAAAA AGAGCAAACGTAGGAAAATT TTAAAAAGAATAATGTTATTG TTCGCCCTTGCACTATTGGTA GTTGTAGGGCTTGGCGGGTAT AAACTTTAT | 90 | MAERVRVRVR KKKKSKRRKI LKRIMLLFAL ALLVVVGLGG YKLY | 91 | P96499 |
| YwbN | ATGAGCGATGAACAGAAAAA GCCAGAACAAATTCACAGAC GGGACATTTTAAAATGGGGA GCGATGGCGGGGGCAGCCGT TGCGATCGGTGCCAGCGGTCT CGGCGGTCTCGCTCCGCTTGT TCAGACTGCGGCT (AAG CCA) | 92 | MSDEQKKPEQ IHRRDILKWG AMAGAAVAIG ASGLGGLAPL VQTAA KP | 93 | P39597 |

### Protoporphyrins

A polypeptide can bind to a tetrapyrrole (e.g., protoporphyrin). A polypeptide can bind to a protoporphyrin with its protoporphyrin binding portion (e.g., domain). A polypeptide can bind to a protoporphyrin as the polypeptide is being translated/folded. A polypeptide can bind to a protoporphyrin after the polypeptide is translated/folded. A polypeptide can remain bound to a protoporphyrin after it has been subcellularly localized (e.g., localized to a subcellular compartment, secreted).

Protoporphyrins can comprise side chains including methyl groups, propionic acid groups, and vinyl groups. Suitable protoporphyrin structures can include, but are not limited to, diiododeuteroporphyrin, mesoporphyrin, metalloporphyrins, and protoporphyrin IX. In some instances, a polypeptide can bind to more than one protoporphyrin. A polypeptide can bind to one, two, three, four, five, six, seven, eight, nine, ten or more protoporphyrins.

A protoporphyrin can be a protoporphyrin IX. Protoporphyrin IX (PpIX), Pheophorbide, a naturally occurring photosensitizer, can be the immediate precursor of heme in the heme biosynthetic pathway. Protoporphyrin IX can be referred to as heme. Heme can comprise a protoporphyrin ring and an iron atom, wherein the iron atom is coordinated by the members of the ring (e.g., the iron atom is inside the ring). In some instances the protoporphyrin can be heme A, heme B, heme C, heme D, heme I, heme M, heme O or Heme S. In some instances, a protoporphyrin can coordinate an atom other than iron (i.e., metalloporphyrin). Other atoms can include for example, zinc, gadolinium, magnesium, manganese, cobalt, nickel, tin, and copper.

### Vectors and Genetically Modified Organisms

### Exogenous nucleic acids

The disclosure describes an exogenous nucleic acid encoding a polypeptide of the disclosure (e.g., a heme-containing polypeptide, a globin). An exogenous nucleic acid can encode any of the heme-containing polypeptides described herein, e.g., a heme-containing polypeptide having at least about 60% identity (e.g., at least 65%, 75%, 80%, 85%, 90%, 95%, 99% identity) to one of the amino acid sequences listed in Figure 9). An exogenous nucleic acid can be RNA or DNA, and can be single stranded, double stranded, and/or codon optimized. An exogenous nucleic acid sequence encoding a polypeptide of the disclosure can be transcribed and/or translated. The term "polynucleotide" can be used interchangeably herein with "exogenous nucleic acid."

The term "exogenous" as used herein with reference to a nucleic acid (or a protein) and a host refers to a nucleic acid that does not occur in (and cannot be obtained from) a cell of that particular type as it is found in nature or a protein encoded by such a nucleic acid. Thus, a non-naturally-occurring nucleic acid is considered to be exogenous to a host once in the host. It is important to note that non-naturally-occurring nucleic acids can contain nucleic acid subsequences or fragments of nucleic acid sequences that are found in nature provided the nucleic acid as a whole does not exist in nature. For example, a nucleic acid molecule containing a cDNA sequence within an expression vector is non-naturally-occurring nucleic acid, and thus is exogenous to a host cell once introduced into the host, since that nucleic acid molecule as a whole (cDNA plus vector DNA) does not exist in nature. Thus, any vector, autonomously replicating plasmid, or virus (e.g., retrovirus, adenovirus, or herpes virus) that as a whole does not exist in nature is considered to be non-naturally-occurring nucleic acid. It follows that genomic DNA fragments produced by PCR or restriction endonuclease treatment as well as cDNAs are considered to be non-naturally-occurring nucleic acid since they exist as separate molecules not found in nature. It also follows that any nucleic acid containing a regulatory element (e.g., a promoter sequence and/or a signal sequence) and a sequence encoding a heme-containing polypeptide (e.g., cDNA or genomic DNA) in an arrangement not found in nature is non-naturally-occurring nucleic acid. A nucleic acid that is naturally-occurring can be exogenous to a particular host (e.g., bacteria or plant). For example, an entire chromosome isolated from a cell of plant x is an exogenous nucleic acid with respect to a cell of plant y once that chromosome is introduced into a cell of plant y.

In contrast, the term "endogenous" as used herein with reference to a nucleic acid (e.g., a gene) (or a protein) and a host refers to a nucleic acid (or protein) that does occur in (and can be obtained from) that particular host as it is found in nature. Moreover, a cell "endogenously expressing" a nucleic acid (or protein) expresses that nucleic acid (or protein) as does a host of the same particular type as it is found in nature. Moreover, a host "endogenously producing" or that "endogenously produces" a nucleic acid, protein, or other compound produces that nucleic acid, protein, or compound as does a host of the same particular type as it is found in nature.

The degeneracy of the genetic code can permit variations of the nucleotide sequence, while still producing a polypeptide having the identical amino acid sequence as the polypeptide encoded by the native polynucleotide sequence. Variations in the polynucleotide sequence can be customized for any organism of interest. In some instances, a polynucleotide encoding a polypeptide can be codon optimized for expression in a bacteria (e.g., a gram positive bacteria such as *B. subtilis*). In some instances, a polynucleotide encoding a polypeptide can be codon optimized for expression in a plant (e.g., *N. tabacum*).

The frequency of individual synonymous codons for cognate amino acids varies widely from genome to genome among eukaryotes and prokaryotes. These differences in codon choice patterns can contribute to the overall expression levels of individual genes by modulating peptide elongation rates.

### Bacterial Vectors

As described herein, an exogenous nucleic acid can include a nucleic acid encoding a signal peptide and a nucleic acid encoding a heme-containing polypeptide. In some instances, the exogenous nucleic acid is a vector. A vector can be suitable for expression in a prokaryote (e.g., bacteria).

The vectors disclosed herein generally comprise regulatory control sequences, (e.g., transcriptional or translational control sequences) required for expressing the polypeptide. Suitable regulatory control sequences can include but are not limited to replication origin, promoter, enhancer, repressor binding regions, transcription initiation sites, ribosome binding sites, translation initiation sites, or termination sites for transcription and translation.

In some variants, the vector can comprise a polynucleotide sequence encoding two or more polypeptides (e.g., heme-containing polypeptides or heme biosynthesis pathway enzymes), which can be present on the same vector. In some variants, when present on the same vector, the polynucleotides are arranged such that they form an operon, (i.e., transcription of the polynucleotides will generate a polycistronic messenger RNA). In some instances, the two or more polynucleotides can be arranged on the same vector such that they are operably linked to their own promoter.

The origin of replication (generally referred to as an ori sequence) can permit replication of the vector in a host cell. The choice of ori can depend on the type of host cells that are employed. Where the host cells are prokaryotes, the expression vector can comprise an ori directing autonomous replication of the vector within the prokaryotic cells. Non-limiting examples of this class of ori include pMBl, pUC, ColE1 as well as other bacterial origins.

The host cell can comprise a polynucleotide encoding a polypeptide of the disclosure for recombinant production, wherein the polypeptide can be linked to a functional signal sequence (e.g., a sequence encoding a signal peptide). Sequences encoding signal peptides can include, for example, those derived from spA, phoA, ribose binding protein, pelB, ompA, ompT, dsbA, torA, torT, and tolT, the signal peptides listed in Table 1, or signal peptides from the TAT secretion pathway in bacteria. Also included within the scope of the disclosure are signal sequences derived from eukaryotic cells that also function as signal sequences in prokaryotic host cells.

The vectors may comprise a selectable marker such as a gene encoding a protein necessary for the survival or growth of a host cell transformed with the vector. A marker gene can be carried on another polynucleotide sequence co-introduced into the host cell. Only those host cells into which a selectable gene has been introduced can survive and/or grow under selective conditions. Typical selection genes can encode protein(s) that (a) confer resistance to antibiotics or other toxins, (e.g., ampicillin, kanamycin, neomycin, G418, methotrexate, etc.); (b) complement auxotrophic deficiencies; or (c) supply critical nutrients not available from complex media.

The vector may comprise a polynucleotide sequence encoding a tag. A tag sequence can be in-frame to the coding sequence of the polypeptide of the disclosure, such that upon translation of the sequence, the polypeptide of the disclosure is covalently bound, e.g., fused, to the tag (e.g., is a fusion protein). The tag can be separated from the polypeptide of the disclosure by a linker, e.g., a polypeptide linker. A vector can comprise a polynucleotide sequence encoding a linker between the sequence encoding the tag and the sequence encoding the polypeptide of the disclosure.

The vectors described herein can be obtained using recombinant cloning methods and/or by chemical synthesis. Recombinant cloning techniques can include PCR, restriction endonuclease digestion and ligation. Sequence data, that can be located in the public or proprietary databases, can be used to obtain a desired vector by any synthetic means. Additionally, using restriction and ligation techniques, appropriate sequences can be excised from various DNA sources and integrated in operative relationship with the exogenous sequences to be expressed in accordance with the present disclosure.

In some instances, the vector can comprise a regulatory element (also referred to as a regulatory control element herein). A regulatory element can include, for example, replication origin, promoters, TATA boxes, enhancers, ribosome binding sites, repressor binding regions, transcription initiation sites, transcription termination sites, non-native sequences (e.g., tags) and untranslated regions. In some instances, the regulatory element can be a promoter. A promoter can be constitutive, inducible, and/or tissue specific. Exemplary promoters can include both constitutive promoters and inducible promoters. A natural promoter can be modified by replacement, substitution, addition or elimination of one or more nucleotides without changing its function.

In some variants, in addition to a promoter sequence, the polynucleotide sequence also includes a transcription termination region downstream of the nucleic acid sequence encoding the polypeptide to provide for efficient termination. In some variants, the termination region can be obtained from the same gene as the promoter sequence, while in other variants it can be obtained from another gene.

In some instances, the vector can comprise a polynucleotide sequence encoding a polypeptide of the disclosure and a tag. In some instances, the tag can be a signal peptide.

In some variants, once the desired form of a polypeptide, nucleic acid sequence, homologue, variant or fragment thereof, is obtained, it can be modified by any number of ways. Where the sequence involves non-coding flanking regions, the flanking regions may be subjected to resection, mutagenesis, etc. Thus, transitions, transversions, deletions, and insertions may be performed on the naturally occurring sequence.

In some particular variants, a polynucleotide encoding a polypeptide can include the coding sequence for at least one polypeptide (e.g., globin), or variant(s), fragment(s) or splice variant(s) thereof: (i) in isolation; (ii) in combination with additional coding sequences; such as fusion protein or signal peptide coding sequences, where the polypeptide-coding sequence is the dominant coding sequence; and/or (iii) in combination with non-coding sequences, such as control elements, such as promoter and terminator elements or 5' and/or 3' untranslated regions, effective for expression of the coding sequence in a suitable host.

In some variants, a polynucleotide encoding a polypeptide, together with appropriate promoter and control sequences, can be introduced into bacterial host cells to permit the cells to express at least one polypeptide (e.g., globin) or variant thereof.

Natural or synthetic polynucleotide fragments encoding a polypeptide (e.g., a heme-containing polypeptide, a globin) may be incorporated into vectors, capable of introduction into, and replication in, a bacterial cell. Any vector may be used as long as it is replicable and viable in the cells into which it is introduced. The appropriate DNA sequence can be inserted into a plasmid or vector by any suitable method. In general, the DNA sequence is inserted into an appropriate restriction endonuclease site(s) by recombinant molecular biology techniques.

In some instances, the disclosure describes a genetically modified organism. In some instances, a genetically modified organism can comprise a polypeptide of the disclosure. A genetically modified organism can comprise a polynucleotide encoding a heme-containing polypeptide.

### Plant vectors

The present disclosure describes for vectors for introduction of exogenous nucleic acid in a method according to the disclosure which can find use in the expression of a nucleic acid in a plant cell, specific plant tissues such as the leaf, the root, the seed or the bean, or a specific compartment of a plant cell.

Transgenic plant cells and plants are disclosed herein comprising at least one exogenous nucleic acid. As described herein, an exogenous nucleic acid can include a nucleic acid encoding a signal peptide and a nucleic acid encoding a heme-containing polypeptide. A plant or plant cell can be transformed by having a construct integrated into its genome, i.e., can be stably transformed. Stably transformed cells typically retain the introduced nucleic acid with each cell division. A plant or plant cell also can be transiently transformed such that the construct is not integrated into its genome. Transiently transformed cells typically lose all or some portion of the introduced nucleic acid construct with each cell division such that the introduced nucleic acid cannot be detected in daughter cells after a sufficient number of cell divisions. Both transiently transformed and stably transformed transgenic plants and plant cells can be useful in the methods described herein.

Typically, transgenic plant cells used in methods described herein constitute part or all of a whole plant. Such plants can be grown in a manner suitable for the species under consideration, either in a growth chamber, a greenhouse, or in a field. Transgenic plants can be bred as desired for a particular purpose, e.g., to introduce a recombinant nucleic acid into other lines, to transfer a recombinant nucleic acid to other species or produce the desired polypeptide. Alternatively, transgenic plants can be propagated vegetatively for those species amenable to such techniques. Progeny includes descendants of a particular plant or plant line provided the progeny inherits the transgene. Progeny of an instant plant include seeds formed on F₁, F₂, F₃, F₄, F₅, F₆ and subsequent generation plants, or seeds formed on BC₁, BC₂, BC₃, and subsequent generation plants, or seeds formed on F₁BC₁, F₁BC₂, F₁BC₃, and subsequent generation plants. Seeds produced by a transgenic plant can be grown and then selfed (or outcrossed and selfed) to obtain seeds homozygous for the exogenous nucleic acid.

Transgenic plant cells growing in suspension culture, or tissue or organ culture, can be useful for extraction of polypeptides. Solid and/or liquid tissue culture techniques can be used. When using solid medium, transgenic plant cells can be placed directly onto the medium or can be placed onto a filter film that is then placed in contact with the medium. When using liquid medium, transgenic plant cells can be placed onto a floatation device, e.g., a porous membrane that contacts the liquid medium. Solid medium typically is made from liquid medium by adding agar. For example, a solid medium can be Murashige and Skoog (MS) medium containing agar and a suitable concentration of an auxin, e.g., 2,4-dichlorophenoxyacetic acid (2,4-D), and a suitable concentration of a cytokinin, e.g., kinetin.

In some variants, the disclosure describes a method wherein *Agrobacterium* cells comprise a vector comprising sequence elements, which are essential for maintenance and replication of the plasmid in *Escherichia coli* and/or *Agrobacterium* cells, and for the transfer of a T-DNA to a plant cell, and further a T-DNA region, comprising the coding sequence of a polypeptide that is under control of regulatory elements functional in a plant and, optionally, a plant selectable marker gene.

In any of the transformation methods, the vector can include a plant selectable marker such as an antibiotic-based selectable marker (e.g., spectinomycin, kanamycin, streptomycin). A plant selectable marker can be a fluorescent protein marker and/or a colorimetric marker (e.g., LacZ). In some instances, a plant selectable marker can comprise peptide deformylase. Peptide deformylase can hydrolyze the N-formyl group on an initiating methionine. Peptide deformylase activity can be essential for cell viability. A peptide deformylase can originate from a number of genes including DEF1 and DEF2. Plants expressing a peptide deformylase selectable marker can be resistant to peptide deformylase inhibitors (e.g., actinonin). Selectable marker genes can be excised. Excision can occur by site-specific recombination (e.g., Cre, Int recombination), transposons (e.g., Ac/Ds family transposons), meganucleases (e.g., homing endonucleases, I-sceI), intrachromosomal homologous recombination, the Cas9/CRISPR endonucleases, and/or zinc fingers. See, for example, the below discussion re homologous recombination.

In some instances, site specific breakage in the plant genome is utilized either to greatly enhance targeted homologous-recombination-based mutation/replacement of endogenous sequences (i.e., to reprogram a globin gene) or to greatly enhance mutation or recombination rates at specific sites (e.g., promoter of globin genes or promoter of globin genes/promoter of a highly endosperm-expressed gene to retarget the globin expression to seeds or other targeted tissues). Site specific breakage can occur by TALENS (plant-transcription factor derived endonucleases that exploit a simple system for designing DNA-recognition elements to create synthetic endonucleases with sufficiently high specificity to cleave a single genomic site in vivo) or the Cas9/Crispr system.

In some instances, a vector can comprise one or more of the following nucleic acid elements: a) a first nucleic acid element comprising a nucleotide sequence encoding a selectable marker (e.g., which can be functional in *Escherichia coli* and/or *Agrobacterium* species); b) a second nucleic acid element comprising a nucleotide sequence of a first origin of replication which can be functional in *Escherichia coli;* c) a third nucleic acid element comprising a nucleotide sequence encoding a replication initiator protein; and/or d) a fourth nucleic acid element comprising a nucleotide sequence of a second origin of replication, which can be different from the first origin of replication and which is functional in*Agrobacterium,* wherein the above nucleic acid elements are provided on a circular polynucleotide molecule and are separated by gap nucleotide sequences which have no function in replication, maintenance or nucleic acid transfer, and wherein said gap nucleotide sequences account for less than 20%, 25%, 30%, 35%, 40%, or 45% of the total vector size.

In another embodiment, the disclosure relates to a method, wherein the regulatory sequences operable in a plant or a plant cell include a promoter that can drive and/or control expression of a gene of interest. Suitable promoters can include mirabilis mosaic virus (MMV), figwort mosaic virus (FMV) or Peanut Chlorotic Streak Caulimovirus (PCLSV) promoters. Other examples of suitable promoters can include a cauliflower mosaic virus 35S promoter, a modified cauliflower mosaic virus 35S promoter, a double cauliflower mosaic virus 35S promoter, a minimal 35 S promoter, nopaline synthase promoter, a cowpea mosaic virus promoter, a HT-CPMV promoter, a tobacco copalyl synthase CPS2p promoter, a dihydrinin promoter, a plastocyanin promoter, a 35S/HT-CPMV promoter, full length transcript (FLt) promoters, sub-genomic transcript promoters, and many other promoters that are derived from DNA viruses belonging to the Caulimoviridae virus family.

Many such promoters can be modified by linking multiple copies, for example two copies, of their enhancer sequence in tandem to enhance the promoter activity, such as but not limited to double CaMV 35S promoter (35Sx2), double MMV promoter (MMVx2), or double FMV promoter (FMVx2). Functional fragments of these promoters can be used in the vector of the disclosure. Nucleotide sequences that are at least 90%, 95%, 96%, 97%, 98%, 99% or 100% identical to these promoter sequences and that are functional in enabling expression in plants of the operably linked nucleotide sequence can also be used in the vectors of the disclosure.

Plant expression vectors which can be functional in a plant cell and may be used within the method of the present disclosure in order to drive and/or control expression of a gene of interest in a plant may also comprise, if desired, a promoter regulatory region (for example, one conferring inducible or constitutive, environmentally- or developmentally- regulated, or cell- or tissue-specific expression), a transcription initiation start site, a ribosome binding site, an RNA processing signal, a transcription termination site, and/or a polyadenylation signal. The regulatory elements to be used within the methods of the disclosure may be present in a vector molecule (e.g., binary vector) operably linked to a nucleotide sequence encoding a polypeptide of the disclosure. In some cases, a regulatory element can be present in the T-DNA region of a binary vector (e.g., a minimally sized binary vector).

In some instances the promoters controlling gene expression do so in a tissue-dependent manner and according to the developmental stage of the plant. The transgene sequences of the disclosure driven by these type of promoters can be expressed in tissues where the transgene product is desired, leaving the rest of the tissues in the plant unmodified by transgene expression. Tissue-specific promoters may be induced by endogenous or exogenous factors. Tissue specific examples can include but are not limited to beta-amylase gene or barley hordein gene promoters (for seed gene expression), tomato pz7 and pz130 gene promoters (for ovary gene expression), tobacco RD2 gene promoter (for root gene expression), banana TRX promoter and melon actin promoter (for fruit gene expression). In some variants, the tissue specific promoters can be chosen to target expression of the polypeptide to bulky and easily harvestable parts of the plant such as the seeds, fruits, tubers and leaves.

Plant expression vectors may further comprise a nucleotide sequence encoding a signal peptide that can target the newly expressed protein to a subcellular location. Signal peptides that may be used within such vector molecules can be, for example, a vacuolar targeting sequence, a chloroplast targeting sequence, a mitochondrial targeting sequence, a sequence that induces the formation of protein bodies in a plant cell, a sequence that induces specific targeting of the protein fused there onto to a specific organelle within the plant or plant cell, or a sequence that induces the formation of oil bodies in a plant cell.

In some instances, the targeting sequence can be a signal peptide for export of a protein into the extracellular space. Signal peptides can be transit peptides that are located at the extreme N-terminus of a protein and cleaved co-translationally during translocation across a plasma membrane.

In some variants, the signal peptide may be a sequence that when fused to a protein results in the formation of non-secretory storage organelles in the endoplasmatic reticulum.

Endogenous nucleic acids can be modified by homologous recombination techniques. For example, sequence specific endonucleases (e.g., zinc finger nucleases (ZFNs)) and meganucleases can be used to stimulate homologous recombination at endogenous plant genes. See, e.g., Townsend et al., Nature 459:442-445 (2009); Tovkach et al., Plant J., 57:747-757 25 (2009); and Lloyd et al., Proc. Natl. Acad. Sci. USA, 102:2232-2237 (2005). CRISPR Cas (Xie and Yang, Mol. Plant 2013, 6:1975-1983) and TALEN (Zhang et al., Plant Physiology 2013 161:20-27) genome editing techniques also can be used to replace an endogenous nucleic acid.

In some variants, the vector can further comprise in the T-DNA region a site-specific recombination site for site-specific recombination. In some embodiments, the site-specific recombination site can be located downstream of the plant regulatory element. In some embodiments, the site-specific recombination site can be located upstream of the plant regulatory element. In some variants, the recombination site can be a LoxP site and part of a Cre-Lox site-specific recombination system. The Cre-Lox site-specific recombination system can use a cyclic recombinase (Cre), which can catalyze the recombination between specific sites (LoxP) that comprise specific binding sites for Cre.

In some variants, the recombination site can be a Gateway destination site. For example, polynucleotides can be cloned into a commercially available "entry vector" and subsequently recombined into a "destination vector". The destination vector can be used for the analysis of promoter activity of a given nucleic acid sequence or number of sequences, for analysis of function, for protein localization, for protein-protein interaction, for silencing of a given gene or for affinity purification experiments. The Gateway cloning technology can be purchased from Invitrogen Inc., USA.

In some variants targeted lesions are created by homologous recombination or other gene editing techniques in the vicinity of an endogenous nucleic acid encoding a hemoprotein or globin and in the vicinity of desired tissue specific promoters and mutants are screened for expression of the desired endogenous hemoprotein or globin in abundant, accessible tissues. In some variants, targeted lesions are created in the vicinity of desired tissue specific promoters and mutants are screened for the expression of the desired endogenous hemoprotein or globin in abundant, accessible tissues. Examples of methods for creating these targeted lesions include but are not limited to TALENS and the cas9/crispr system as discussed above.

In some variants, the resultant plants may contain more target protein than the original plant. For example the resultant plant may express more than 2X the level of the target native protein compared to the original plant. In some variants, the resultant plant may express the native target protein in a tissue, such as the seed or leaf, in which it is not normally found. The native target protein may be expressed in a tissue that it is not normally found at 1 fold, 2 fold, 3 fold, 4, fold, 5 fold, 6 fold, 7 fold, 8 fold, 9 fold, 10 fold or more higher or lower than the levels of the native target protein in the tissue in which it is normally found. In some variants the target protein is a hemoglobin such as the leghemoglobin of *Glycine max* (e.g., SEQ ID NO:4) or the nonsymbiotic hemoglobin of barley (SEQ ID NO: 5) produced in engineered soy or barley plants respectively.

In some variants the resultant plant contains no foreign DNA. In some instances, lesions in the vicinity of the endogenous polypeptide of the disclosure and/or tissue specific promoters can be used for the engineering the expression of the polypeptide of the disclosure. Methods for assessing this type of engineering can include screening for production of the endogenous polypeptide.

### Methods of Expression in Bacteria

The disclosure describes methods for expression of a polypeptide (e.g., globin) in a host cell (e.g., bacteria). Suitable bacteria for expression of a polypeptide of the disclosure can be gram negative or gram positive bacteria. For example, the bacteria can be a species of *Escherichia* (e.g., *E. coli*) or a species of *Bacillus* (e.g., *B. subtilis, B. licheniformis, B. lentus, B. brevis, B. stearothermophilus, B. alkalophilus, B. amyloliquefaciens*, *B. clausii, B. coagulans, B. circulans, B. lautus, B. megaterium,* or *B. thuringiensis*). In some instances, the bacteria suitable for expression of a polypeptide of the disclosure can be *B. subtilis.*

In some instances, expression of a polypeptide can include introducing a vector comprising a polynucleotide sequence encoding the polypeptide into the host cell, and inducing expression of the polypeptide.

In some variants, the methods of the disclosure provide for a host cell that comprises a stably integrated sequence of interest (i.e., polypeptide-encoding nucleic acid). However, in alternative variants, the methods of the present disclosure describe maintenance of a self-replicating extra-chromosomal transformation vector.

Methods of introducing the polynucleotide into cells for expression of the polynucleotide sequence can include, but are not limited to electroporation, transformation, transduction, high velocity bombardment with DNA-coated microprojectiles, infection with modified viral (e.g., phage) nucleic acids; chemically-mediated transformation, or competence. In some instances, polynucleotides encoding a polypeptide of the disclosure can be transcribed in vitro, and the resulting RNA can be introduced into the host cell.

Following introduction of a polynucleotide comprising the coding sequence for a polypeptide of the disclosure, the host cell can be cultured in conventional nutrient media modified as appropriate for activating promoters, selecting transformants, and/ or amplifying expression of a polypeptide-encoding polynucleotide. The culture conditions, such as temperature, pH and the like, can be those previously used for the host cell selected for expression. The progeny of cells into which such polynucleotide constructs have been introduced can be considered to comprise the polypeptide-encoding polynucleotide.

In some variants, the polypeptide or variant thereof can be expressed as a fusion protein by the host bacterial cell. Although cleavage of the fusion polypeptide to release the desired protein can often be useful, it is not necessary. Polypeptides and variants thereof expressed and secreted as fusion proteins can retain their function.

Expression of a polypeptide of the disclosure can comprise transient expression and/or constitutive expression (e.g., developing of a stable cell line).

### Expression of Recombinant Polypeptides

Expression of a polypeptide can comprise inducing the host cell to transcribe and/or translate the polypeptide encoded in the polynucleotide introduced to the host cell. Induction can occur after the host cell has been cultured for at least about 0.1, 0.5, 1, 2, 3, 4, 5, 6, 7, 8, 9, 10, 15 or 20 or more hours. Induction can occur after the host cell culture has an optical density (OD) of at least about 0.1, 0.2, 0.3, 0.4, 0.5, 0.6, 0.7, 0.8, 0.9, 1.0, 1.1, 1.2, 1.3, 1.4, or 1.5 or more. Induction can occur after the host cell culture has an optical density (OD) of at most about 0.1, 0.2, 0.3, 0.4, 0.5, 0.6, 0.7, 0.8, 0.9, 1.0, 2, 3, 5, 10, or 20 or more. Induction may be caused by addition of chemicals such as IPTG, arabinose or in response to a limiting nutrient such as Nitrogen, phosphorus, glucose or oxygen. In some instances the polypeptide is linked to a promoter, such as aprE, liaG, lepA, cry3Aa, or gsiB that leads to constitutive expression of the polypeptide.

In some instances, chemical agents can be added to the media. In some instances, the chemical agents can aid the stability, heme content, and/or protein folding capability of the expressed polypeptide. A chemical agent can comprise a small molecule such as a metal. Examples of suitable metals for addition to media can include iron fluorides (iron difluoride, iron trifluoride), iron dichloride, iron trichloride, iron dibromide, iron tribromide, iron diiodide, iron triiodide, iron oxide, diiron trioxide, triiron tetraoxide, iron sulfide, iron persulfide, iron selenide, iron telluride, di-iron nitride, iron pentacarbonyl, diiron nonacarbonyl, triiron dodecacarbonyl, iron dichloride dihydrate, iron trifluoride trihydrate, iron dibromide hexahydrate, iron dichloride tetrahydrate, iron nitrate hexahydrate, iron trichloride hexahydrate, iron difluoride tetrahydrate, iron sulphate heptahydrate, iron trinitrate nonahydrate, diiron trisulfate nonahydrate, iron chromate, iron citrate, iron gluconate, magnesium iron hexahydride, iron lactate, iron phosphate, iron pentacarbonyl, ammonium iron sulfate, ammonium ferric citrate, ferric oxalate, and triiron diphosphate octahydrate.

A chemical agent can be added to the media at a final concentration of at least about 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 5.0, 10.0 or more millimolar. A chemical agent can be added to the media at a final concentration of at most about 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 5.0, 10.0 or more millimolar.

In some instances, a chemical agent can be a heme derivative. A heme derivative can increase the heme content of the expressed polypeptide (e.g., increase the number of globin molecules that comprise a heme). Suitable heme derivatives can include delta-aminolevulinic acid, derivatives of heme A, derivatives of heme B, derivatives of heme C, derivatives of heme O, heme precursors, derivatives of heme I, derivatives of heme m, derivatives of heme D, and derivatives of heme S. A heme derivative can be added to the media at a final concentration of at least about 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 5.0, 10.0 or more millimolar. A heme derivative can be added to the media at a final concentration of at most about 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 5.0, 10.0 or more millimolar. In some instances, no heme derivative is added to the media.

After inducing the polypeptide, the host cell can be cultured for a period of time favoring maximal expression levels of the polypeptide. For example, a polypeptide can be expressed for at least about 0.1 hours, 0.5 hours, 1 hour, 2 hours, 3 hours, 4 hours, 5 hours, 6 hours, 7 hours, 8 hours, 9 hours, 10 hours, 11 hours, 12 hours, 13 hours, 14 hours, 15 hours, 16 hours, 17 hours, 18 hours , 19 hours, 20 hours, 21 hours, 22 hours, 23 hours, 24 hours, 1 days, 2 days, 3 days, 4 days, 5 days, 6 days, 7 days, 1 week, 2 weeks, 3 weeks, 4 weeks, 5 weeks, 6 weeks, 7 weeks, 8 weeks, 9 weeks, 10 or more weeks. A polypeptide can be expressed in a host cell for at most about at least about 0.1 hours, 0.5 hours, 1 hour, 2 hours, 3 hours, 4 hours, 5 hours, 6 hours, 7 hours, 8 hours, 9 hours, 10 hours, 11 hours, 12 hours, 13 hours, 14 hours, 15 hours, 16 hours, 17 hours, 18 hours , 19 hours, 20 hours, 21 hours, 22 hours, 23 hours, 24 hours, 1 days, 2 days, 3 days, 4 days, 5 days, 6 days, 7 days, 1 week, 2 weeks, 3 weeks, 4 weeks, 5 weeks, 6 weeks, 7 weeks, 8 weeks, 9 weeks, or 10 or more weeks.

A polypeptide can be expressed at a variety of temperatures. A polypeptide can be expressed at a temperature of at least about 4, 10, 16, 18, 21, 25, 26, 27, 28, 29, 30, 31, 32, 33, 34, 35, 36, 37, 38, 39, 40, 42, 42, 43, 44, 45, 46, 47, 48, 49, or 50°C. A polypeptide can be expressed at a temperature of at most about 4, 10, 16, 18, 21, 25, 26, 27, 28, 29, 30, 31, 32, 33, 34, 35, 36, 37, 38, 39, 40, 42, 42, 43, 44, 45, 46, 47, 48, 49, or 50°C.

Accessory proteins such as thiol-disulfide oxidoreductases or chaperones may be beneficial to help fold the polypeptide into its active conformation. Thiol-disulfide oxidoreductases and protein disulfide isomerases can catalyze the formation of the correct disulfide bonds in the protein. Expression of the bdbDC operon in *B. subtilis* has been shown to be beneficial for the production of a polypeptide with disulfide bonds. Chaperones can help the secretory protein to fold by binding to exposed hydrophobic regions in the unfolded states and preventing unfavorable interactions and prolyl-peptidyl cis-trans isomerases assist in formation of the proper conformation of the peptide chain adjacent to proline residues. In some variants, the host cells can be transformed with an expression vector encoding at least one thiol-disulfide oxidoreductase or chaperone.

In some instances, the fraction of properly folded polypeptide can be increased by the addition of chemicals to the growth medium that reduce/oxidize disulfide bonds, and/or alter the general redox potential, and/or chemicals that alter solvent properties thus affecting protein conformation and aggregation. In some instances, a reagent that reduces disulfide bonds, such as 2-mercaptoethanol, can increase the fraction of correctly folded protein. In some cases and depending on the medium used, other disulfide reducing or oxidizing agents (e.g., DTT, TCEP, reduced and oxidized glutathione, cysteine, cystine, cysteamine, thioglycolate, S₂0₃², S₂0₄², S₂0₅², S0₃², S₂0₇², Cu+, etc. ), either used alone or in combination, can find use in the present disclosure. It can be contemplated that other adjuvants that alter solvent properties, (e.g., urea, DMSO, TWEEN^{®}-80, etc. ), either added to the growth medium alone or preferably in combination with disulfide reducing/oxidizing agents, such as βME, can also increase the fraction of correctly folded secretory protein and find use in various cases of the present disclosure . In some cases, the βME can be used at concentrations ranging from 0.5 to 4 mM, or from about 0.1 mM to 10 mM.

The polypeptide can be recovered from the culture (e.g., by centrifugation, purification, etc.), as described below and herein.

### Fermentation Parameters

The present disclosure describes fermentation procedures for culturing bacterial species. Culturing can be accomplished in a growth medium comprising an aqueous mineral salts medium, organic growth factors, the carbon and energy source material, molecular oxygen (for aerobic and facultative bacteria), and, of course, a starting inoculum of one or more particular microorganism species to be employed.

In addition to the carbon and energy source, oxygen, assimilable nitrogen, and an inoculum of the microorganism, it can be necessary to supply suitable amounts in proper proportions of mineral nutrients to assure proper microorganism growth, maximize the assimilation of the carbon and energy source by the cells in the microbial conversion process, and achieve maximum cellular yields with maximum cell density in the fermentation medium.

Various culture media can be used. Standard bacterial culture media can be used. The media can include, in addition to nitrogen, suitable amounts of phosphorus, magnesium, calcium, potassium, sulfur, and sodium, in suitable soluble assimilable ionic and combined forms, and can also comprise certain trace elements such as copper, manganese, molybdenum, zinc, iron, boron, and iodine, and others, again in suitable soluble assimilable form.

In some instances, the fermentation reaction can involve an aerobic process in which the molecular oxygen needed can be supplied by a molecular oxygen-containing gas such as air, oxygen-enriched air, or even substantially pure molecular oxygen, provided to maintain the contents of the fermentation vessel with a suitable oxygen partial pressure effective in assisting the microorganism species to grow in a thriving fashion. In effect, by using an oxygenated hydrocarbon substrate, the oxygen requirement for growth of the microorganism can be reduced. Nevertheless, molecular oxygen can be supplied for growth of aerobic and to a lesser extent, facultative organisms.

Although the aeration rate can vary over a considerable range, aeration generally can be conducted at a rate that is in the range from about 0.5 to 10 or from about 0.5 to 7 volumes (at the pressure employed and at 25°C.) of oxygen-containing gas per liquid volume in the fermenter per minute. This amount can be based on air of normal oxygen content being supplied to the reactor, and in terms of pure oxygen the respective ranges can be from about 0.1 to 1.7, or from about 0.1 to 1.3, volumes (at the pressure employed and at 25°C) of oxygen per liquid volume in the fermenter per minute.

The pressure employed for the microbial conversion process can range widely. Pressures generally can be within the range of about 0 to 50 psig, from about 0 to 30 psig, or at least slightly over atmospheric pressure, as a balance of equipment and operating cost versus oxygen solubility achieved. Greater than atmospheric pressures can increase a dissolved oxygen concentration in the aqueous ferment, which in turn can help increase cellular growth rates.

The fermentation temperature can vary somewhat, but for most bacterial host species used in the present, the temperature generally can be within the range of from about 20°C to 40°C, or in the range of from about 28°C to 37°C, depending on the strain of microorganism chosen.

In some instances, the microorganisms may require a source of assimilable nitrogen. The source of assimilable nitrogen can be any nitrogen-containing compound or compounds capable of releasing nitrogen in a form suitable for metabolic utilization by the microorganism. While a variety of organic nitrogen source compounds, such as protein hydrolysates, can be employed, usually cheap nitrogen-containing compounds such as ammonia, ammonium hydroxide, urea, and various ammonium salts such as ammonium phosphate, ammonium sulfate, ammonium pyrophosphate, ammonium chloride, or various other ammonium compounds can be utilized. Ammonia gas itself can be convenient for large-scale operations, and can be employed by bubbling through the aqueous ferment (fermentation medium) in suitable amounts. At the same time, such ammonia can also be employed to assist in pH control.

The pH range in the aqueous microbial ferment (fermentation admixture) can be in the exemplary range from about 2.0 to 8.0. However, pH range optima for certain microorganisms can be dependent on the media employed to some extent, as well as the particular microorganism, and thus change somewhat with change in media.

The average retention time of the fermentation admixture in the fermenter can vary considerably, depending in part on the fermentation temperature and culture employed. In some variants, the fermentation can be conducted in such a manner that the carbon-containing substrate can be controlled as a limiting factor, thereby providing good conversion of the carbon-containing substrate to cells and avoiding contamination of the cells with a substantial amount of unconverted substrate. The latter may not be a problem with water-soluble substrates, since any remaining traces can be readily removed. It may be a problem, however, in the case of non-water-soluble substrates, and may use added product-treatment steps such as suitable washing steps. The time needed to reach this limiting substrate level may vary with the particular microorganism and fermentation process being conducted. The fermentation can be conducted as a batch or continuous operation, fed batch operation can be used for ease of control, production of uniform quantities of products, and most economical uses of all equipment.

If desired, part or all of the carbon and energy source material and/or part of the assimilable nitrogen source such as ammonia can be added to the aqueous mineral medium prior to feeding the aqueous mineral medium into the fermenter. Indeed, each of the streams introduced into the reactor can be controlled at a predetermined rate, or in response to a need determinable by monitoring such as concentration of the carbon and energy substrate, pH, dissolved oxygen, oxygen or carbon dioxide in the off-gases from the fermenter, cell density measurable by light transmittance, or the like. The feed rates of the various materials can be varied so as to obtain as rapid a cell growth rate as possible, consistent with efficient utilization of the carbon and energy source, to obtain as high a yield of microorganism cells relative to substrate charge as possible, and to obtain the highest production of the desired protein per unit volume.

In a batch, equipment, reactor, or fermentation means, vessel or container, piping, attendant circulating or cooling devices, and the like, can be initially sterilized, usually by employing steam at about 121°C for at least about 15 minutes. The sterilized reactor can be inoculated with a culture of the selected microorganism in the presence of all the required nutrients, including oxygen, and the carbon-containing substrate.

### Methods of Secretion in bacteria

In some instances, an expressed polypeptide can be secreted from a host cell (e.g., bacteria). Secretion of a polypeptide can comprise releasing the polypeptide from a cell or subcellular compartment in a cell (e.g., nucleus, cell wall, plasma membrane). Secretion can occur through plasma membranes, which can surround cells and/or subcellular compartments. In some instances, secretion can refer to releasing a polypeptide to the cell envelope. In some instances, secretion can refer to releasing a polypeptide to the extracellular space (e.g., into the culture media).

A host cell of the disclosure can comprise secretory pathways, which can comprise a number of proteins that function together to secrete a protein. In some instances, the host cell can comprise a twin-arginine translocation (TAT) secretory pathway. In some instances, an organism can comprise a SEC secretory pathway. The TAT secretory pathway can comprise secretion of polypeptides (e.g., globins) in a folded state. The TAT secretory pathway can transport proteins across a plasma membrane (e.g., lipid layer, i.e., lipid bilayer).

The disclosure describes secretion factors and methods that can be used in host cells to ameliorate the bottleneck to protein secretion and the production of proteins in secreted form, in particular when the polypeptides are recombinantly introduced and expressed by the host cell. The present disclosure describes the secretion factors TatC and TatA derived from *Bacillus subtilis.* In particular, the TatAdCd and TatAyCy peptide, as well as the genes encoding them are also suitable secretion factors. PhoD of *B. subtilis,* can be secreted via the twin-arginine translocation pathway. TatAdCd is of major importance for the secretion of PhoD, whereas TatAyCy may not be required for this process.

### Expression of polypeptides in plants

A polypeptide can be expressed in monocot plants and/or dicot plants. Techniques for introducing nucleic acids into plants are known in the art, and include, without limitation, *Agrobacterium*-mediated transformation, viral vector-mediated transformation, electroporation, and particle gun transformation (also referred to as biolistic transformation). See, for example, U.S. Patent Nos. 5,538,880; 5,204,253; 6,329,571; and 6,013,863; Richards et al., Plant Cell. Rep. 20:48-20 54 (2001); Somleva et al., Crop Sci. 42:2080-2087 (2002); Sinagawa-Garcia et al., Plant Mol Biol (2009) 70:487-498; and Lutz et al., Plant Physiol., 2007, Vol. 145, pp. 1201-1210. In some instances, intergenic transformation of plastids can be used as a method of introducing a polynucleotide into a plant cell. In some instances, the method of introduction of a polynucleotide into a plant comprises chloroplast transformation. In some instances, the leaves and/or stems can be the target tissue of the introduced polynucleotide. If a cell or cultured tissue is used as the recipient tissue for transformation, plants can be regenerated from transformed cultures if desired, by techniques known to those skilled in the art.

Other suitable methods for introduce polynucleotides include electroporation of protoplasts, polyethylene glycol-mediated delivery of naked DNA into plant protoplasts, direct gene transformation through imbibition (e.g., introducing a polynucleotide to a dehydrated plant), transformation into protoplasts (which can comprise transferring a polynucleotide through osmotic or electric shocks), chemical transformation (which can comprise the use of a polybrene-spermidine composition), microinjection, pollen-tube pathway transformation (which can comprise delivery of a polynucleotide to the plant ovule), transformation via liposomes, shoot apex method of transformation (which can comprise introduction of a polynucleotide into the shoot and regeneration of the shoot), sonication-assisted agrobacterium transformation (SAAT) method of transformation, infiltration (which can comprise a floral dip, or injection by syringe into a particular part of the plant (e.g., leaf)), silicon-carbide mediated transformation (SCMT) (which can comprise the addition of silicon carbide fibers to plant tissue and the polynucleotide of interest), electroporation, and electrophoresis.

A polypeptide can be expressed in many different plant species, including, for example, grains such as, e.g., corn, maize, oats, rice, wheat, barley, rye, triticale, teff, oilseeds including cottonseed, sunflower seed, safflower seed, crambe, camelina, mustard, rapeseed, leafy greens such as, e.g., lettuce, spinach, kale, collard greens, turnip greens, chard, mustard greens, dandelion greens, broccoli, cabbage, sugar cane, trees, root crops such as cassava, sweet potato, potato, carrots, beets, turnips, plants from the legume family, such as, e.g., clover, peas such as cowpeas, English peas, yellow peas, green peas, beans such as, e.g., soybeans, fava beans, lima beans, kidney beans, garbanzo beans, mung beans, pinto beans, lentils, lupins, mesquite, carob, soy, and peanuts, coconut, vetch (vicia), stylo (stylosanthes), arachis, indigofera, acacia, leucaena, cyamopsis, and sesbania. Plants not ordinarily consumed by humans, including biomass crops, including switchgrass, miscanthus, tobacco, *Arundo donax,* energy cane, sorghum, other grasses, alfalfa, corn stover, kelp, or other seaweeds. Polypeptides that can be found in any organism in the plant kingdom may be used in the present disclosure. In some instances, the plant can be soy (*Glycine max*). In some instances, the plant can be barley (*Hordeum vulgare*). In some instances, the plant can be *Nicotiana tabacum.* In some instances, the plant or plant cell is not a *Nicotiana* plant or plant cell.

In some instances, the *Nicotiana tabacum* variety, breeding line, or cultivar can be *N. tabacum* accession PM016, PM021, PM92, PM102, PM132, PM204, PM205, PM215, PM216 or PM217 as deposited with NCIMB, Aberdeen, Scotland, or DAC Mata Fina, P02, BY-64, AS44, RG17, RG8, HB04P, Basma Xanthi BX 2A, Coker 319, Hicks, McNair 944 (MN 944), Burley 21, K149, Yaka JB 125/3, Kasturi Mawar, NC 297, Coker 371 Gold, P02, Wislica, Simmaba, Turkish Samsun, AA37-1, B13P, F4 from the cross BU21 x Hoja Parado line 97, Samsun NN, Izmir, Xanthi NN, Karabalgar, Denizli and P01.

Expression of a polypeptide of the disclosure can comprise transient expression and/or constitutive expression (e.g., developing of a stable cell line).

### Agrobacterium species and strains

The disclosure describes Agrobacterium strains for use in methods for producing a polypeptide by expression of an expressible sequence (e.g., a sequence encoding a polypeptide of the disclosure). One of the Agrobacterium strains can be used to infiltrate a preselected plant variety in order to optimize the yield of the polypeptide. In certain instances, the Agrobacterium species that may be used in method according to the disclosure can include but are not limited to *Agrobacterium tumefaciens, Agrobacterium rhizogenes Agrobacterium radiobacter, Agrobacterium rubi, Argobacterium vitis.* In some variants, at least one Agrobacterium strain comprises *Agrobacterium tumefaciens*. The Agrobacterium species used can be a wild type (e.g., virulent) or a disarmed strain. Suitable strains of Agrobacterium can include wild type strains (e.g., such as *Agrobacterium tumefaciens*) or strains in which one or more genes is mutated to increase transformation efficiency, (e.g., such as Agrobacterium strains wherein the vir gene expression and/or induction thereof is altered due to the presence of mutant or chimeric virA or virG genes), Agrobacterium strains comprising an extra virG gene copies, such as the super virG gene derived from pTiBo542, linked to a multiple-copy plasmid. Other suitable strains can include, but are not limited to: *A. tumefaciens* C58C1, A136; LBA401 1, LBA4404; EHA101; EHA105; AGL1; and A281. In some cases, the selected Agrobacterium strain can be AGL1, EHA105, GV2260, GV3101, or Chry5.

In some instances, multiple suspensions of Agrobacterium cells, each expressing different genes can be used to produce the polypeptide, or to enhance the level of expression of a polypeptide of the disclosure. In such instances, it is contemplated that the Agrobacterium cells in the different suspensions of Agrobacterium cells can be the same strain or different strains. Alternatively, or additionally, a single Agrobacterium strain may comprise a plurality of sequences comprising different polynucleotides, particularly polynucleotides encoding polypeptides of the disclosure. The different genes may be comprised within a single nucleic acid molecule (e.g., a single vector) or may be provided in different vectors. A non-limiting example of a second gene that can be expressed in the host plant is a gene that encodes a suppressor of silencing, of viral origin.

Expression of a polypeptide in a host cell (e.g., plant cell), can occur for at least about 0.1 hours, 0.5 hours, 1 hour, 2 hours, 3 hours, 4 hours, 5 hours, 6 hours, 7 hours, 8 hours, 9 hours, 10 hours, 11 hours, 12 hours, 13 hours, 14 hours, 15 hours, 16 hours, 17 hours, 18 hours, 19 hours, 20 hours, 21 hours, 22 hours, 23 hours, 24 hours, 1 days, 2 days, 3 days, 4 days, 5 days, 6 days, 7 days, 1 week, 2 weeks, 3 weeks, 4 weeks, 5 weeks, 6 weeks, 7 weeks, 8 weeks, 9 weeks, 10 weeks, 15 weeks, 20 weeks, 30 or more weeks. A polypeptide can be expressed in a host cell for at most about at least about 0. 1 hours, 0.5 hours, 1 hour, 2 hours, 3 hours, 4 hours, 5 hours, 6 hours, 7 hours, 8 hours, 9 hours, 10 hours, 11 hours, 12 hours, 13 hours, 14 hours, 15 hours, 16 hours, 17 hours, 18 hours , 19 hours, 20 hours, 21 hours, 22 hours, 23 hours, 24 hours, 1 days, 2 days, 3 days, 4 days, 5 days, 6 days, 7 days, 1 week, 2 weeks, 3 weeks, 4 weeks, 5 weeks, 6 weeks, 7 weeks, 8 weeks, 9 weeks, or 10 weeks, 15 weeks, 20 weeks, 30 or more weeks.

A polypeptide can be expressed at a variety of temperatures. A polypeptide can be expressed at a temperature of at least about 4, 10, 16, 18, 21, 25, 26, 27, 28, 29, 30, 31, 32, 33, 34, 35, 36, 37, 38, 39, 40, 42, 42, 43, 44, 45, 46, 47, 48, 49, or 50°C. A polypeptide can be expressed at a temperature of at most about 4, 10, 16, 18, 21, 25, 26, 27, 28, 29, 30, 31, 32, 33, 34, 35, 36, 37, 38, 39, 40, 42, 42, 43, 44, 45, 46, 47, 48, 49, or 50°C.

### Enhancement of endogenous polypeptides

The disclosure describes enhanced production of an endogenous polypeptide (e.g., an endogenous heme-containing polypeptide). Enhanced production of an endogenous polypeptide can be accomplished by modulating the pathway that produces the endogenous polypeptide. Modulation can refer to modulation of transcription, translation, subcellular localization, localization to different tissues, timing of expression, folding, affinity for binding partners, and the like. Modulation can occur at the DNA level (e.g., knock-out the gene, knock-in an enhancer/promoter element). Modulation can occur at the RNA level (e.g., silence the gene via RNA interference). Modulation can occur at the protein level (e.g., modulation by allosteric inhibitors, small molecule binders).

In some instances, modulation can refer to altering the activity and/or levels of the endogenous polypeptide by at least about 1 fold, 2-fold, 3-fold, 4-fold, 5-fold, 10-fold, 20-fold, 50-fold, or 100-fold or more higher or lower relative to the wild-type levels of the endogenous polypeptide. In some instances, modulation can refer to altering the activity and/or levels of the endogenous polypeptide by at most about 1 fold, 2-fold, 3-fold, 4-fold, 5-fold, 10-fold, 20-fold, 50-fold, or 100-fold or more higher or lower relative to the wild-type levels of the endogenous polypeptide. In some instances, modulation can refer to altering the activity and/or levels of the endogenous polypeptide by at least about 5, 10, 20, 30, 40, 50, 60, 70, 80, 90, or 100% of the wild-type levels of the endogenous polypeptide. In some instances, modulation can refer to altering the activity and/or levels of the endogenous polypeptide by at least about 5, 10, 20, 30, 40, 50, 60, 70, 80, 90, or 100% of the wild-type levels of the endogenous polypeptide.

In some instances, polypeptides in the heme biosynthesis pathway that can produce the heme cofactor can be modulated. See, for example, Tanaka and Tanaka, Annu. Rev. Plant Biol. 2007. 58:321-46, which describes modifications to the tetrapyrrole biosynthesis pathway in plants. The modulation of polypeptides in the heme biosynthetic pathway can be at the DNA, RNA, or protein level. In some instances, the modulation of other polypeptides in the pathway can refer to increasing the levels and/or activity of an activator of the pathway. In some instances, the modulation of other polypeptides in the pathway can refer to decreasing the levels and/or activity of a suppressor of the pathway.

In some instances, modulation can refer to altering the activity and/or levels of polypeptides in the heme biosynthesis pathway (including the heme cofactor that associates with a heme-containing polypeptide of the disclosure) by at least about 1 fold, 2-fold, 3-fold, 4-fold, 5-fold, 10-fold, 20-fold, 50-fold, or 100-fold or more higher or lower relative to the wild-type levels of the polypeptide in the pathway. In some instances, modulation can refer to altering the activity and/or levels of polypeptides in the heme biosynthesis pathway (including the heme cofactor) by at most about 1 fold, 2-fold, 3-fold, 4-fold, 5-fold, 10-fold, 20-fold, 50-fold, or 100-fold or more higher or lower relative to the wild-type levels of the polypeptide in the pathway. In some instances, modulation can refer to altering the activity and/or levels of polypeptides in the heme biosynthesis pathway (including the heme cofactor) by at least about 5, 10, 20, 30, 40, 50, 60, 70, 80, 90, or 100% of the wild-type levels of the polypeptide in the pathway. In some instances, modulation can refer to altering the activity and/or levels of polypeptides in the heme biosynthesis pathway (including the heme cofactor) by at least about 5, 10, 20, 30, 40, 50, 60, 70, 80, 90, or 100% of the wild-type levels of the polypeptide in the pathway.

In some instances, modulation can refer to altering the expression levels of an endogenous polypeptide in a specific location of the host cell. For example, the expression levels of an endogenous polypeptide can be altered in a leaf, a seed, a bean, a stalk, a xylem, a stamen, and a petal, or any combination thereof.

### Methods of purification

An expressed and/or secreted polypeptide of the disclosure may be recovered (e.g., from the culture medium or from a plant tissue). For example, when the expressed heme-containing polypeptide is secreted from the bacterial cells, the polypeptide can be purified from the culture medium. In some instances, the host cells expressing the polypeptide can be removed from the media before purification of the polypeptide (e.g., by centrifugation).

When the expressed recombinant desired polypeptide is not secreted from a host cell, the host cell can be disrupted and the polypeptide released into an aqueous "extract" which can be the first stage of purification. The expression host cells can be collected from the media before the cell disruption. The cell disruption may be performed by using any suitable means, such as by lysozyme or beta-glucanase digestion, grinding, sonication, homogenization, milling or by forcing the cells through high pressure.

A recovered polypeptide may be purified. Purification may be accomplished by means of a salt (e.g., ammonium sulfate) or low pH (typically less than 3) wash/fractionation or chromatographic procedures (e.g., ion exchange chromatography, affinity chromatography, hydrophobic interaction chromatography, hydrophobic charge induction chromatography, size exclusion chromatography etc.). During purification, the cumulative abundance by mass of protein components other than the specified protein, which can be a single monomeric or multimeric protein species, can be reduced by a factor of 2 or more, 3 or more, 5 or more, 10 or more, 20 or more, 50 or more, 100 or more or 1000 or more relative to the source material from which the specified protein was isolated.

In some instances, a polypeptide can be recovered from a fermenter. The fermentation broth can generally comprise cellular debris, including cells, various suspended solids and other biomass contaminants, as well as the desired protein product, which can be removed from the fermentation broth. Suitable processes for such removal can include conventional solid-liquid separation techniques (e.g., centrifugation, filtration, dialysis, microfiltration, rotary vacuum filtration, or other known processes), to produce a cell-free filtrate. In some variants, it can be acceptable to further concentrate the fermentation broth or the cell-free filtrate prior to the purification and/or crystallization process using techniques such as ultrafiltration, evaporation and/or precipitation. In some instances the polypeptide is further purified to reduce the cumulative abundance by mass of protein components other than the specified protein, which can be a single monomeric or multimeric protein species, by a factor of 2 or more, 3 or more, 5 or more, 10 or more, 20 or more, 50 or more, 100 or more or 1000 or more relative to the source material from which the specified protein was isolated. Purification may be accomplished by means of a salt (e.g., ammonium sulfate) or low pH (typically less than 3) wash/fractionation or chromatographic procedures (e.g., ion exchange chromatography, affinity chromatography, hydrophobic interaction chromatography, and/or hydrophobic charge induction chromatography etc).

### Characterization of a polypeptide

A purified polypeptide can be characterized for purity, heme content, oligmerization state, stability, degradation, binding affinity and the like. For some applications the polypeptides (e.g., globins) produced using the present disclosure can be very highly pure (e.g., having a purity of more than 99%). A purified polypeptide can be characterized for odor, taste and color.

The purified polypeptide can be at least about 1, 2, 3, 4, 5, 6, 7, 8, 9, 10, 20, 30, 40, 50, 60, 70, 80, 90, 91, 92, 93, 94, 95, 96, 97, 98, 99, or 100% pure. The purified polypeptide can be at most about 1, 2, 3, 4, 5, 6, 7, 8, 9, 10, 20, 30, 40, 50, 60, 70, 80, 90, 91, 92, 93, 94, 95, 96, 97, 98, 99, or 100% pure. The purified polypeptide can comprise at least about 0.01, 0.05, 0.1, 0.2, 0.3, 0.4, 0.5, 0.6, 0.7, 0.8, 0.9, 1, 2, 3, 4, 5, 6, 7, 8, 9, 10, 20, 30, 40, 50, 60, 70, 80, 90, 91, 92, 93, 94, 95, 96, 97, 98, or 99% impurities. The purified polypeptide can comprise at most about 0.01, 0.05, 0.1, 0.2, 0.3, 0.4, 0.5, 0.6, 0.7, 0.8, 0.9, 1, 2, 3, 4, 5, 6, 7, 8, 9, 10, 20, 30, 40, 50, 60, 70, 80, 90, 91, 92, 93, 94, 95, 96, 97, 98, or 99% impurities. The purified polypeptide can comprise at least about 0.001, 0.005, 0.01, 0.05, 0.1, 0.2, 0.3, 0.4, 0.5, 0.6, 0.7, 0.8, 0.9, 1, 2, 3, 4, 5, 6, 7, 8, 9, or 10 parts per million impurities. The purified polypeptide can comprise at most about 0.001, 0.005, 0.01, 0.05, 0.1, 0.2, 0.3, 0.4, 0.5, 0.6, 0.7, 0.8, 0.9, 1, 2, 3, 4, 5, 6, 7, 8, 9, or 10 parts per million impurities. The purified polypeptide can comprise at least about 0.001, 0.005, 0.01, 0.05, 0.1, 0.2, 0.3, 0.4, 0.5, 0.6, 0.7, 0.8, 0.9, 1, 2, 3, 4, 5, 6, 7, 8, 9, or 10 parts per billion impurities. The purified polypeptide can comprise at most about 0.001, 0.005, 0.01, 0.05, 0.1, 0.2, 0.3, 0.4, 0.5, 0.6, 0.7, 0.8, 0.9, 1, 2, 3, 4, 5, 6, 7, 8, 9, or 10 parts per billion impurities.

In some instances, the purified globin can be tested for activity, oligomerization state, proper protein folding, stability, secondary structure and/or heme content. Activity, oligomerization state, protein folding, and/or stability can be determined by a number of methods including spectroscopy, ELISA, binding assays, analytical ultracentrifugation, circular dichroism, x-ray crystallography, surface plasmon resonance, mass spectrometry, or NMR.

A polypeptide of this disclosure may have similar properties to myoglobin isolated from animal tissues. In one variant a group of people can be asked to rate a myoglobin isolated from an animal tissue, according to properties that describe the myoglobin. These ratings can be used as an indication of the properties of the animal tissue derived myoglobin. The polypeptide of the disclosure can then be compared to the animal derived globin to determine how similar the polypeptide of this disclosure is to the animal tissue derived myoglobin. So, in some embodiments, the polypeptide is rated similar to animal tissue derived myoglobin according to human evaluation. In some variants the polypeptide is indistinguishable from animal tissue derived myoglobin to a human.

In some instances the polypeptides of this disclosure are compared to animal tissue derived myoglobin based upon olfactometer readings. In various variants the olfactometer can be used to assess odor concentration and odor thresholds, odor suprathresholds with comparison to a reference gas, hedonic scale scores to determine the degree of appreciation, or relative intensity of odors. In some variants the olfactometer allows the training and automatic evaluation of expert panels. In some instances the consumable is a product that causes similar or identical olfactometer readings. In some variants the similarity is sufficient to be beyond the detection threshold of human perception.

Gas chromatography-mass spectrometry (GCMS) is a method that combines the features of gas-liquid chromatography and mass spectrometry to separate and identify different substances within a test sample. GCMS can, in some variants, be used to evaluate the properties of polypeptides of this disclosure. For example, volatile chemicals can be isolated from the head space around animal tissue derived myoglobin. These chemicals can be identified using GCMS. A profile of the volatile chemicals in the headspace around animal tissue derived myoglobin is thereby created. In some instances each peak of the GCMS can be further evaluated. For instance, a human could rate the experience of smelling the chemical responsible for a certain peak. This information could be used to further refine the profile. GCMS could then be used to evaluate the properties of a polypeptide of the disclosure. The GCMS profile could be used to refine the polypeptide.

Heme content can refer to the percentage of polypeptide molecules that comprise the correct amount of heme moieties. For example, if a polypeptide of the disclosure binds one heme moiety, then heme content can refer to the number of polypeptides that are bound to the heme moiety. Heme content of a polypeptide can be at least about 1, 2, 3, 4, 5, 6, 7, 8, 9, 10, 20, 30, 40, 50, 60, 70, 75, 80, 85, 90, 95, or 100%. Heme content of a polypeptide can be at most about 1, 2, 3, 4, 5, 6, 7, 8, 9, 10, 20, 30, 40, 50, 60, 70, 75, 80, 85, 90, 95, or 100%. In some instances, heme content can be expressed as a molar ratio of polypeptide concentration to heme concentration. The molar ratio heme content can be at least about 1:1, 1:2, 1:3, 1:4, 1:5, 1:6, 1:7, 1:8, 1:9, 1:10, 1:20, 1:30, or 1:40 or less. The molar ratio heme content can be at most about 1:1, 1:2, 1:3, 1:4, 1:5, 1:6, 1:7, 1:8, 1:9, 1:10, 1:20, 1:30, or 1:40 or less. The heme content can be 1-fold, 2-fold, 3-fold, 4-fold, 5-fold, 10-fold, 15-fold, 20-fold, 30-fold, or 40-fold or more lower than the heme content of a full-occupied polypeptide (e.g., the polypeptide is 100% occupied with heme). The heme content can be 1-fold, 2-fold, 3-fold, 4-fold, 5-fold, 10-fold, 15-fold, 20-fold, 30-fold, or 40-fold or more higher than the heme content of a fully-unoccupied polypeptide (e.g., the polypeptide is 0% occupied with heme). Heme content can be determined by a number of methods including spectroscopy (Raman, UV-Vis), electron paramagnetic resonance (EPR), protein denaturation assays, heme stealing assays, and heme reduction assays.

### Methods for Using a Polypeptide in a Meat Consumable

The disclosure describes methods for the use of a polypeptide of the disclosure in a meat consumable. The consumables can compete with, supplement or replace animal based foods. For instance the consumables can be meat replicas made entirely from plant sources. The consumables can be made to mimic the cut or appearance of meat as it is currently sold. For instance a consumable may be visually similar to or indistinguishable from ground beef or a particular cut of beef. Alternatively, the consumables can be made with a unique look or appearance. For instance the consumable could contain patterns or lettering that is based upon the structure of the consumable. In some instances the consumables can look like traditional meat products after they are prepared. For example a consumable may be produced which is larger than a traditional cut of beef but which, after the consumable is sliced and cooked appears the same as a traditional cooked meat. In some cases the consumable may resemble a traditional meat shape in two dimensions, but not in a third. For example the consumable may resemble a cut of meat in two dimensions (for example when viewed from the top), but may be much longer (or thicker) than the traditional cut. A meat consumable (e.g., substitute) can have similar physical characteristics as traditional meat (taste, texture, force, nutrients). In some cases, a meat consumable can comprise a similar cook loss characteristic as meat. In some variants a meat consumable can comprise a similar fat and protein content as ground beef has the same reduction in size when cooked as real ground beef. Methods of producing a meat consumable are described in PCT/US2012/046560.

In some instances, a meat consumable can comprise a polypeptide of the disclosure. A polypeptide of the disclosure can be used as a colorant or indicator of cooking of the meat consumable.

In some instances, the disclosure describes a method for expressing a polypeptide (e.g., globin), in a host cell, secreting the polypeptide from the host cell, purifying the secreted polypeptide, and mixing the purified polypeptide with fats and lipids to produce a meat substitute.

In some instances, the disclosure describes a method for enhancing the expression of an endogenous polypeptide (e.g., globin) in a host cell, purifying the polypeptide from the cell, and mixing the purified polypeptide with fats and lipids to produce a meat substitute.

In some instances, the disclosure describes a method for expressing a polypeptide (e. g, globin), in a host cell (e.g., a plant), purifying the secreted polypeptide, and mixing the purified polypeptide with fats and lipids to produce a meat substitute.

### Compositions

In some instances, the disclosure describes a composition comprising a polypeptide of the disclosure. A composition can comprise media into which the polypeptide was secreted. A composition can comprise at least about 0.001, 0.005, 0.01, 0.05, 0.1, 0.5, 1, 2, 3, 4, 5, 6, 7, 8, 9, or 10% (w/w) or more media. A composition can comprise at most about 0.001, 0.005, 0.01, 0.05, 0.1, 0.5, 1, 2, 3, 4, 5, 6, 7, 8, 9, or 10% or more media. A composition can comprise at least about 1, 2, 3, 4, 5, 6, 7, 8, 9, or 10 or more parts per million media. A composition can comprise at most about 1, 2, 3, 4, 5, 6, 7, 8, 9, or 10 or more parts per million media. A composition can comprise at least about 1, 2, 3, 4, 5, 6, 7, 8, 9, or 10 or more parts per billion media. A composition can comprise at most about 1, 2, 3, 4, 5, 6, 7, 8, 9, or 10 or more parts per billion media.

A composition can comprise a host cell (e.g., a bacterium). A host cell of the composition can be the host cell from which the polypeptide was expressed and/or secreted. A composition can comprise at least about 0.001, 0.005, 0.01, 0.05, 0.1, 0.5, 1, 2, 3, 4, 5, 6, 7, 8, 9, or 10% (w/w) or more host cells. A composition can comprise at most about 0.001, 0.005, 0.01, 0.05, 0.1, 0.5, 1, 2, 3, 4, 5, 6, 7, 8, 9, or 10% host cells. A composition can comprise at least about 1, 2, 3, 4, 5, 6, 7, 8, 9, or 10 or more parts per million host cells. A composition can comprise at most about 1, 2, 3, 4, 5, 6, 7, 8, 9, or 10 or more parts per million host cells. A composition can comprise at least about 1, 2, 3, 4, 5, 6, 7, 8, 9, or 10 or more parts per billion host cells. A composition can comprise at most about 1, 2, 3, 4, 5, 6, 7, 8, 9, or 10 or more parts per billion host cells. A composition can be at least about 1, 2, 3, 4, 5, 6, 7, 8, 9, 10, 20, 30, 40, 50, 60, 70, 80, 90, 91, 92, 93, 94, 95, 96, 97, 98, 99, or 100% free of a host cell. A composition can be at most about 1, 2, 3, 4, 5, 6, 7, 8, 9, 10, 20, 30, 40, 50, 60, 70, 80, 90, 91, 92, 93, 94, 95, 96, 97, 98, 99, or 100% free of a host cell.

A composition can comprise a component of a recombinant host cell (e.g., a recombinant bacterial cell or plant cell). A component of a host cell can include, for example, a cell wall, a subcellular compartment (e.g., Golgi complex, endoplasmic reticulum, or nucleus), nucleic acid, protein, genomic DNA, and/ or a plasma membrane. For a bacterial cell, a component also can include flagella, and for a plant or plant cell, a component can be any part of the plant such as a shoot, a stem, a seed, a bean, a leave, xylem tissue, a rosette, a root. A component of a host cell can be a component of a host cell from which the polypeptide was expressed and/or secreted. For example, a composition can include a non-naturally occurring component of a recombinant host cell such as a fusion protein or an exogenous nucleic acid. A composition can comprise at least about 0.001, 0.005, 0.01, 0.05, 0.1, 0.5, 1, 2, 3, 4, 5, 6, 7, 8, 9, or 10% (w/w) or more of a component of a host cell. A composition can comprise at most about 0.001, 0.005, 0.01, 0.05, 0.1, 0.5, 1, 2, 3, 4, 5, 6, 7, 8, 9, or 10% of a component of a host cell. A composition can comprise at least about 1, 2, 3, 4, 5, 6, 7, 8, 9, or 10 or more parts per million of the component of a bacterium. A composition can comprise at most about 1, 2, 3, 4, 5, 6, 7, 8, 9, or 10 parts per million of a component of a host cell. A composition can comprise at least about 1, 2, 3, 4, 5, 6, 7, 8, 9, or 10 or more parts per billion of a component of a host cell. A composition can comprise at most about 1, 2, 3, 4, 5, 6, 7, 8, 9, or 10 parts per billion of a component of a host cell. A composition can be at least about 1, 2, 3, 4, 5, 6, 7, 8, 9, 10, 20, 30, 40, 50, 60, 70, 80, 90, 91, 92, 93, 94, 95, 96, 97, 98, 99, or 100% free of a component of a host cell. A composition can be at most about 1, 2, 3, 4, 5, 6, 7, 8, 9, 10, 20, 30, 40, 50, 60, 70, 75, 80, 85, 90, 91, 92, 93, 94, 95, 96, 97, 98, 99, or 100% free of a component of a host cell.

### Meat consumable compositions

In some instances, a composition of the disclosure can comprise a meat consumable and a host cell (e.g., bacterium, a part of a bacterium, and/or a part of a plant). In some instances, a composition can further comprise a polypeptide of the disclosure. In some instances a composition comprises a polypeptide and a meat consumable (i.e., meat substitute) (as described in PCT/US2012/046560.

A meat consumable can refer to meat-like product (e.g., a meat substitute) that is not made of meat. A meat consumable can refer to a meat substitute that is made from non-animal products (e.g., a plant). A meat consumable can be meat replicas made entirely from plant sources. The consumables may also be made from a combination of plant based sources and animal based sources. The consumables can be made to mimic the cut or appearance of meat as it is currently sold. For instance a consumable may be visually similar to or indistinguishable from ground beef or a particular cut of beef. In some instances the consumables look like traditional meat products after they are prepared. The meat consumable can be substantially or entirely composed of ingredients derived from non-animal sources, yet recapitulates key features associated with the cooking and consumption of an equivalent meat product derived from animals.

A composition can comprise a meat consumable and a polypeptide of the disclosure. A meat consumable can comprise at least about 1, 5, 10, 15, 20, 25, 30, 35, 40, 45, 50, 55, 60, 65, 70, 75, 80, 85, 90, 95, or 100% (w/w) of one or more polypeptides of the disclosure. In some instances, a meat consumable can comprise at most about 1, 5, 10, 15, 20, 25, 30, 35, 40, 45, 50, 55, 60, 65, 70, 75, 80, 85, 90, 95, or 100% of one or more polypeptides of the disclosure. A meat consumable can comprise at least about 1, 5, 10, 15, 20, 25, 30, 35, 40, 45, 50, 55, 60, 65, 70, 75, 80, 85, 90, 95, or 100% weight/volume of one or more polypeptides of the disclosure. In some instances, a meat consumable can comprise at most about 1, 5, 10, 15, 20, 25, 30, 35, 40, 45, 50, 55, 60, 65, 70, 75, 80, 85, 90, 95, or 100% weight/volume of one or more polypeptides of the disclosure.

A composition can comprise a meat consumable and a host cell (e.g., bacterium). A host cell of the composition can be the host cell from which the polypeptide was expressed and/or secreted. A composition can comprise a meat consumable and at least about 0.001, 0.005, 0.01, 0.05, 0.1, 0.5, 1, 2, 3, 4 ,5 ,6, 7, 8, 9, or 10% (w/w) or more host cells. A composition can comprise a meat consumable and at most about 0.001, 0.005, 0.01, 0.05, 0.1, 0.5, 1, 2, 3, 4 ,5 ,6, 7, 8, 9, or 10% host cells. A composition can comprise a meat consumable and at least about 1, 2, 3, 4, 5, 6, 7, 8, 9, or 10 or more parts per million host cell. A composition can a meat consumable and comprise at most about 1, 2, 3, 4, 5, 6, 7, 8, 9, or 10 parts per million host cell. A composition can comprise a meat consumable and at least about 1, 2, 3, 4, 5, 6, 7, 8, 9, or 10 or more parts per billion host cell. A composition can comprise a meat consumable and at most about 1, 2, 3, 4, 5, 6, 7, 8, 9, or 10 parts per billion host cell. A composition can comprise a meat consumable and be at least about 1, 2, 3, 4, 5, 6, 7, 8, 9, 10, 20, 30, 40, 50, 60, 70, 80, 90, 91, 92, 93, 94, 95, 96, 97, 98, 99, or 100% free of a host cell. A composition comprise a meat consumable and can be at most about 1, 2, 3, 4, 5, 6, 7, 8, 9, 10, 20, 30, 40, 50, 60, 70, 80, 90, 91, 92, 93, 94, 95, 96, 97, 98, 99, or 100% free of a host cell.

A composition can comprise a part of a meat consumable and a component of a host cell (e.g., a part of a bacterium). A component of a host cell can include a cell wall, a subcellular compartment (e.g., Golgi complex, endoplasmic reticulum, nucleus), a flagella, nucleic acid, protein, genomic DNA, or a plasma membrane. A component of a host cell can be a part of a bacterium from which the polypeptide was expressed and/or secreted. A composition can comprise a meat consumable and at least about 0.001, 0.005, 0.01, 0.05, 0.1, 0.5, 1, 2, 3, 4, 5, 6, 7, 8, 9, or 10% or more of part of a host cell. A composition can comprise a meat consumable and at most about 0.001, 0.005, 0.01, 0.05, 0.1, 0.5, 1, 2, 3, 4, 5, 6, 7, 8, 9, or 10% of a component of a host cell. A composition can comprise a meat consumable and at least about 1, 2, 3, 4, 5, 6, 7, 8, 9, or 10 or more parts per million of part of a host cell. A composition can comprise a meat consumable and at most about 1, 2, 3, 4, 5, 6, 7, 8, 9, or 10 parts per million of a component of a host cell. A composition can comprise a meat consumable and at least about 1, 2, 3, 4, 5, 6, 7, 8, 9, or 10 or more parts per billion of a component of a host cell. A composition can comprise a meat consumable and at most about 1, 2, 3, 4, 5, 6, 7, 8, 9, or 10 parts per billion of a component of a host cell. A composition can comprise a meat consumable and be at least about 1, 2, 3, 4, 5, 6, 7, 8, 9, 10, 20, 30, 40, 50, 60, 70, 80, 90, 91, 92, 93, 94, 95, 96, 97, 98, 99, or 100% free of a component of a host cell. A composition can comprise a meat consumable and be at most about 1, 2, 3, 4, 5, 6, 7, 8, 9, 10, 20, 30, 40, 50, 60, 70, 80, 90, 91, 92, 93, 94, 95, 96, 97, 98, 99, or 100% free of a component of a host cell.

A composition can comprise a meat consumable and a component of a host cell (e.g., plant, e.g., a tobacco plant, i.e., a *Nicotiana tabacum* species or a soybean plant, i.e., a *Glycine max* species). In some instances, the host cell is not a *Nicotiana* plant. A part of a host cell can include a cell wall, a subcellular compartment (e.g., Golgi complex, endoplasmic reticulum, nucleus), a shoot, a stem, a leave, a seed, a bean, a xylem, a rosette, a root, nucleic acid, protein, genomic DNA, and a plasma membrane. A component of a host cell can be a part of a plant from which the polypeptide was expressed and/or secreted. A composition can comprise a meat consumable and at least about 0.001, 0.005, 0.01, 0.05, 0.1, 0.5, 1, 2, 3, 4,5 ,6, 7, 8, 9, or 10% or more of a component of a host cell. A composition can comprise a meat consumable and at most about 0.001, 0.005, 0.01, 0.05, 0.1, 0.5, 1, 2, 3, 4, 5, 6, 7, 8, 9, or 10% of a component of a host cell. A composition can comprise a meat consumable and at least about 1, 2, 3, 4, 5, 6, 7, 8, 9, or 10 or more parts per million of a component of a host cell. A composition can comprise a meat consumable and at most about 1, 2, 3, 4, 5, 6, 7, 8, 9, or 10 parts per million of a component of a host cell. A composition can comprise a meat consumable and at least about 1, 2, 3, 4, 5, 6, 7, 8, 9, or 10 or more parts per billion of a component of a host cell. A composition can comprise a meat consumable and at most about 11, 2, 3, 4, 5, 6, 7, 8, 9, or 10 parts per billion of a component of a host cell. A composition can comprise a meat consumable and can be at least about 1, 2, 3, 4, 5, 6, 7, 8, 9, 10, 20, 30, 40, 50, 60, 70, 80, 90, 91, 92, 93, 94, 95, 96, 97, 98, 99, or 100% free of a component of a host cell. A composition can comprise a meat consumable and be at most about 1, 2, 3, 4, 5, 6, 7, 8, 9, 10, 20, 30, 40, 50, 60, 70, 80, 90, 91, 92, 93, 94, 95, 96, 97, 98, 99, or 100% free of a component of a host cell.

In some instances, the disclosure describes a consumable that can be substantially or entirely composed of ingredients derived from non-animal sources, yet recapitulates key features associated with the cooking and consumption of an equivalent meat product derived from animals. The equivalent meat product can be a white meat or a dark meat. The equivalent meat product can be derived from any animal. Non-limiting examples of animals used to derive the equivalent meat product include farmed animals such as, e.g., cattle, sheep, pig, chicken, turkey, goose, duck, horse, dog or game animals (whether wild or farmed) such as, e.g., rabbit, deer, bison, buffalo, boar, snake, pheasant, quail, bear, elk, antelope, pigeon, dove, grouse, fox, wild pig, goat, kangaroo, emu, alligator, crocodile, turtle, groundhog, marmot, possum, partridge, squirrel, raccoon, whale, seal, ostrich, capybara, nutria, guinea pig, rat, mice, vole, any variety of insect or other arthropod, seafood such as, e. g, fish, crab, lobster, oyster, muscle, scallop, abalone, squid, octopus, sea urchin, tunicate and others. Many meat products are typically derived from skeletal muscle of an animal but it is understood that meat can also come from other muscles or organs of the animal. In some embodiments, the equivalent meat product is a cut of meat derived from skeletal muscle. In some variants, the equivalent meat product is an organ such as, e.g., a kidney, heart, liver, gallbladder, intestine, stomach, bone marrow, brain, thymus, lung, tongue. Accordingly, in some instances the compositions of the present are consumables similar to skeletal muscle or organs.

In some instances, the disclosure describes meat substitute products comprising one or more of a first composition comprising a muscle tissue replica, a second composition comprising an adipose tissue replica, and/or a third composition comprising a connective tissue replica, wherein the one or more compositions are combined in a manner that recapitulates the physical organization of meat. In other instances, the present disclosure describes compositions for a muscle tissue replica (herein referred to as "muscle replica"), an adipose tissue replica (herein referred to as "fat replica"), and a connective tissue replica (herein referred to as "connective tissue replica"). In some instances, the compositions and meat substitute products are principally or entirely composed of ingredients derived from non-animal sources. In alternative variants, the muscle, fat, and/or connective tissue replica, or the meat substitute products comprising one or more of said replicas, are partially derived from animal sources but supplemented with ingredients derived from non-animal sources.

In some instances, meat products can be substantially derived from animal sources but which are supplemented with one or more of a muscle tissue replica, a fat replica, and/or a connective tissue replica, wherein the replicas can be derived substantially or entirely from non-animal sources. A non- limiting example of such a meat product is an ultra-lean ground beef product supplemented with a non-animal derived fat replica which can improve texture and mouthfeel while preserving the health benefits of a consumable low in animal fat. Such alternative variants result in products with properties that more closely recapitulate key features associated with preparing and consuming meat but which are less costly and associated with a lesser environmental impact, less animal welfare impact, or improved health benefits for the consumer.

The physical organization of the meat substitute product can be manipulated by controlling the localization, organization, assembly, or orientation of the muscle, fat, and/or connective tissue replicas described herein. In some cases the product is designed in such a way that the replicas described herein are associated with one another as in meat. In some instances the consumable is designed so that after cooking the replicas described herein are associated with one another as in cooked meat. In some variants, one or more of the muscle, fat, and/or connective tissue replicas are combined in a manner that recapitulate the physical organization of different cuts or preparations of meat. In an example variant, the replicas are combined in a manner that approximates the physical organization of natural ground meat. In other variants, the replicas are combined in a manner that approximates different cuts of beef, such as, e.g., rib eye, filet mignon, London broil, among others.

### Indicators of Cooking Meat

In some instances, a polypeptide of the disclosure can be used in a composition of the disclosure as an indicator for cooking meat. The release of odorants upon cooking is an important aspect of meat consumption. In some variants, the consumable is a meat replica entirely composed of non-animal products that when cooked generates an aroma recognizable by humans as typical of cooking beef. In some cases, the consumable when cooked generates an aroma recognizable by humans as typical of cooking pork. In some cases, the consumable is a meat replica entirely composed of non-animal products that when cooked generates an aroma recognizable by humans as typical of cooking bacon. In some instances, the consumable is a meat replica entirely composed of non-animal products that when cooked generates an aroma recognizable by humans as typical of cooking chicken. In some variants, the consumable is a meat replica entirely composed of non-animal products that when cooked generates an aroma recognizable by humans as typical of cooking lamb. In some cases, the consumable is a meat replica entirely composed of non-animal products that when cooked generates an aroma recognizable by humans as typical of cooking fish. In some instances, the consumable is a meat replica entirely composed of non-animal products that when cooked generates an aroma recognizable by humans as typical of cooking turkey. In some cases the consumable is a meat replica principally or entirely composed of ingredients derived from non-animal sources, with an odorant that is released upon cooking. In some variants the consumable is a meat replica principally or entirely composed of ingredients derived from non-animal sources, with an odorant that is produced by chemical reactions that take place upon cooking. In some cases the consumable is a meat replica principally or entirely composed of ingredients derived from non-animal sources, comprising a polypeptide of the disclosure and mixtures of proteins, peptides, amino acids, nucleotides, sugars and polysaccharides and fats in combinations and spatial arrangements that enable these compounds to undergo chemical reactions during cooking to produce odorants and flavor-producing compounds. In some variants the consumable is a meat replica principally or entirely composed of ingredients derived from non-animal sources (e.g., a polypeptide of the disclosure), with a volatile or labile odorant that is released upon cooking. In some cases the consumable is a method for preparing a meat replica where meat replicas principally or entirely composed of ingredients derived from non-animal sources are heated to release a volatile or labile odorant.

Odorants released during cooking of meat are generated by reactions that can involve as reactants fats, protein, amino acids, peptides, nucleotides, organic acids, sulfur compounds, sugars and other carbohydrates. In some instances, a reactant can be a polypeptide of the disclosure (e.g, a globin, a secreted globin). In some cases the odorants that combine during the cooking of meat are identified and located near one another in the consumable, such that upon cooking of the consumable the odorants combine. In some variants, the characteristic flavor and fragrance components are produced during the cooking process by chemical reactions involving amino acids, fats and sugars found in plants as well as meat. In some cases, the characteristic flavor and fragrance components are mostly produced during the cooking process by chemical reactions involving one or more amino acids, fats, peptides, nucleotides, organic acids, sulfur compounds, sugars and other carbohydrates found in plants as well as meat.

Some reactions that generate odorants released during cooking of meat can be catalyzed by iron, in particular the iron of heme, which may be comprised (e.g., bound) by a polypeptide of the disclosure. Thus in some cases, some of the characteristic flavor and fragrance components are produced during the cooking process by chemical reactions catalyzed by iron. In some variants, some of the characteristic flavor and fragrance components are produced during the cooking process by chemical reactions catalyzed by heme. In some instances, some of the characteristic flavor and fragrance components are produced during the cooking process by chemical reactions catalyzed by the heme iron in leghemoglobin. In some cases, some of the characteristic flavor and fragrance components are produced during the cooking process by chemical reactions catalyzed by the heme iron in a heme protein (e.g., the polypeptides listed in Figure 9, hemoglobin, myoglobin, neuroglobin, cytoglobin, leghemoglobin, non-symbiotic hemoglobin, Hell's gate globin I, bacterial hemoglobins, ciliate myoglobins, flavohemoglobins, androglobin, cytoglobin, globin E, globin X, globin Y, myoglobin, leghemoglobins, erythrocruorins, beta hemoglobins, alpha hemoglobins, non-symbiotic hemoglobins, protoglobins, cyanoglobins, Hell's gate globin I, bacterial hemoglobins, ciliate myoglobins, histoglobins and neuroglobins, etc).

### Color Indicators

The color of meat is an important part the experience of cooking and eating meat. For instance, cuts of beef are of a characteristic red color in a raw state and gradually transition to a brown color during cooking. As another example, white meats such as chicken or pork have a characteristic pink color in their raw state and gradually transition to a white or brownish color during cooking. The amount of the color transition is used to indicate the cooking progression of beef and titrate the cooking time and temperature to produce the desired state of done-ness. The disclosure describes a non-meat based meat substitute product that provides a visual indicator of cooking progression. In some cases, the visual indicator is a color indicator that undergoes a color transition during cooking. In particular instances, the color indicator recapitulates the color transition of a cut of meat as the meat progresses from a raw to a cooked state. In some variants, the color indicator colors the meat substitute product a red color before cooking to indicate a raw state and causes the meat substitute product to transition to a brown color during cooking progression. In some cases, the color indicator colors the meat substitute product a pink color before cooking to indicate a raw state and causes the meat substitute product to transition to a white or brown color during cooking progression.

The main determinant of the nutritional definition of the color of meat is the concentration of iron carrying proteins in the meat. In the skeletal muscle component of meat products, one of the main iron-carrying proteins is myoglobin. So, in some cases, the composition is a meat consumable (e.g., replica) which comprises an iron-carrying protein. In some variants, the composition comprises about 0.05%, about 0.1%>, about 0.2%, about 0.3%, about 0.4%, about 0.5%, about 0.6%, about 0.7%, about 0.8%, about 0.9%, about 1%, about 1.1%, about 1.2%, about 1.3%, about 1.4%, about 1. 5%, about 1.6%, about 1.7%, about 1.8%, about 1.9%, about 2%, or more than about 2% of an iron-carrying protein by dry weight or total weight. In some instances, the composition comprises at least about 10% of a polypeptide of the disclosure. In some cases, the composition comprises at most about 10% of a polypeptide of the disclosure. In some cases, the iron carrying protein has been isolated and purified from a source. In other cases, the iron carrying protein has not been isolated and purified. In some cases, the source of the iron-carrying protein is an animal source, or a non-animal source such as a plant, fungus, or genetically modified organisms such as, e.g., bacteria or yeast. In some cases, the iron-carrying protein is myoglobin. In some instances the composition comprises a consumable that is a plant based meat replica that has animal myoglobin added. So, for example a replica of young beef can have about 0.4-1%) myoglobin. In some cases, the iron-carrying protein is leghemoglobin. In some variants the composition comprises a consumable that is a plant based meat replica that has leghemoglobin added. So, for example a replica of young beef can have about 0.4-1% leghemoglobin. In some cases, the iron-carrying protein is a cytochrome. In some variants the composition comprises a consumable that is a plant based meat replica that has a cytochrome added. So, for example a replica of young beef can have about 0.4-1% of a cytochrome. In some cases the consumable is a plant-based meat replica containing hemoglobin. In some instances, the iron-carrying protein is a polypeptide of the disclosure (e.g., a globin).

Additional iron containing proteins exist in nature. In some cases the composition (e.g., consumable) comprises an iron containing protein that is not myoglobin. In some variants the composition (e.g., consumable) does not contain myoglobin. In some instances the compositions (e.g., consumable) does not contain hemoglobin. In some cases the consumable is a meat replica that comprises an iron containing protein other than myoglobin or hemoglobin (e.g., the globins listed in Figure 9, and described herein, e.g., hemoglobin, myoglobin, neuroglobin, cytoglobin, leghemoglobin, non-symbiotic hemoglobin, Hell's gate globin I, bacterial hemoglobins, ciliate myoglobins, flavohemoglobins).

In some cases the composition comprises a consumable that is a meat replica principally or entirely composed of ingredients derived from non-animal sources, including a muscle tissue replica, an adipose tissue replica, a connective tissue replica, and leghemoglobin. In some variants the composition comprises a consumable that is a meat replica principally or entirely composed of ingredients derived from non-animal sources, containing a heme protein. In some instances the composition comprises a consumable that is a meat replica principally or entirely composed of ingredients derived from non-animal sources, containing a leghemoglobin. In some cases the composition comprises a consumable that is a meat replica principally or entirely composed of ingredients derived from non-animal sources, containing a member of the globin protein family. In some instances the composition comprises a consumable that is a meat replica principally or entirely composed of ingredients derived from non-animal sources, with a high iron content from a heme protein. In some variants the iron content is similar to meat. In some cases the consumable has the distinctive red color of meat, such color provided by leghemoglobin.

Leghemoglobin is, in some cases, used as an indicator that the consumable is finished cooking. In some variants of the disclosure there is a method for cooking a consumable comprising detecting leghemoglobin which has migrated from the interior of the consumable to the surface when the product is cooked. In some variants of the disclosure there is described a method for cooking a consumable comprising detecting the change in color of from red to brown when the product is cooked.

The oxidation state of the iron ion in leghemoglobin can be important for its color. Leghemoglobin with the heme iron in the +2 oxidation state can appear vivid red in color, while leghemoglobin with the heme iron in the +3 oxidation state can appear brownish red. Thus, in using leghemoglobin as a source of red color in a meat replica for example, it can be desirable to reduce the heme iron from the +3 state to the +2 state. Heme iron in leghemoglobin can be switched from oxidized (+3) state to reduced (+2) state with reducing reagents.

A heme protein can, in some cases, be used as an indicator that the consumable is finished cooking. In some cases, there is disclosed a method for cooking a consumable comprising detecting leghemoglobin which has migrated from the interior of the consumable to the surface when the product is cooked. In some cases, there is disclosed a method for cooking a consumable comprising detecting the change in color of from red to brown when the product is cooked.

A heme protein (e.g., Hemoglobin, myoglobin, neuroglobin, cytoglobin, leghemoglobin, non-symbiotic hemoglobin, Hell's gate globin I, bacterial hemoglobins, ciliate myoglobins, flavohemoglobins), can be, in some cases, used as an indicator that the consumable is finished cooking. So, in some instances, the disclosure describes a method for cooking a consumable comprising detecting leghemoglobin which has migrated from the interior of the consumable to the surface when the product is cooked. The disclosure can describe a method for cooking a consumable comprising detecting the change in color of from red to brown when the product is cooked.

### Food products comprising purified polypeptide

In some cases a polypeptide of the disclosure (e.g., a heme-containing polypeptide, a globin such as leghemoglobin) is added to meat to enhance the properties of meat. See, for example, WO 2014/110532, WO 2014/110539, and WO 2013/010042. For example, a polypeptide-containing solution can be injected into raw or cooked meat. In another example a solution comprising a polypeptide of the disclosure is dripped over meat or a consumable to enhance appearance. In some cases advertising, photography, or videography of food products such as meat or a meat substitute is enhanced with leghemoglobin.

Polypeptides, for example leghemoglobin and hemoglobin, can be combined with other plant based meat replica components. In some instances the polypeptides are captured in a gel that contains other components, for example lipids and or proteins. In some cases multiple gels are combined with non-gel based heme proteins. In some cases the combination of the polypeptides and the other compounds of the consumable are done to ensure that the heme proteins are able to diffuse through the consumable. In some variants the consumable comprises a heme-protein containing solution, for instance a leghemoglobin solution. In some cases the consumable is soaked in a heme protein containing solution, for instance a leghemoglobin solution for 1, 5, 10, 15, 20 or 30 hours. In some variants the consumable is soaked in a heme containing solution, for instance a leghemoglobin solution for 1, 5, 10, 15, 30, or 45 minutes.

### Muscle Replicas

A large number of meat products comprise a high proportion of skeletal muscle. Accordingly, the present disclosure describes a composition derived from non-animal sources which replicates or approximates key features of animal skeletal muscle. The present disclosure also describes a meat substitute product that comprises a composition derived from non-animal sources which replicates or approximates animal skeletal muscle. Such a composition will be labeled herein as "muscle replica". In some cases, the muscle replica and/or meat substitute product comprising the muscle replica are partially derived from animal sources. In some cases, the muscle replica and/or meat substitute product comprising the muscle replica are entirely derived from non-animal sources.

Many meat products comprise a high proportion of striated skeletal muscle in which individual muscle fibers are organized mainly in an isotropic fashion. Accordingly, in some variants the muscle replica comprises fibers that are to some extent organized isotropically. In some instances the fibers comprise a protein component. In some cases, the fibers comprise about 1%, about 2%, about 5%, about 10%, about 15%, about 20%, about 30%, about 40%, about 50%, about 60%, about 70%, about 80%), about 90%, about 95%, about 99% or more of a protein component. Animal skeletal muscle typically contains around 1% myoglobin, but can be as much as 7% of muscle mass in some whale muscles. In some variants the muscle replica comprises hemoglobins of this disclosure.

In some cases, the protein component comprises one or more isolated, purified proteins. For example the one or more isolated, purified protein can comprise the 8S globulin from Moong bean seeds, or the albumin or globulin fraction of pea seeds. These proteins provide examples of proteins with favorable properties for constructing meat replicas because of their ability to form gels with textures similar to animal muscle or fat tissue. Examples of the one or more isolated, purified proteins are described herein. The list of potential candidates here is essentially open and may include Rubisco, any major seed storage proteins, proteins isolated from fungi, bacteria, archaea, viruses, or genetically engineered microorganisms, or synthesized in vitro. The proteins may be artificially designed to emulate physical properties of animal muscle tissue. The proteins may be artificially designed to emulate physical properties of animal muscle tissue. In some variants, one or more isolated, purified proteins accounts for about 0.1%, 0.2%, 0.5%, 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 99% or more of the protein component by weight.

Skeletal muscle of animals such as beef cattle typically contain substantial quantities of glycogen, which can comprise on the order of 1%> of the mass of the muscle tissue at the time of slaughter. After slaughter, a fraction of this glycogen continues to be metabolized yielding products including lactic acid, which contributes to lowering the pH of the muscle tissue, a desirable quality in meat. Glycogen is a branched polymer of glucose linked together by alpha (1->4) glycosidic bonds in linear chains, with branch points comprising alpha (1->6) glycosidic bonds. Starches from plants, particularly amylopectins are also branched polymers of glucose linked together by alpha (1->4) glycosidic bonds in linear chains, with branch points comprising alpha (1->6) glycosidic bonds and can therefore be used as an analog of glycogen in constructing meat replicas. Thus in some cases, the muscle or meat replica includes a starch or pectin.

Additional components of animal muscle tissue include sodium, potassium, calcium, magnesium, other metal ions, lactic acid, other organic acids, free amino acids, peptides, nucleotides and sulfur compounds. Thus in some cases, the muscle replica can include sodium, potassium, calcium, magnesium, other metal ions, lactic acid, other organic acids, free amino acids, peptides, nucleotides and sulfur compounds. In some instances the concentration of sodium, potassium, calcium, magnesium, other metal ions, lactic acid, other organic acids, free amino acids, peptides, nucleotides and/or sulfur compounds in the muscle replica or consumable are within 10%> of the concentrations found in a muscle or meat being replicated.

The disclosure describes methods for making a muscle replica. In some cases, the composition is formed into asymmetric fibers prior to incorporation into the consumable. In some cases these fibers replicate muscle fibers. In some instances the fibers are spun fibers. In other variants the fibers are extruded fibers. Accordingly, the present disclosure describes methods for producing asymmetric or spun protein fibers. In some cases, the fibers are formed by extrusion of the protein component through an extruder.

In some cases extrusion can be conducted using an MPF19 twin-screw extruder (APV Baker, Grand Rapids, Mich.) with a cooling die. The cooling die can cool the extrudate prior to return of the extrudate to atmospheric pressure, thus substantially inhibiting expansion or puffing of the final product. In the MPF19 apparatus, dry feed and liquid can be added separately and mixed in the barrel. Extrusion parameters can be, for example: screw speed of 200 rpm, product temperature at the die of 150 C, feed rate of 23 g/min, and water- flow rate of 11 g/min. Product temperature can be measured during extrusion by a thermocouple at the end of the extrusion barrel. Observations can be made on color, opacity, structure, and texture for each collected sample. Collected samples can be optionally dried at room temperature overnight, then ground to a fine powder (<60 mesh) using a Braun food grinder. The pH of samples can be measured in duplicate using 10% (w/v) slurries of powdered sample in distilled water.

### Fat Replica

Animal fat is important for the experience of eating cooked meat. Accordingly, the present disclosure describes a composition derived from non-animal sources which recapitulates key features of animal fat. Furthermore the present disclosure describes a meat substitute product that comprises a composition derived from non-animal sources which recapitulates animal fat. Such a composition will be labeled herein as a "fat replica". In some cases, the fat replica and/or meat substitute product comprising the fat replica are partially derived from animal sources.

In some instances the meat substitute product has a fat component. In some cases the fat content of the consumable is 1%, 5%, 10%, 15%, 20%, 25%, 30%, 35%, 40%, 45%, 50%, 55%, or 60%) fat. In some variants, the fat replica comprises a gel with droplets of fat suspended therein. In some instances, the gel is a soft, elastic gel comprising proteins and optionally carbohydrates. In particular variants, the proteins used in the gel are plant or microbial proteins. In some cases, the proteins used in the fat replica might include Rubisco, any major seed storage proteins, proteins isolated from fungi, bacteria, archaea, viruses, or genetically engineered microorganisms, or synthesized in vitro. The proteins may be artificially designed to emulate physical properties of animal fat. The proteins may be artificially designed to emulate physical properties of animal fat.

The fat droplets used in some variants of the present disclosure can be from a variety of sources. In some cases, the sources are non-animal sources. In particular instances, the sources are plant sources. Non-limiting examples of oils include corn oil, olive oil, soy oil, peanut oil, walnut oil, almond oil, sesame oil, cottonseed oil, rapeseed oil, canola oil, safflower oil, sunflower oil, flax seed oil, algal oil, palm oil, palm kernel oil, coconut oil, babassu oil, shea butter, mango butter, cocoa butter, wheat germ oil, rice bran oil, oils produced by bacteria, algae, archaea or fungi or genetically engineered bacteria, algae, archaea or fungi, triglycerides, monoglycerides, diglycerides, sphingosides, glycolipids, lecithin, lysolecithin, phophatidic acids, lysophosphatidic acids, oleic acid, palmitoleic acid, palmitic acid, myristic acid, lauric acid, myristoleic acid, caproic acid, capric acid, caprylic acid, pelargonic acid, undecanoic acid, linoleic acid, 20:1 eicosanoic acid, arachidonic acid, eicosapentanoic acid, docosohexanoic acid, 18:2 conjugated linoleic acid, conjugated oleic acid, or esters of: oleic acid, palmitoleic acid, palmitic acid, myristic acid, lauric acid, myristoleic acid, caproic acid, capric acid, caprylic acid, pelargonic acid, undecanoic acid, linoleic acid, 20:1 eicosanoic acid, arachidonic acid, eicosapentanoic acid, docosohexanoic acid, 18:2 conjugated linoleic acid, or conjugated oleic acid, or glycerol esters of oleic acid, palmitoleic acid, palmitic acid, myristic acid, lauric acid, myristoleic acid, caproic acid, capric acid, caprylic acid, pelargonic acid, undecanoic acid, linoleic acid, 20:1 eicosanoic acid, arachidonic acid, eicosapentanoic acid, docosohexanoic acid, 18:2 conjugated linoleic acid, or conjugated oleic acid, or triglyceride derivatives of oleic acid, palmitoleic acid, palmitic acid, myristic acid, lauric acid, myristoleic acid, caproic acid, capric acid, caprylic acid, pelargonic acid, undecanoic acid, linoleic acid, 20:1 eicosanoic acid, arachidonic acid, eicosapentanoic acid, docosohexanoic acid, 18:2 conjugated linoleic acid, or conjugated oleic acid.

In some cases, fat droplets are derived from pulp or seed oil. In other cases, the source may be yeast or mold. For instance, in one variant the fat droplets comprise triglycerides derived from *Mortierella isabellina.*

In some variants plant oils are modified to resemble animal fats. The plant oils can be modified with flavoring or other agents to recapitulate the taste and smell of meat during and after cooking. Accordingly, some variants of the disclosure involve methods for testing the qualitative similarity between the cooking properties of animal fat and the cooking properties of plant oils in the consumable.

In some cases, the fat replica comprises a protein component comprising one or more isolated, purified proteins. The purified proteins contribute to the taste and texture of the meat replica. In some variants purified proteins can stabilize emulsified fats. In some cases the purified proteins can form gels upon denaturation or enzymatic crosslinking, which replicate the appearance and texture of animal fat. Examples and variants of the one or more isolated, purified proteins are described herein. In particular cases, the one or more isolated proteins comprise a protein isolated from the legume family of plants. Non- limiting examples of legume plants are described herein, although variations with other legumes are possible. In some cases, the legume plant is a pea plant. In some cases the isolated purified proteins stabilize emulsions. In some instances the isolated purified proteins form gels upon crosslinking or enzymatic crosslinking. In some variants, the isolated, purified proteins comprise seed storage proteins. In some cases, the isolated, purified proteins comprise albumin. In some instances, the isolated, purified proteins comprise globulin. In a particular variant, the isolated, purified protein is a purified pea albumin protein. In another particular variant, the isolated, purified protein is a purified pea globulin protein. In another particular variant the isolate purified protein is a Moong bean 8S globulin. In another particular variant, the isolated, purified protein is an oleosin. In another particular variant, the isolated, purified protein is a caloleosin. In another particular variant, the isolated, purified protein is Rubisco. In some variants, the protein component comprises about 0.1%, 0.5%, 1%, 2%, 5%, 10%, 15%, 20%, 25%, 30%, 35%, 40%, 45%, 50%, 55%, 60%, 65%, 70%, 75%, 80%, 85%, 90% or more of the fat replica by dry weight or total weight. In some variants, the protein component comprises about 0.1-5%, about 0.5-10%, about 1-20%, about 5-30%, about 10-50%, about 20-70%, or about 30-90% or more of the fat replica by dry weight or total weight. In some variants, the protein component comprises a solution containing one or more isolated, purified proteins.

In some instances, the fat replica comprises cross-linking enzymes that catalyze reactions leading to covalent crosslinks between proteins. Cross-linking enzymes can be used to create or stabilize the desired structure and texture of the adipose tissue replica, to mimic the desired texture of an equivalent desired animal fat. Non-limiting examples of cross-linking enzymes include, e.g., transglutaminase, lysyl oxidases, or other amine oxidases (e.g. Pichia pastoris lysyl oxidase). In some cases, the cross-linking enzymes are isolated and purified from a non-animal source, examples and versions of which are described herein. In some cases, the fat replica comprises at least 0.0001%, or at least 0.001%, or at least 0.01%, or at least 0.1%, or at least 1% (wt/vol) of a cross-linking enzyme. In particular variants, the cross-linking enzyme is transglutaminase.

The disclosure further describes methods for making a fat replica. In some cases, the fat droplets are suspended in a gel. In some variants the present disclosure describes methods for producing droplets of fat suspended in the gel. The fat can isolated and homogenized. For example an organic solvent mixture can be used to help mix a lipid. The solvent can then be removed. At this point the lipid can be frozen, lyophilized, or stored. So in some variants the disclosure describes a method for isolating and storing a lipid which has been selected to have characteristics similar to animal fat. The lipid film or cake can then be hydrated. The hydration can utilize agitation or temperature changes. The hydration can occur in a precursor solution to a gel. After hydration the lipid suspension can be sonicated or extruded to further alter the properties of the lipid in the solution.

In some cases, the fat replica is assembled to approximate the organization adipose tissue in meat. In some instances some or all of the components of the fat replica are suspended in a gel. In various variants the gel can be a proteinaceous gel, a hydrogel, an organogel, or a xerogel. In some variants, the gel can be thickened to a desired consistency using an agent based on polysaccharides or proteins. For example fecula, arrowroot, cornstarch, katakuri starch, potato starch, sago, tapioca, alginin, guar gum, locust bean gum, xanthan gum, collagen, egg whites, furcellaran, gelatin, agar, carrageenan, cellulose, methylcellulose, hydroxymethylcellulose, acadia gum, konjac, starch, pectin, amylopectin or proteins derived from legumes, grains, nuts, other seeds, leaves, algae, bacteria, of fungi can be used alone or in combination to thicken the gel, forming an architecture or structure for the consumable.

In particular variants, the fat replica is an emulsion comprising a solution of one or more proteins and one or more fats suspended therein as droplets. In some cases, the emulsion is stabilized by one or more cross-linking enzymes into a gel. In some cases, the one or more proteins in solution are isolated, purified proteins. In some cases, the isolated, purified proteins comprise a purified pea albumin enriched fraction. In some variants, the isolated, purified proteins comprise a purified pea globulin enriched fraction. In some instances, the isolated, purified proteins comprise a purified Moong bean 8S globulin enriched fraction. In some cases, the isolated, purified proteins comprise a Rubisco enriched fraction. In some cases, the one or more fats are derived from plant-based oils. In some cases, the one or more fats are derived from one or more of: corn oil, olive oil, soy oil, peanut oil, walnut oil, almond oil, sesame oil, cottonseed oil, rapeseed oil, canola oil, safflower oil, sunflower oil, flax seed oil, algal oil, palm oil, palm kernel oil, coconut oil, babassu oil, shea butter, mango butter, cocoa butter, wheat germ oil, rice bran oil, oils produced by bacteria, algae, archaea or fungi or genetically engineered bacteria, algae, archaea or fungi, triglycerides, monoglycerides, diglycerides, sphingosides, glycolipids, lecithin, lysolecithin, phophatidic acids, lysophosphatidic acids, oleic acid, palmitoleic acid, palmitic acid, myristic acid, lauric acid, myristoleic acid, caproic acid, capric acid, caprylic acid, pelargonic acid, undecanoic acid, linoleic acid, 20: 1 eicosanoic acid, arachidonic acid, eicosapentanoic acid, docosohexanoic acid, 18:2 conjugated linoleic acid, conjugated oleic acid, or esters of: oleic acid, palmitoleic acid, palmitic acid, myristic acid, lauric acid, myristoleic acid, caproic acid, capric acid, caprylic acid, pelargonic acid, undecanoic acid, linoleic acid, 20: 1 eicosanoic acid, arachidonic acid, eicosapentanoic acid, docosohexanoic acid, 18:2 conjugated linoleic acid, or conjugated oleic acid, or glycerol esters of oleic acid, palmitoleic acid, palmitic acid, myristic acid, lauric acid, myristoleic acid, caproic acid, capric acid, caprylic acid, pelargonic acid, undecanoic acid, linoleic acid, 20: 1 eicosanoic acid, arachidonic acid, eicosapentanoic acid, docosohexanoic acid, 18:2 conjugated linoleic acid, or conjugated oleic acid, or triglyceride derivatives of oleic acid, palmitoleic acid, palmitic acid, myristic acid, lauric acid, myristoleic acid, caproic acid, capric acid, caprylic acid, pelargonic acid, undecanoic acid, linoleic acid, 20: 1 eicosanoic acid, arachidonic acid, eicosapentanoic acid, docosohexanoic acid, 18:2 conjugated linoleic acid, or conjugated oleic acid. In yet even more particular variants, the one or more fats is a rice bran oil. In another particular variant, the one or more fats is a canola oil. In some variants, the cross-linking enzyme is transglutaminase, lysyl oxidase, or other amine oxidase. In some variants, the cross-linking enzyme is transglutaminase. In particular variants, the fat replica is a high fat emulsion comprising a protein solution of purified pea albumin emulsified with 40-80% rice bran oil, stabilized with 0.5-5% (wt/vol) transglutaminase into a gel. In some variants, the fat replica is a high fat emulsion comprising a protein solution of partially-purified moong bean 8S globulin emulsified with 40-80%) rice bran oil, stabilized with 0.5-5% (wt/vol) transglutaminase into a gel. In some variants, the fat replica is a high fat emulsion comprising a protein solution of partially-purified moong bean 8S globulin emulsified with 40-80%) canola oil, stabilized with 0.5-5% (wt/vol) transglutaminase into a gel. In some variants, the fat replica is a high fat emulsion comprising a protein solution of purified pea albumin emulsified with 40-80%> rice bran oil, stabilized with 0.0001 -1%) (wt/vol) transglutaminase into a gel. In some variants, the fat replica is a high fat emulsion comprising a protein solution of partially-purified moong bean 8S globulin emulsified with 40-80%) rice bran oil, stabilized with 0.0001 - 1%) (wt/vol) transglutaminase into a gel. In some variants, the fat replica is a high fat emulsion comprising a protein solution of partially-purified moong bean 8S globulin emulsified with 40-80%) canola oil, stabilized with 0.0001 -1%) (wt/vol) transglutaminase into a gel.

### Connective Tissue Replica

Animal connective tissue provides key textural features that are an important component of the experience of eating meat. Accordingly, the present disclosure describes a composition derived from non-animal sources which recapitulates key features of animal connective tissue. The present disclosure further describes a meat substitute product that comprises a composition derived from non-animal sources which recapitulates important textural and visual features of animal connective tissue. Such a composition will be labeled herein as "connective tissue replica". In some variants, the connective tissue replica and/or meat substitute product comprising the connective tissue replica are partially derived from animal sources.

Animal connective tissue can generally be divided into fascia-type and cartilage-type tissue. Fascia-type tissue is highly fibrous, resistant against extension (has high elastic modulus), and has a high protein content, a moderate water content (ca. 50%), and low-to-none fat and polysaccharide content. Accordingly, the present disclosure describes a connective tissue replica that recapitulates key features of fascia type tissue. In some cases, the connective tissue replica comprises about 50% protein by total weight, about 50% by liquid weight, and has a low fat and polysaccharide component.

The protein content of most fascia-type connective tissue is comprised mainly of collagen. Collagen is characterized by a high fraction of proline and alanine, and also is assembled into characteristic elongated fibrils or rod- like, flexible structures. Prolamins are one family of proteins found in non-animal sources, such as plant sources. Prolamins are highly abundant in plants and are similar in amino acid composition to collagen. Among proteins we tested for this purpose, prolamins were particularly favorable because of their low cost and their ability to readily form fibers or sheets when spun or extruded. Non-limiting examples of prolamin family proteins include, e.g., zein (found in corn), these include hordein from barley, gliadin from wheat, secalin, extensins from rye, kafirin from sorghum, avenin from oats. In fascia-type connective tissue, the prolamin family of proteins, individually or combinations thereof, demonstrates suitability for the protein component because they are highly abundant, similar in global amino acid composition to collagen (high fraction of proline and alanine), and amenable to processing into films and fibers. In addition to zein (found in corn), these include hordein from barley, gliadin from wheat, secalin, extensins from rye, kafirin from sorghum, avenin from oats. Other proteins may be necessary to supplement prolamins in order to achieve targets specifications for physicochemical and nutritional properties. The list of potential candidates here is essentially open and may include Rubisco, any major seed storage proteins, proteins isolated from fungi, bacteria, archaea, viruses, or genetically engineered microorganisms, or synthesized in vitro. The proteins may be artificially designed to emulate physical properties of animal connective tissue, animal-derived or recombinant collagen, extensins (hydroxyproline-rich glycoproteins abundant in cell walls e.g. Arabidopsis thaliana, monomers of which are "collagen-like" rod- like flexible molecules). The proteins may be artificially designed to emulate physical properties of animal connective tissue.

Methods for forming fascia-type connective tissue will be as those practiced in the art with a bias towards methods producing fibrous or fibrous-like structures by biological, chemical, or physical means, individually or in combination, serially or in parallel, before final forming. These methods may include extrusion or spinning.

Cartilage-type tissue can be macroscopically homogenous, resistant against compression, has higher water content (up to 80%), lower protein (collagen) content, and higher polysaccharide (proteoglycans) contents (ca. 10% each).

Compositionally, cartilage-type connective tissue can be very similar to fascia-type tissue with the relative ratios of each adjusted to more closely mimic 'meat' connective tissue.

Methods for forming cartilage-type connective tissue can be similar to those for fascia-type connective tissue, but with a bias towards methods producing isotropically homogenous structures.

The fat can be suspended in a gel. The present disclosure describes methods for producing droplets of fat suspended in the proteinaceous gel. The fat can be isolated from plant tissues and emulsified. The emulsification can utilize high-speed blending, homogenization, agitation or temperature changes. The lipid suspension can be sonicated or extruded to further alter the properties of the lipid in the solution. At this point, in some cases other components of the consumable are added to the solution followed by a gelling agent. In some instances crosslinking agents (e.g. transglutaminase or lysyl oxidase) are added to bind the components of the consumable. In other variants the gelling agent is added and the lipid/gel suspension is later combined with additional components of the consumable. In fascia-type connective tissue, the prolamin family of proteins, individually or combinations thereof, demonstrates suitability for the protein component because they are highly abundant, similar in global amino acid composition to collagen (high fraction of proline and alanine), and amenable to processing into films. In addition to zein (found in corn), these include hordein from barley, gliadin from wheat, secalin, extensions from rye, kafirin from sorghum, avenin from oats. Other proteins may be necessary to supplement prolamins in order to achieve targets specifications for physicochemical and nutritional properties. The list of potential candidates here is essentially open and may include any major seed storage proteins, animal-derived or recombinant collagen, extensins (hydroxyproline-rich glycoproteins abundant in cell walls e.g. Arabidopsis thaliana, monomers of which are "collagen- like" rod-like flexible molecules).

In some cases some or all of the components of the consumable are suspended in a gel. In various instances the gel can be a hydrogel, an organogel, or a xerogel. The gel can be made thick using an agent based on polysaccharides or proteins. For example fecula, arrowroot, cornstarch, katakuri starch, potato starch, sago, tapioca, alginin, guar gum, locust bean gum, xanthan gum, collagen, egg whites, furcellaran, gelatin, agar, carrageenan, cellulose, methylcellulose, hydroxymethylcellulose, acadia gum, konjac, starch, pectin, amylopectin or proteins derived from legumes, grains, nuts, other seeds, leaves, algae, bacteria, of fungi can be used alone or in combination to thicken the gel, forming an architecture or structure for the consumable. Enzymes that catalyze reactions leading to covalent crosslinks between proteins can also be used alone or in combination to form an architecture or structure for the consumable. For example transglutaminase, lysyl oxidases, or other amine oxidases (e.g. Pichia pastoris lysyl oxidase (PPLO)) can be used alone or in combination to form an architecture or structure for the consumable. In some cases multiple gels with different components are combined to form the consumable. For example a gel containing a plant-based protein can be associated with a gel containing a plant-based fat. In some instances fibers or stings of proteins are oriented parallel to one another and then held in place by the application of a gel containing plant based fats.

The compositions of the disclosure can be puffed or expanded by heating, such as frying, baking, microwave heating, heating in a forced air system, heating in an air tunnel, and the like.

In some cases multiple gels with different components are combined to form the consumable. For example a gel containing a plant-based protein can be associated with a gel containing a plant-based fat. In some versions fibers or strings of proteins are oriented parallel to one another and then held in place by the application of a gel containing plant based fats.

In some cases the meat replica contains no animal products, less than 1% wheat gluten, no methylcellulose, no carrageenan, no caramel color and no Konjac flour, no gum Arabic, and no acacia gum. In some variants the meat replica contains no animal products, no wheat gluten, no methylcellulose, no carrageenan, no caramel color and no Konjac flour, no gum Arabic, and no acacia gum. In some variants the meat replica contains no animal products, no soy protein isolate, no wheat gluten, no methylcellulose, no carrageenan, no caramel color and no Konjac flour, no gum Arabic, and no acacia gum. In some variants the meat replica contains no animal products, no soy protein concentrate, no wheat gluten, no methylcellulose, no carrageenan, no caramel color and no Konjac flour, no gum Arabic, and no acacia gum. In some variants the meat replica contains no animal products, no soy protein, no wheat gluten, no methylcellulose, no carrageenan, no caramel color and no Konjac flour, no gum Arabic, and no acacia gum. In some variants the meat replica contains no animal products, no tofu, no wheat gluten, no methylcellulose, no carrageenan, no caramel color and no Konjac flour, no gum Arabic, and no acacia gum. In some variants the meat replica contains no animal products, no tofu, and no wheat gluten. In some variants the meat replica contains no animal products, no soy protein, and no wheat gluten. In some variants the meat replica contains no methylcellulose, no carrageenan, no caramel color, no Konjac flour, no gum Arabic, and no acacia gum. In some variants the meat replica contains no animal products and less than 5% carbohydrates.

In some variants the meat replica contains no animal products, no soy protein, no wheat gluten, no methylcellulose, no carrageenan, no caramel color and no Konjac flour, no gum Arabic, and no acacia gum and less than 5% carbohydrates. In some embodiments the meat replica contains no animal products, and less than 1% cellulose. In some variants the meat replica contains no animal products, and less than 5% insoluble carbohydrates. In some variants the meat replica contains no animal products, no soy protein, and less than 1% cellulose. In some variants the meat replica contains no animal products, no soy protein, and less than 5% insoluble carbohydrates. In some variants the meat replica contains no animal products, no wheat gluten, and less than 1% cellulose. In some variants the meat replica contains no animal products, no wheat gluten, and less than 5% insoluble carbohydrates.

The percentage of different components may also be controlled. For example non-animal-based substitutes for muscle, fat tissue, connective tissue, and blood components can be combined in different ratios and physical organizations to best approximate the look and feel of meat. The various can also components can be arranged to insure consistency between bites of the consumable. The components can be arranged to insure that no waste is generated from the consumable. For example, while a traditional cut of meat may have portions that are not typically eaten, a meat replicate can improve upon meat by not including these inedible portions. Such an improvement allows for all of the product made or shipped to be consumed, which cuts down on waste and shipping costs. Alternatively, a meat replica may include inedible portions to mimic the experience of meat consumption. Such portions can include bone, cartilage, connective tissue, or other materials commonly referred to as gristle, or materials included simulating these components. In some cases the consumable may contain simulated inedible portions of meat products which are designed to serve secondary functions. For example a simulated bone can be designed to disperse heat during cooking, making the cooking of the consumable faster or more uniform than meat. In other cases a simulated bone may also serve to keep the consumable at a constant temperature during shipping. In other instances, the simulated inedible portions may be biodegradable.

In some cases the meat substitute compositions contains no animal protein, comprising between 10-30% protein, between 5-80% water, between 5-70% fat, comprising one or more isolated purified proteins. In particular variants, the meat substitute compositions comprise transglutaminase. In some cases the consumable contains components to replicate the components of meat. The main component of meat is typically skeletal muscle. Skeletal muscle typically consists of roughly 75 percent water, 19 percent protein, 2.5 percent intramuscular fat, 1.2 percent carbohydrates and 2.3 percent other soluble non-protein substances. These include organic acids, sulfur compounds, nitrogenous compounds, such as amino acids and nucleotides, and inorganic substances such as minerals.

Accordingly, some variants of the present disclosure describe replicating approximations of this composition for the consumable. F or example, in some cases the consumable is a plant-based meat replica can comprise roughly 75% water, 19%> protein, 2.5% fat, 1.2% carbohydrates; and 2.3 percent other soluble non-protein substances. In some cases the consumable is a plant-based meat replica comprising between 60-90%) water, 10-30%) protein, 1-20% fat, 0.1-5%) carbohydrates; and 1-10 percent other soluble non-protein substances. In some cases the consumable is a plant-based meat replica comprising between 60-90%) water, 5-10% protein, 1-20% fat, 0.1-5%) carbohydrates; and 1-10 percent other soluble non-protein substances. , In some cases the consumable is a plant-based meat replica comprising between 0-50%> water, 5-30% protein, 20-80%> fat, 0.1-5%) carbohydrates; and 1-10 percent other soluble non-protein substances. In some cases, the replica contains between 0.01%) and 5% by weight of a heme protein. In some cases, the replica contains between 0.01% and 5%o by weight of leghemoglobin. Some meat also contains myoglobin, a heme protein, which accounts for most of the red color and iron content of some meat. In some cases, the replica contains between 0.01% and 5% by weight of a heme protein. In some cases, the replica contains between 0.01% and 5% by weight of leghemoglobin. It is understood that these percentages can vary in meat and the meat replicas can be produced to approximate the natural variation in meat. Additionally, in some instances, the present disclosure describes improved meat replicas, which comprise these components in typically unnatural percentages. For example a meat replica can be produced with a higher than typical average fat content. The percentages of these components may also be altered to increase other desirable properties.

In some instances a meat replica is designed so that, when cooked, the percentages of components are similar to cooked meat. So, in some cases, the uncooked consumable has different percentages of components than uncooked meat, but when cooked the consumable is similar to cooked meat. For example, a meat replica may be made with a higher than typical water content for raw meat, but when cooked in a microwave the resulting product has percentages of components similar to meat cooked over a fire.

In some variants the consumable is a meat replica with a lower that typical water content for meat. In some cases the disclosures describe methods for hydrating a meat replica to cause the meat replica to have a content similar to meat. For example a meat replica with a water content that would be low for meat, for example 1%, 10%, 20%, 30%, 40% or 50% water, is hydrated to roughly 75% water. Once hydrated, in some cases, the meat replica is then cooked for human consumption.

While preferred embodiments of the present have been shown and described herein, it will be obvious to those skilled in the art that such embodiments are provided by way of example only. Numerous variations, changes, and substitutions will now occur to those skilled in the art without departing from the present disclosure. It should be understood that various alternatives to the embodiments of the invention described herein may be employed in practicing the invention. It is intended that the following claims define the scope of the disclosure and that methods and structures within the scope of these claims and their equivalents be covered thereby.

### Examples

### EXAMPLE 1: METHOD OF PRODUCING AND CHARACTERIZING A HEME-CONTAINING POLYPEPTIDE

A plasmid was produced that comprised the polynucleotide sequence from *Aquifex aeolicus* encoding hemoglobin (*AaHb*)*,* where the nucleotide sequence was codon-optimized for *E. coli.* The plasmid was sub-cloned into pBE-S expression vector provided in the *B.subtilis Secretory Protein Expression System* (Takara Bio). This vector contained the *aprE* promoter sequence to promote constitutive expression of AaHb in *B. subtilis* and a C-terminal 6-histidine tag. A polynucleotide encoding the secretion signal peptide from the Twin arginine translocation (Tat)-dependent *B. subtilis* protein PhoD was synthesized and cloned in frame at the 5' end of *AaHb,* replacing the *aprE* secretion signal peptide within the *pBE-S* backbone. To generate a cytosolic *AaHb* expression construct, the *aprE* secretion signal peptide was deleted from the 5' end of the *AaHb* open reading frame using inverse PCR followed by ligation.

The expression plasmids were transformed into *B.subtilis* strain *RIK1285.* AaHb expression was monitored by growing transformed strains in LB media, 10 µg/ml kanamycin, 0.1 mM FeCl₃, and 20 µg/ml d-aminolevulinic acid. Expression was carried out at 37°C, with shaking at 200 RPM for 24 hours. After expression, the culture was collected and the secreted polypeptide was separated from the bacteria.

Cytosolic and secreted expression of AaHb was monitored by Ni-NTA affinity purification of the cell pellet and media supernatant followed by SDS-PAGE and coomassie staining of the elution fractions. Heme loading was monitored by UV-vis analysis of purified AaHb-containing fractions.

As shown in Figure 1, cytosolic expression of AaHb in *B.subtilis* was compared with (Figure 1C) and without (Figure 1B) a secretion signal peptide. The PhoD secretion peptide did not disrupt cytosolic expression of the AaHb polypeptide. Cytosolic AaHb was tested for heme content using UV-Vis spectroscopy. As shown in Figure 2, addition of the PhoD signal peptide did not interfere with AaHb heme binding in the cytosol.

As shown in Figure 3, when the AaHb polypeptide was fused to the PhoD secretion peptide, the fusion protein was effectively secreted outside of the host cell (Fig. 3B, lanes A, B). Additionally, it was shown that the PhoD secretion peptide was properly cleaved since both forms of the polypeptide (cleaved and uncleaved) were localized in the cell pellet fraction (e.g., inside the host cell) (Fig. 3B lane "cell pellet"). However, only the cleaved version of the AaHb fusion polypeptide was secreted, which indicated proper function of the PhoD signal peptide.

In support of the effectiveness of the PhoD signal peptide, N-terminal protein sequencing was performed on the secreted polypeptide. Figure 4 depicts the protein sequence of the PhoD-AaHb fusion protein sequence (which also comprises a His6 tag). A protein band was removed from the gel for N-terminal protein sequencing analysis. This band corresponded to the secreted protein because of its abundance and size on the gel. The N-terminal sequencing results indicated that the PhoD peptide was indeed cleaved (e.g., the size was not due to non-specific degradation) at the correct site because the N-terminus of the sequencing results matched the predicted cut site of the protease.

The secreted AaHb was further characterized for heme content by monitoring the UV-vis absorbance of AaHb purified from the media. Figure 5 illustrates the effects of the PhoD signal peptide on the heme content of the secreted AaHb. The secreted AaHb was heme bound, as evidenced by a noticeable absorption peak at approximately 415 nm.

Taken together, these results suggest that the PhoD signal peptide does not interfere with cytosolic secretion of a polypeptide, is properly cleaved, enhances secretion of a polypeptide, and maintains the heme content of the secreted polypeptide.

### PROPHETIC EXAMPLE 1: METHOD OF USING A POLYPEPTIDE IN A MEAT CONSUMABLE

In some instances, a polypeptide in this disclosure will be expressed and purified as described in Example 1.

In some instances, a muscle tissue analog will be constructed with the polypeptide and pea vicilin protein using transglutaminase cross-linking.

In some instances, a muscle tissue analog will be constructed by heat/cool gel formation of purified pea vicilin proteins. The heme-containing polypeptide will be thoroughly mixed with the partially gelled muscle tissue upon cooling to room temperature.

In some instances, a muscle tissue analog will be constructed by co-extruding heme-containing polypeptide with purified pea vicilin proteins.

In some instances, a fat tissue analog will be constructed by emulsifying pea albumin proteins, coconut oil, and lecithin through high-pressure homogenization followed by a heat/cool treatment. Heme-containing polypeptide will be thoroughly mixed with the partially gelled adipose tissue upon cooling to room temperature.

In some instances, a connective tissue analogue will be prepared with a zein protein source by extrusion or electrospinning.

In some instances, a ground beef replica (e.g., meat consumable) will be prepared by combining the muscle analog comprising a purified polypeptide of the disclosure with varying amounts of the fat tissue analog and the connective tissue analog. In some instances, the different tissues may be combined using a meat grinder. The resulting meat consumable can be cooked before eating. The cooking procedure will induce the red color of the meat consumable to change to a brown color, indicating cooking. In some instances, the red color of the meat consumable is due to the purified heme-containing polypeptide of the disclosure. The cooking procedure will catalyze the release of meat flavors and aromas. In some instances, the flavors and aromas of the meat consumable are due to the purified heme-containing polypeptide of this disclosure.

### EXAMPLE 2: METHOD TO MODULATE EXPRESSION AND SECRETION OF AN ENDOGENOUS HEME-CONTAINING POLYPEPTIDE

Two plasmids were produced, transformed, and cultured as described in Example 1. Both plasmids include the polynucleotide sequence from *B. subtilis* encoding the endogenous truncated hemoglobin gene, *yjbI* (Figure 9, SEQ ID NO:26). In the case of the first plasmid, a polynucleotide encoding the secretion signal peptide from the Twin arginine translocation (Tat)-dependent *B. subtilis* protein *PhoD* (Table 1) was synthesized and cloned in frame at the 5' end of the *yjbI* gene (*PhoD-yjbI*). The second plasmid included the signal peptide *YwbN* (Table 1), which was also synthesized and cloned in frame at the 5' end of the *yjbI* gene (*YwbN-yjbI*)*,* as described in Example 1.

As shown in Figure 6, after 24 hours of growth, cytosolic and secreted expression of *yjbI* was monitored. The cell pellet and media fractions were separated by SDS-PAGE, transferred onto a PVDF membrane, and probed with an anti-His6 antibody (Abcam). Specifically, the figure shows the detection of the fused polypeptides (*PhoD-yjbI* and *YbwN-yjbI)* in the cell pellet following expression of the endogenous polypeptide from the exogenous nucleic acid, and media detection of the polypeptide, which indicated proper cleavage of the signal peptide.

### EXAMPLE 3: METHOD TO MODULATE EXPRESSION AND SECRETION OF A HETEROLOGOUS HEME-CONTAINING POLYPEPTIDE

Two plasmids were produced, transformed, and cultured as described in Example 1. The first plasmid included the polynucleotide sequence from *Glycine max* (soybean) encoding *LGB2* (Figure 9, SEQ ID NO:4). The second plasmid included the protein coding polynucleotide sequence from *M. infemorum, HGbI* (Figure 9, SEQ ID NO: 2). In both plasmids, a polynucleotide encoding the secretion signal peptide from the Twin arginine translocation (Tat)-dependent *B. subtilis* protein *PhoD* (Table 1) was synthesized and cloned in frame at the 5' end of each polynucleotide sequence (*PhoD-LGB2* and *PhoD-HGbI*).

As shown in Figure 7, after 24 hours of growth, cytosolic and secreted expression of the two polypeptides was monitored. The cell pellet and media fractions were separated by SDS-PAGE, transferred onto a PVDF membrane, and probed with an anti-His6 antibody (Abcam). Specifically, the figure shows the detection of the fused polypeptides (*PhoD-LGB2* and *PhoD-HGbI)* in the cell pellet following expression of each heterologous polypeptide, and media detection of each heterologous polypeptide, which indicated proper cleavage of the signal peptide.

### EXAMPLE 4: METHOD OF PRODUCING AND CHARACTERIZING A HEME-CONTAINING POLYPEPTIDE FUSED TO DIFFERENT SIGNAL PEPTIDES

A series of plasmids were produced, transformed, and cultured as described in Example 1. Each plasmid included polynucleotide sequence from *Aquifex aeolicus* encoding hemoglobin (*AaHb*)*.* To the 5'end of the *AaHb* sequence, a nucleic acid sequence encoding a secretion signaling peptide selected from *AbnA, AlbB, AppB, BgIS, LipA, OppA, SpoIIIJ, TipA, WapA, WprA, YkpC, YmaC, YolA, YuiC,* or *YwbN* (Table 1) was fused as described previously.

As shown in Figure 8, after 24 hours of growth, the media supernatants of a subset of the fusion polypeptides were separated by SDS-PAGE, transferred onto a PVDF membrane, and blotted with an anti-His6 antibody (Abcam). The figure illustrates the media detection of the endogenous polypeptide (*AaHb*) after fusion with a subset of a number of different exemplary secretion signaling peptides (*PhoD, TipA, WapA, WprA, YmaC, YolA, YuiC, YwbN, AppB,* and *BglS),* expression, and cleavage of the secretion signal peptide.

## Claims

1. A recombinant bacterium cell, wherein said recombinant bacterium cell comprises at least one exogenous nucleic acid encoding a heme-containing polypeptide and is capable of secreting said heme-containing polypeptide, wherein said at least one exogenous nucleic acid encoding said heme-containing polypeptide comprises first and second nucleic acid sequences, wherein said first nucleic acid sequence encodes a signal peptide and said second nucleic acid sequence encodes said heme-containing polypeptide, wherein said first and second nucleic acid sequences are operably linked to produce a fusion polypeptide comprising said signal peptide and said heme-containing polypeptide;
wherein said heme-containing polypeptide is selected from the group consisting of an androglobin, a cytoglobin, globin E, globin X, globin Y, a hemoglobin, a myoglobin, a leghemoglobin, an erythrocruorin, a beta hemoglobin, an alpha hemoglobin, a non-symbiotic hemoglobin, a flavohemoglobin, a protoglobin, a cyanoglobin, a Hell's gate globin I, a bacterial hemoglobin, a ciliate myoglobin, a histoglobin, a neuroglobin, a protoglobin, and a truncated globin, wherein said truncated globin is preferably truncated 2/2 globin, HbN, HbO, or Glb3;
wherein said at least one exogenous nucleic acid is a vector; and
wherein said vector further comprises a polynucleotide sequence encoding two or more heme biosynthesis pathway enzymes.

2. The recombinant bacterium cell of claim 1, wherein said heme-containing polypeptide has at least 60% sequence identity to an amino acid sequence set forth in any one of SEQ ID NOs: 1-31.

3. The recombinant bacterium cell of claim 1 or claim 2, wherein said recombinant bacterium cell secretes said heme-containing polypeptide from said recombinant bacterium cell, and upon secretion, said signal peptide is removed from said heme-containing polypeptide.

4. The recombinant bacterium cell of any one of claims 1-3, wherein said signal peptide comprises an amino acid sequence having at least 60% identity to a signal peptide set forth in SEQ ID NO: 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, or 93.

5. The recombinant bacterium cell of any one of claims 1-3, wherein said signal peptide comprises an amino acid sequence having at least 60% amino acid sequence identity to the amino acid sequence set forth in SEQ ID NO:55 or to residues 1-52 of SEQ ID NO:55.

6. The recombinant bacterium cell of any one of claims 1-5, wherein said recombinant bacterium cell is a *Bacillus* cell.

7. A method for producing a heme-containing polypeptide, said method comprising culturing said recombinant bacterium cell of any one of claims 1-6 in a culture medium under conditions that allow said heme-containing polypeptide to be secreted into said culture medium, wherein upon secretion of said fusion polypeptide from said recombinant bacterium cell into said culture medium, said signal peptide is removed from said heme-containing polypeptide.

8. The method of claim 7, further comprising recovering said heme-containing polypeptide from said culture medium.

## Patentansprüche

1. Rekombinante Bakterienzelle, wobei die rekombinante Bakterienzelle mindestens eine exogene Nukleinsäure, die für ein hämhaltiges Polypeptid codiert, umfasst und zum Sekretieren des hämhaltigen Polypeptids fähig ist, wobei die mindestens eine exogene Nukleinsäure, die für das hämhaltige Polypeptid codiert, erste und zweite Nukleinsäuresequenzen umfasst, wobei die erste Nukleinsäuresequenz für ein Signalpeptid codiert und die zweite Nukleinsäuresequenz für das hämhaltige Polypeptid codiert, wobei die erste und die zweite Nukleinsäuresequenz funktionsfähig verknüpft sind, um ein Fusionspolypeptid, umfassend das Signalpeptid und das hämhaltige Polypeptid, herzustellen;
wobei das hämhaltige Polypeptid ausgewählt ist aus der Gruppe bestehend aus einem Androglobin, einem Cytoglobin, Globin E, Globin X, Globin Y, einem Hämoglobin, einem Myoglobin, einem Leghämoglobin, einem Erythrocruorin, einem Beta-Hämoglobin, einem Alpha-Hämoglobin, einem nichtsymbiotischen Hämoglobin, einem Flavohämoglobin, einem Protoglobin, einem Cyanoglobin, einem Hell's-Gate-Globin I, einem bakteriellen Hämoglobin, einem Ziliaten-Myoglobin, einem Histoglobin, einem Neuroglobin, einem Protoglobin und einem trunkierten Globin, wobei das trunkierte Globin vorzugsweise trunkiertes 2/2-Globin, HbN, HbO oder Glb3 ist;
wobei die mindestens eine exogene Nukleinsäure ein Vektor ist; und
wobei der Vektor ferner eine Polynukleotidsequenz, die für zwei oder mehr Hämbiosynthesewegenzyme codiert, umfasst.

2. Rekombinante Bakterienzelle nach Anspruch 1, wobei das hämhaltige Polypeptid mindestens 60 % Sequenzidentität zu einer Aminosäuresequenz, angegeben in einer der SEQ ID NOs: 1-31, aufweist.

3. Rekombinante Bakterienzelle nach Anspruch 1 oder 2, wobei die rekombinante Bakterienzelle das hämhaltige Polypeptid aus der rekombinanten Bakterienzelle sekretiert und bei Sekretion das Signalpeptid von dem hämhaltigen Polypeptid entfernt wird.

4. Rekombinante Bakterienzelle nach einem der Ansprüche 1-3, wobei das Signalpeptid eine Aminosäuresequenz umfasst, die mindestens 60 % Identität zu einem Signalpeptid, angegeben in SEQ ID NO: 33, 35, 37, 39, 41, 43, 45, 47, 49, 51, 53, 55, 57, 59, 61, 63, 65, 67, 69, 71, 73, 75, 77, 79, 81, 83, 85, 87, 89, 91 oder 93, aufweist.

5. Rekombinante Bakterienzelle nach einem der Ansprüche 1-3, wobei das Signalpeptid eine Aminosäuresequenz umfasst, die mindestens 60 % Aminosäuresequenzidentität zu der Aminosäuresequenz, angegeben in SEQ ID NO:55, oder zu den Resten 1-52 von SEQ ID NO:55 aufweist.

6. Rekombinante Bakterienzelle nach einem der Ansprüche 1-5, wobei die rekombinante Bakterienzelle eine *Bacillus-*Zelle ist.

7. Verfahren zum Herstellen eines hämhaltigen Polypeptids, wobei das Verfahren Kultivieren der rekombinanten Bakterienzelle nach einem der Ansprüche 1-6 in einem Kulturmedium unter Bedingungen, die es ermöglichen, dass das hämhaltige Polypeptid in das Kulturmedium sekretiert wird, umfasst, wobei bei Sekretion des Fusionspolypeptids aus der rekombinanten Bakterienzelle in das Kulturmedium das Signalpeptid von dem hämhaltigen Polypeptid entfernt wird.

8. Verfahren nach Anspruch 7, ferner umfassend Gewinnen des hämhaltigen Polypeptids aus dem Kulturmedium.

## Revendications

1. Cellule bactérienne recombinante, dans laquelle ladite cellule bactérienne recombinante comprend au moins un acide nucléique exogène codant pour un polypeptide contenant de l'hème et est capable de sécréter ledit polypeptide contenant de l'hème, dans laquelle ledit au moins un acide nucléique exogène codant pour ledit polypeptide contenant de l'hème comprend des première et seconde séquences d'acide nucléique, dans laquelle ladite première séquence d'acide nucléique code pour un peptide signal et ladite seconde séquence d'acide nucléique code pour ledit polypeptide contenant de l'hème, dans laquelle lesdites première et seconde séquences d'acide nucléique sont liées de manière fonctionnelle pour produire un polypeptide de fusion comprenant ledit peptide signal et ledit polypeptide contenant de l'hème ;
dans laquelle ledit polypeptide contenant de l'hème est choisi parmi le groupe constitué d'une androglobine, d'une cytoglobine, d'une globine E, d'une globine X, d'une globine Y, d'une hémoglobine, d'une myoglobine, d'une léghémoglobine, d'une érythrocruorine, d'une bêta-hémoglobine, d'une alpha-hémoglobine, d'une hémoglobine non symbiotique, d'une flavohémoglobine, d'une protoglobine, d'une cyanoglobine, d'une globine Hell's Gate I, d'une hémoglobine bactérienne, d'une myoglobine de cilié, d'une histoglobine, d'une neuroglobine, d'une protoglobine et d'une globine tronquée, dans laquelle ladite globine tronquée est de préférence la globine tronquée 2/2, HbN, HbO ou Glb3 ;
dans laquelle ledit au moins un acide nucléique exogène est un vecteur ; et
dans laquelle ledit vecteur comprend en outre une séquence polynucléotidique codant pour deux enzymes de la voie de biosynthèse de l'hème ou plus.

2. Cellule bactérienne recombinante selon la revendication 1, dans laquelle ledit polypeptide contenant de l'hème présente au moins 60 % d'identité de séquence avec une séquence d'acides aminés définie dans l'une quelconque des SEQ ID NO : 1 à 31.

3. Cellule bactérienne recombinante selon la revendication 1 ou la revendication 2, dans laquelle ladite cellule bactérienne recombinante sécrète ledit polypeptide contenant de l'hème à partir de ladite cellule bactérienne recombinante, et lors de la sécrétion, ledit peptide signal est retiré dudit polypeptide contenant de l'hème.

4. Cellule bactérienne recombinante selon l'une quelconque des revendications 1 à 3, dans laquelle ledit peptide signal comprend une séquence d'acides aminés présentant au moins 60 % d'identité avec un peptide signal défini dans SEQ ID NO : 33, 35, 37, 39, 41, 43, 45, 47, 49, 51, 53, 55, 57, 59, 61, 63, 65, 67, 69, 71, 73, 75, 77, 79, 81, 83, 85, 87, 89, 91 ou 93.

5. Cellule bactérienne recombinante selon l'une quelconque des revendications 1 à 3, dans laquelle ledit peptide signal comprend une séquence d'acides aminés présentant au moins 60 % d'identité de séquence d'acides aminés avec la séquence d'acides aminés définie dans SEQ ID NO :55 ou avec les résidus 1 à 52 de SEQ ID NO :55.

6. Cellule bactérienne recombinante selon l'une quelconque des revendications 1 à 5, dans laquelle ladite cellule bactérienne recombinante est une cellule de *Bacillus.*

7. Procédé de production d'un polypeptide contenant de l'hème, ledit procédé comprenant la culture de ladite cellule bactérienne recombinante selon l'une quelconque des revendications 1 à 6 dans un milieu de culture dans des conditions permettant la sécrétion dudit polypeptide contenant de l'hème dans ledit milieu de culture, dans lequel, lors de la sécrétion dudit polypeptide de fusion à partir de ladite cellule bactérienne recombinante dans ledit milieu de culture, ledit peptide signal est retiré dudit polypeptide contenant de l'hème.

8. Procédé selon la revendication 7, comprenant en outre la récupération dudit polypeptide contenant de l'hème à partir dudit milieu de culture.
